# EUROPEAN PATENT APPLICATION

(11) **EP 4 483 954 A1**
(43) Date of publication of application: **01.01.2025**
(21) Application number: 23182520.9
(22) Date of filing: 29.06.2023
(51) Int. Cl.: A61P 25/00, A61P 35/00, C07D 495/04, C07D 519/00, A61K 31/4365

(54) **SUBSTITUTED THIENO [3,2-B]PYRIDINES AS INHIBITORS OF PROTEIN KINASES**

(71) Applicant: Masarykova Univerzita, 60177 Brno (CZ); FAKULTNI NEMOCNICE U SV. ANNY V BRNE, 60200 Brno (CZ)
(72) Inventor: PARUCH, Kamil, CZ-66601 Tisnov (CZ); MARTIN MOYANO, Paula, ES-41913 (ES)
(74) Representative: Harber IP s.r.o.

(57) **Abstract**

The present invention provides compounds of general formula I or pharmaceutically acceptable salt thereof.

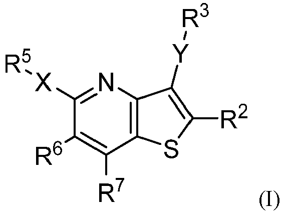

These compounds are particularly useful in the treatment of cancers and neurodegenerative diseases.

## Description

### Field of Art

The present invention relates to new heterocyclic compounds useful for therapeutic use based on inhibition of all kinases.

### Background Art

Protein kinases are involved in regulation of numerous cellular signaling pathways (International Journal of Molecular Medicine 2017, 40, 271.). Aberrant activity of various protein kinases is therefore frequently related to the initiation and progression of numerous diseases, in particular cancers (Nature Reviews Cancer 2016, 16, 83.). Correspondingly, protein kinases represent one of the most attractive class of targets for pharmacological inhibition and >70 small-molecule kinase inhibitors have been approved for clinical use (Nature Reviews Drug Discovery 2021, 20, 839.). While potent and selective inhibitors have been identified for some kinases, for many other kinases they are yet to be discovered (Nucleic Acids Research 2021, 49, D529.).

Haploid germ cell - specific nuclear protein kinase (Haspin) (Drug Discovery Today 2018, 23, 409.) phosphorylates histone H3 at threonine 3; this process initiated in late G2 phase becomes highly significant in prophase and metaphase. H3T3ph plays an essential role during mitosis providing a chromatin binding site for the chromosomal passenger complex (CPC) at centromeres to control chromosome segregation (Current Biology 2011, 21, 1061.). Haspin is thus essential in the control of the cell cycle progression, namely regulation of centromeric cohesion, spindle stability and chromosome alignment. Inhibition of Haspin activity can therefore affect correct mitotic progression, and can lead to abnormal chromosome segregation, mitotic catastrophe of cancer cells.
In recent years, Haspin has become an emerging target in oncology (Oncogene 2012, 31, 1408.; Cancer Research 2020, 80, 798.). This is further illustrated by the selected studies summarized below. Elimination of Haspin has beeen reported to lead to cell cycle defects and mitotic cell death of cancer cells (FASEB Journal 2021, 35, e21923.).
Haspin has been found to be overexpressed in diverse cancers, e.g. multiple myeloma, Ewing sarcoma, pancreatic ductal adenocarcinoma, and gallbladder carcinoma (Cancer Research 2020, 80, 798.; Experimental Cell Research 2020, 390, 111863.); modulation of ist activity can be therefore particularly attractive in the treatment of cancers with overexpressed levels of the kinase (Scientific Reports 2019, 9, 16588.; Experimental Cell Research 2019, 385, 111605.). Clinically, high levels of Haspin overexpression have been found to correlate with shorter survival of patients (Scientific Reports 2019, 9, 16588.).
Correspondingly, significant effort has been invested into identification of Haspin inhibitors and over the last two decades, several classes of them having variable potency and selectivity have been described in the literature (Journal of Pharmacy and Pharmacology 2022, https://doi.org/10.1093/jpp/rgac080).
Inhibition of Haspin (by the compound CHR-6494) was found to suppress proliferation of cancer cells (Oncogene 2012, 31, 1408.; PLoS One 2021, 16, e0249912.). Increasing number of reports suggest that inhibition of Haspin can be used in combination with other agents. E.g., combined Haspin and mTOR inhibition elicited mitotic catastrophe in KRAS-driven carcinoma cells (Translational Oncology 2022, 26, 101540.), and synergistic inhibition of Haspin, Aurora A or B emerged recently as an attractive strategy to treat head and neck squamous cell carcinoma, non - small cell lung cancer and colorectal cancer (Biochemical Pharmacology 2022, 206, 115289.; Oncogene 2020, 39, 4312.).

Cyclin-dependent kinase-like kinases (CDKLs) form a family of kinases whose biology remains to be explored in greater detail (Cell Reports 2018, 22, 885.).
CDKL2 has been recently recognized as a regulator of epithelial-mesenchymal transition of cancer cells (BioMed Research International 2020, article ID 1712723).
CDKL3 is a serine/threonine-protein kinase whose function has not been fully elucidated. However, inhibition or depletion of CDKL3 have been shown to elicit inhibition of proliferation, migration, and cell cycle progression of human cholangiocarcinoma cells (Frontiers in Physiology 2018, 9, 234, 1-11.). It was also demonstrated that high levels of CDKL3 promote progression of osteosarcoma and negatively correlate with the patient survival (Life Science Alliance 2020, 3, e202000648.).
CDKL4 is a serine/threonine-protein kinase that has been found to affect the clinical outcome of the treatment of colorectal cancer (Oncotarget 2015, 6, 16774.). In addition, high levels of CLK4 have been found in malignant bone tumour (chordomas) (Biochimica et Biophysica Acta Reviews on Cancer 2022, 1877, 188812.).
In mouse models, genetic or pharmacological CDKL5 inhibition has been shown to mitigate nephrotoxic and ischemia-associated acute kidney injury (Nature Communications 2020, 11, article number 1924.). Recent genetic studies indicate that mutations of CDKLs are associated with neurodevelopmental and neuropsychiatric disorders in humans (Frontiers in Behavioral Neuroscience 2010, 4, article 17.).

Cdc - like kinase 2 (CLK2) is involved in numerous biological processes for instance regulation of signaling cascades and the spliceosome molecular machinery, whose aberrant activity can lead to several diseases such as cancer (International Journal of Molecular Sciences 2020, 21, 7549.; Journal of Medicinal Chemistry 2021, 64, 13191.; Scientifc Reports 2021, 11, 7963.). Recently, CLK2 has ben also recognized as an attractive therapeutic target for neurodegenerative diseases, for instance Phelan-McDermid syndrome (PMDS), including a high risk of autism spectrum disorder (ASD), and Alzheimer (ChemMedChem 2018, 13, 1997.; Journal of Neurochemistry 2006, 96, 1199.; Science 2016, 351, 6278.).
Several classes of CLK2 inhibitors have been reported over the last decade (Journal of Medicinal Chemistry 2021, 64, 13191.). First CLK2 inhibitors have already entered in clinical trials, namely SM04690 (degenerative disc disease and osteoarthritis), SM08502 (gastrointestinal and pancreatic cancers) and SM04755 (psoriasis, scleroderma) (Osteoarthritis and Cartilage 2021, 29, 654e666.; Journal of Medicinal Chemistry 2021, 64, 13191.).

The serine/threonine kinase PIM1 is a key protein in cellular signaling pathways such as JAK STAT (cellular processes) and NF kB (cell stimuli) (American Journal of Cancer Research 2020, 10, 4085.; Blood 2017, 130, 1418.). PIM1 regulates various oncogenic pathways, for example hypoxia response, cell cycle progression and apoptosis resistance; in addition, it is overexpressed in human cancers and has been associated with metastasis and overall treatment response - in experimental models, inhibition of PIM1 suppressed cell proliferation and migration, induced apoptotic cell death and synergized with other chemotherapeutic agents (Expert Opinion on Investigational Drugs 2012, 21, 425.). PIM1 expression has been also found to correlate with resistance of prostate tumor cell lines to chemotherapy (Journal of Biological Chemistry 2008, 283, 20635.). Considerable effort invested into the identification of PIM1 inhibitors yielded several classes of them (Haematologica 2010, 95, 1004; Signal Transduction and Targeted Therapy 2020, 5, 7.).

The Dual specificity tyrosine-phosphorylation - regulated kinase 2 (DYRK2) regulates proteostasis in cancers, promotes nuclear stability and transcriptional activity of its substrate, heat-shock factor 1 triggering protein folding (Journal Biological Chemistry 2021, 296, 100233.). Moreover, DYRK2 is essential in regulation of the Hedgehog pathway and cellular growth proliferation (eLife, 2020, 9, e57381.). DYRK2 is highly expressed in prostate cancer cells and its knock-down was found to remarkably reduce prostate cancer burden *in vitro* and *in vivo* (Nature Communications 2022, 13, article number 2903.). Several classes of DYRK2 inhibitors have been identified and several DYRK2-targeting compounds have been undergoing preclinical and clinical profiling (Journal of Biological Chemistry 2021, 296, 100233.).

TAF1L kinase is mainly localized in the nucleus (in large chromatin complexes) and it is essential for the regulation of the transcriptional processes. It was recently identified their role in gastric and colorectal cancers (Pathology Oncology Research 2017, 23, 125.). Overexpression of TAF1L has been found to promote cell proliferation, migration and invasion of esophageal squamous cell carcinoma (Journal of Cancer 2019, 10, 979.; International Journal of Biological Sciences 2020, 16, 1180.).
Pharmacological inhibition of TAF1L is relatively underexplored and only several inhibitors have been reported in the literature; namely benzoisoquinolinedione-based inhibitors of TAF1L bromodomain (Journal of Medicinal Chemistry 2017, 60, 4002.) and the triple inhibitor BAY-299 targeting BRPF2 and TAF1/TAF1L *(*ChemMedChem 2019, 14, 362.).

TRB proteins play a crucial role in multiple signaling networks and their overexpression is associated with cancer, making them a novel class of drug target. (Biochemical Journal 2015, 467, 47.). Namely, TRB2 was identified as an oncogene, which induces acute myeloid leukemia (Cancer Cell, 2006, 10, 401.).

### Summary of the Invention

The present invention relates to compounds of general formula I or pharmaceutically acceptable salts thereof 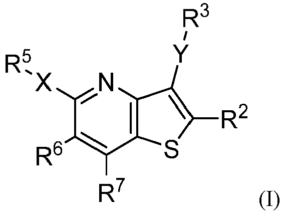 wherein:
R² is selected from H, C1-C4 alkyl and CF₃;
Y is selected from bond, -C(=O)-, -CH(OH)-, -C(=CH₂)-, -CH₂-O-, -CH₂-S-, -CH₂-NH₂-, O, S, SO₂, NR⁸ or CR⁸R⁸;
R³ is selected from the group consisting of:
   - C6-C14 aryl,
   - 3-10-membered heteroaryl comprising at least one heteroatom selected from S, O, N,
   - C5-C8 cycloalkyl,
   - 5-8-membered heterocycloalkyl comprising at least one heteroatom selected from S, O, N,
   - C5-C8 cycloalkenyl,
   - 5-8-membered heterocycloalkenyl comprising at least one heteroatom selected from S, O, N,
   wherein each of the listed substituents can optionally be substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, cyclo(C3-C6 alkyl), cyclo(C3-C6 alkyl)-C1-C2 alkyl-, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NOz, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, - (C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, - COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SOz-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SOz-NH-(C1-C4 alkylene)-, (C1-C4 alkyl)-SOz-N(C1-C4 alkyl)-, (C1-C4 alkyl)-SOz-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CON(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
X is selected from bond, O, S, SOz, -C(=O)-, -CH(OH)-, -CH₂-O-, -CH₂-S-, -CH₂-NH₂-, -C(=CH₂)-, NR⁸ or CR⁸R⁸;
R⁵ is selected from the group consisting of:
   - C6-C14 aryl,
   - 3-10-membered heteroaryl comprising at least one heteroatom selected from S, O, N,
   - C5-C8 cycloalkyl,
   - 5-8-membered heterocycloalkyl comprising at least one heteroatom selected from S, O, N,
   - C5-C8 cycloalkenyl,
   - 5-8-membered heterocycloalkenyl comprising at least one heteroatom selected from S, O, N,
   wherein each of the listed substituents can optionally be substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, cyclo(C3-C6 alkyl), cyclo(C3-C6 alkyl)-C1-C2 alkyl-, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NOz, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, - (C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, - COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SOz-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SOz-NH-(C1-C4 alkylene)-, (C1-C4 alkyl)-SOz-N(C1-C4 alkyl)-, (C1-C4 alkyl)-SOz-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CON(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
R⁶ is selected from H, C1-C4 alkyl and CF₃;
R⁷ is selected from H, C1-C4 alkyl, F, Cl, Br, OR⁸ and CF₃;
R⁸ is independently selected from H and C1-C4 alkyl.

The compounds of formula I can be in the form of free bases or in the form of addition salts with pharmaceutically acceptable organic or inorganic acids, such as hydrochloric acid. Other suitable pharmaceutically acceptable acids include: acetic, aspartic, benzenesulfonic, benzoic, besylic, bicarbonic, bitartaric, hydrobromic, camsylic, carbonic, citric, decanoic, edetic, esylic, fumaric, gluceptic, gluconic, glutamic, glycolic, hexanoic, hydroxynaphthoic, hydroiodic, isethionic, lactic, lactobionic, malic, maleic, mandelic, methanesulphonic, methylsulfonic, mucoic, napsylic, nitric, octanoic, oleic, pamoic, pantothenic, phosphonic, polygalacturonic, propionic, salicylic, stearic, succinic, sulfonic, tartaric, teoclic, toluenesulphonic acids.

Halogens are selected from fluorine, chlorine, bromine and iodine.
Alkyl is a branched or linear saturated hydrocarbyl.
Alkenyl is a branched or linear hydrocarbyl comprising at least one double bond.
Alkylene is a divalent branched or linear, preferably linear, hydrocarbyl residue. A preferred alkylene is methylene.
Cycloalkyl is a saturated hydrocarbyl comprising at least one aliphatic cycle.
Cycloalkenyl is a hydrocarbyl comprising at least one aliphatic cycle and at least one double bond in the cycle.
Aryl is a hydrocarbyl comprising at least one aromatic cycle. Examples of aryl are phenyl, benzyl.

Heteroaryl is a heterohydrocarbyl comprising at least one aromatic cycle comprising at least one heteroatom selected from O, S, N.
Heterocyclyl or cycloheteroalkyl is a heterohydrocarbyl comprising at least one aliphatic cycle which contains at least one heteroatom selected from O, S, N in the cycle.
Cycloheteroalkenyl is a heterohydrocarbyl comprising at least one double bond and at least one aliphatic cycle which contains at least one heteroatom selected from O, S, N in the cycle.
Cyclic structures can thus contain one or more cycles, wherein the cycles can be conjugated or connected by a C1-C3 linker.

Preferably, R² is selected from H, methyl, ethyl.

Preferably, Y is selected from bond, -C(=O)-, -CH(OH)-, -C(=CH₂)-, -NH-, -N(CH₃)-.

Preferably, R³ is selected from the group consisting of C6-C10 aryl and 5-10-membered heteroaryl comprising at least one heteroatom selected from S, O, N, wherein the aryl or heteroaryl are optionally substituted.

Preferably, R³ is selected from the group consisting of phenyl and 5-10-membered heteroaryl comprising at least one heteroatom N and optionally at least one other heteroatom selected from S, O, N, wherein the phenyl or heteroaryl are optionally substituted.

Preferably, R³ is selected from the group consisting of pyridinyl, phenyl, thiazolyl, thiadiazolyl, isothiazolyl, pyrimidinyl, pyridazinyl, pyrazolyl, pyrrolopyridinyl, quinazolinyl, quinoxalinyl, indazolyl, triazolopyridinyl, pyrazolopyrimidinyl, pyrazolopyridinyl, imidazopyridinyl, dihydropyridinyl and tetrahydropyridinyl, wherein any of these substituent groups is optionally substituted.

Preferably, R³ is substituted by one or more substituents selected independently from C1-C4 alkyl (preferably methyl), halogen (preferably fluorine), morpholinyl, benzyl, amino, aminomethyl and trifluoromethyl.

Preferably, X is selected from bond, -NH-, -N(CH₃)-, -O-, -S-, -CH₂-O-, -CH₂-NH₂-, -C(=CH₂)-.

R⁵ is preferably selected from the group consisting of C6-C10 aryl, 5-10-membered heteroaryl comprising at least one heteroatom selected from S, O, N, and 5-6-membered heterocycloalkyl comprising at least one heteroatom selected from S, O, N, wherein the aryl or heteroaryl or heterocycloalkyl are optionally substituted.

R⁵ is preferably selected from the group consisting of phenyl, morpholinyl and 5-10-membered heteroaryl comprising at least one heteroatom N and optionally at least one other heterotom selected from S, O, N, wherein the phenyl, morpholinyl, or heteroaryl are optionally substituted.

R⁵ is preferably selected from the group consisting of pyrazolyl, phenyl, morpholinyl, imidazolyl, isothiazolyl, triazolyl, pyridinyl, pyrimidinyl, pyridazinyl, pyrazolopyrimidinyl, furanyl, and isoxazolyl, , wherein any of these substituent groups is optionally substituted.

R⁵ is preferably substituted by one or more substituents selected independently from C1-C4 alkyl (preferably methyl, ethyl, isopropyl, tert-butyl), trifluoromethyl, benzyl, phenyl, cyclo(C3-C6 alkyl), cyclo(C3-C6 alkyl)-C1-C2 alkyl-, amino, halogen (preferably F), -COO(C1-C4 alkyl), C1-C4 alkylthio, C1-C4-alkoxy, CN.

Preferably, R⁶ is selected from Hand C1-C4 alkyl (preferably methyl), phenyl.

Preferably, R⁷ is selected from H, C1-C4 alkyl (preferably methyl), F, Cl, Br, O(C1-C4 alkyl) (preferably methoxy).

Particularly preferred are the following compounds (in the form of free bases or in the form of salts with pharmaceutically acceptable acids):

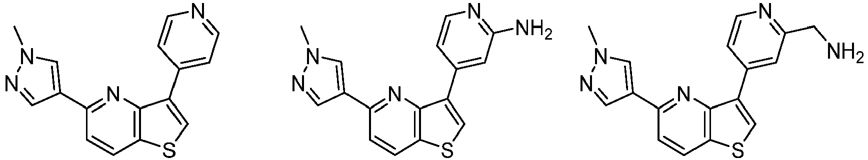

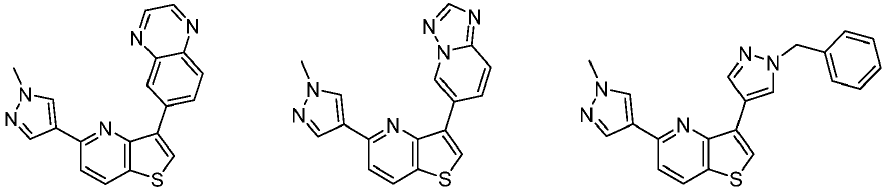

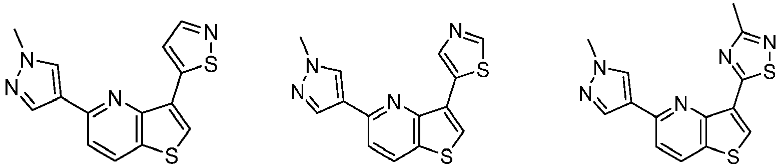

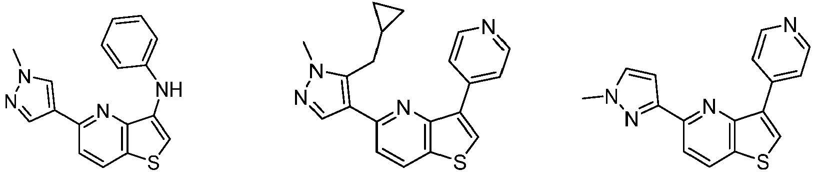

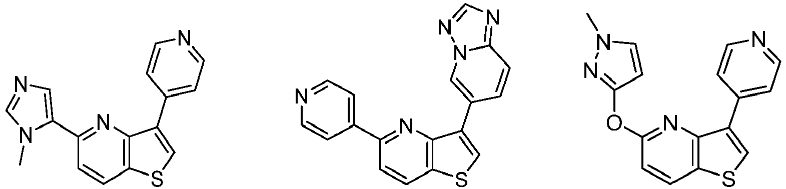

In another aspect, the invention provides the compounds of formula I for use as medicaments.

The compounds of formula I are suitable for the treatment of diseases involving the kinases CDKL, CLK, DYRK, Haspin, PIM, TAF1L and/or TRB.

In particular, the compounds of formula I are suitable for use in the treatment of cancers or neurodegenerative diseaes.

More specifically, the compounds of formula I are suitable for use in the treatment of leukemia such as acute myeloid leukemia; autoimmune diseases such as rheumatoid arthritis or autoimmune hepatitis; peripheral neuropathic pain; cancers such as non-small-cell lung carcinoma (NSCLC), ovarian cancer, glioblastoma, breast cancer, lung cancer, lung adenocarcinoma, pancreas cancer, prostate cancer, brain cancer, ovarian cancer, colorectal cancer, liver cancer, hepatocellular carcinoma, squamous cell carcinoma; periodontitis; pulmonary fibrosis; obesity; insulin resistance; obesity-induced hyperinslinemia; atherosclerosis; neurodegenerative disorders such as Alzheimer's disease.

The compounds of formula I may be formulated using the methods known to a person skilled in the art of pharmaceutical formulation. The pharmaceutical compositions contain at least one compound of formula I or a pharmaceutically acceptable salt thereof and at least one auxiliary compound. Auxiliary compounds may be selected in particular from fillers, binders, solvents, glidants and preservatives.

### Examples

### I. Preparative Examples

### Materials and Methods

All commercially available reagents were used as supplied without further purification. The reaction solvents were purchased anhydrous and were stored under nitrogen. Unless noted otherwise, the reactions were carried out in oven - dried glassware under atmosphere of nitrogen. Column chromatography was carried out using silica gel (pore size 60 Å, 230 - 400 mesh particle size, 40 - 63 µm particle size). Purification by preparative thin layer chromatography was performed using plates from Merck (PLC Silica gel 60 F₂₅₄, 1 mm). Reverse phase column chromatography was carried out using C₁₈ - reversed phase silica gel (pore size 90 Å, 230 - 400 mesh particle size, 40 - 63 µm particle size). NMR spectra were obtained in indicated deuterated solvents; chemical shifts are quoted in parts per million (*δ*) referenced to the appropriate deuterated solvent employed. Multiplicities are indicated by s (singlet), d (doublet), t (triplet), q (quartet), p (pentet), quin (quintet), sept (septet), m (multiplet) or (br) broad, or combinations thereof. Coupling constant values are given in Hz.

### General procedure A

### General procedure A for Suzuki cross - coupling of 3-bromo-5-chlorothieno[3,2-b]pyridine at position 3.

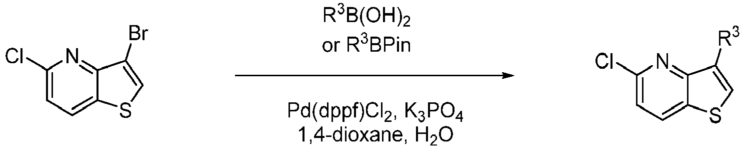

To a degassed solution of 3-bromo-5-chlorothieno[3,2-*b*]pyridine (2.05 mmol), potassium phosphate (6.15 mmol), and boronic acid or ester (2.46 mmol) in a mixture of 1,4-dioxane/H₂O (4:1; 2.45 mL per 0.1 mmol of 3-bromo-5-chlorothieno[3,2-*b*]pyridine) was added [1,1'-Bis(diphenylphosphino)ferrocene]dichloropalladium(II) (0.102 mmol), and the reaction mixture was stirred at 110 °C; the progress of the reaction was followed by TLC. After consumption of the starting material, the mixture was cooled to 25 °C and the solvent was evaporated *in vacuo* and the residue was purified by flash chromatography.

### General procedure B for Suzuki cross - coupling of 3-substituted-5-chlorothieno[3.2-b]pyridine at position 5.

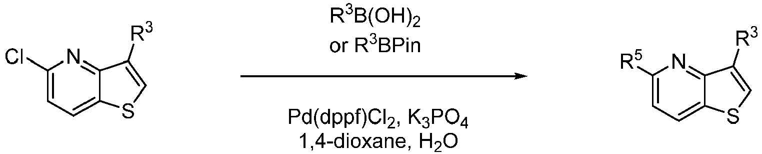

To a degassed solution of 3-substituted-5-chlorothieno[3,2-*b*]pyridine (0.650 mmol), potassium phosphate (1.95 mmol), and boronic acid or ester (0.780 mmol) in a mixture of 1,4-dioxane/H₂O (5: 1; 0.95 mL per 0.1 mmol of 3-substituted-5-chlorothieno[3,2-*b*]pyridine) was added [1,1'-Bis(diphenylphosphino)ferrocene]dichloropalladium(II) (0.031 mmol), and the reaction mixture was stirred at 110 °C (the progress of the reaction was followed by TLC). After consumption of the starting material, the mixture was cooled to 25 °C, and the solvent was evaporated *in vacuo* and the residue was purified by flash chromatography.

### General procedure C for Buchwald cross - coupling of 3-substituted-5-chlorothieno[3,2-b]pyridine at position 5.

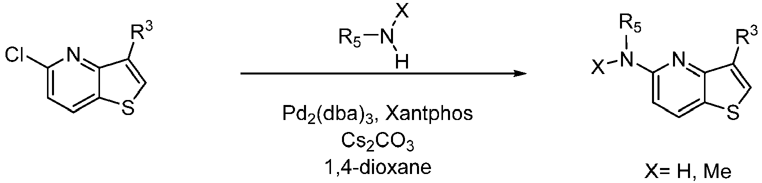

To a degassed solution of 3-substituted-5-chlorothieno[3,2-*b*]pyridine (0.203 mmol), cesium carbonate (0.312 mmol), amine (0.244 mmol) and tris(dibenzylidenacetone)dipalladium (0.008 mmol), 4,5-bis(diphenylphosphino)-9,9-dimethylxanthene (0.016 mmol) in 1,4-dioxane (1 mL), and the reaction mixture was stirred at 110 °C (the progress of the reaction was followed by TLC). After consumption of the starting material, the mixture was cooled to 25 °C, and the solvent was evaporated *in vacuo* and the residue was purified by flash chromatography.

### General procedure D for deprotection of N-Boc-protected compounds.

4 N HCl in dioxane (1.46 mL, 12.3 mmol) was added to a solution of the respective *N*-Boc-protected compound (0.098 mmol) in methanol (2 mL) and the reaction mixture was stirred at 25 °C (the progress of the reaction was followed by TLC). After the time indicated for particular reaction, the solvent was evaporated, the crude was solved in methanol (2 mL) the pH was adjusted to 8 with 7 M NH₃ in methanol (0.25 mL) and the resulting solution was stirred for 30 minutes. The solvent was evaporated *in vacuo* and the residue was purified by flash chromatography or preparative thin layer chromatography.

### Preparative Example 1: 5-chloro-3-phenylthieno[3,2-b]pyridine (intermediate)

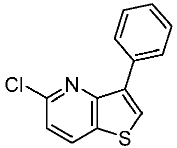

The compound was prepared according to General procedure A using 3-bromo-5-chlorothieno[3,2-*b*]pyridine (256 mg, 1.03 mmol) and 4,4,5,5-tetramethyl-2-phenyl-1,3,2-dioxaborolane (231 mg, 1.13 mmol). Reaction temperature: 50 °C. Reaction time: 1.5 hours. The solvents were evaporated and the residue was purified twice by flash chromatography (hexane/EtOAc, gradient from 100:0 to 90:10; then hexane, 100 %). The product was obtained as a pale yellow solid (273 mg, 55 % yield).
¹H NMR (500 MHz, Chloroform-*d*) *δ* (ppm) 8.14 (d, *J* = 8.4 Hz, 1H), 7.97 (dd, *J* = 8.3, 1.2 Hz, 2H), 7.87 (s, 1H), 7.49 (t, *J* = 7.6 Hz, 2H), 7.40 (t, *J* = 7.4 Hz, 1H), 7.32 (d, *J* = 8.4 Hz, 1H).
¹³C NMR (126 MHz, Chloroform-*d*) *δ* (ppm) 153.00, 149.03, 136.65, 133.79, 132.93, 132.83, 128.62, 128.38, 128.19, 127.98, 119.52.
HRMS calculated for C₁₃H₉ClNS [M+H]⁺ = 246.0139, found [M+H]⁺ = 246.0136.
FTIR (neat), cm⁻¹: 3094, 1598, 1561, 1517, 1482, 1379, 1143, 1122, 816, 797, 748, 717, 692, 643, 592, 558, 543.

### Preparative Example 2: 5-(1-methyl-1H-pyrazol-4-yl)-3-phenylthieno[3.2-b]pyridine

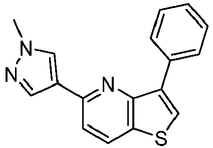

The compound was prepared according to General procedure B using 5-chloro-3-phenylthieno[3,2-b]pyridine (**Prep. Example 1**) (43 mg, 0.175 mmol) and 1-methyl-4-(4,4,5,5-tetramethyl-1,3,2-dioxaborolan-2-yl)-1*H*-pyrazole (42 mg, 0.201 mmol). After 1.5 hours at 70 °C, the reaction was cooled to 25 °C and stirred for additional 1.5 hours. The solvents were evaporated and the residue was purified twice by preparative TLC (hexane/EtOAc, 50:50). The product was obtained as a white solid (25 mg, 49 % yield).
¹H NMR (500 MHz, Chloroform-*d*) *δ* (ppm) 8.16 (d, *J* = 8.4 Hz, 1H), 8.09 (dd, *J* = 8.2, 1.1 Hz, 2H), 8.04 (s, 1H), 7.99 (s, 1H), 7.83 (s, 1H), 7.55 - 7.46 (m, 3H), 7.40 (t, *J=* 7.4 Hz, 1H), 3.98 (s, 3H).
¹³C NMR (126 MHz, Chloroform-*d*) *δ* (ppm) 153.21, 149.50, 137.82, 136.76, 134.72, 131.62, 131.11, 129.09, 128.56, 128.37, 127.56, 126.94, 124.20, 115.42, 39.19.
HRMS calcd. for C₁₇H₁₄N₃S [M+H]⁺ = 292.0903, found [M+H]⁺ = 292.0902.
FTIR (neat), cm⁻¹: 3097, 2927, 1579, 1398, 1382, 1211, 1051, 810, 751, 721.

### Preparative Example 3: 4-(5-chlorothieno[3,2-b]pyridin-3-yl)benzonitrile (intermediate)

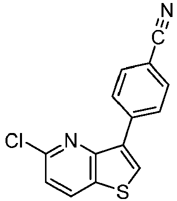

The compound was prepared according to General procedure A using 3-bromo-5-chlorothieno[3,2-*b*]pyridine (100 mg, 0.402 mmol) and 4-(4,4,5,5-tetramethyl-1,3,2-dioxaborolan-2-yl)benzonitrile (110 mg, 0.482 mmol). Reaction temperature: 50 °C. Reaction time: 45 min. The solvents were evaporated and the residue was purified by flash chromatography (hexane/EtOAc, gradient from 100:0 to 80:20). The product was obtained as a pale yellow solid (68 mg, 63 % yield).
¹H NMR (500 MHz, Chloroform-*d*) *δ* (ppm) 8.18 *(d, J=* 8.5 Hz, 1H), 8.14 *(d, J=* 8.5 Hz, 2H), 8.00 (s, 1H), 7.77 (d, *J* = 8.4 Hz, 2H), 7.37 (d, *J* = 8.5 Hz, 1H).
¹³C NMR (126 MHz, Chloroform-*d*) *δ* (ppm) 152.42, 149.41, 138.08, 134.44, 133.13, 132.87, 132.42, 130.17, 128.75, 120.07, 118.92, 111.37.
HRMS (APCI): calcd. for C₁₄H₈ClN₂S [M+H]⁺ = 271.0091, found [M+H]⁺ = 271.0090.
FTIR (neat), cm⁻¹: 3096, 2224, 1604, 1563, 1534, 1378, 1146, 1131, 785, 553.

### Preparative Example 4: 4-(5-(1-methyl-1H-pyrazol-4-yl)thieno[3.2-b]pyridin-3-yl)benzonitrile

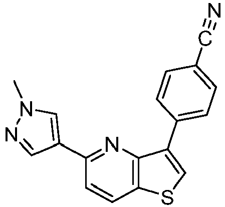

The compound was prepared according to General procedure B using 4-(5-chlorothieno[3,2-b]pyridin-3-yl)benzonitrile (**Prep. Example 3**) (50 mg, 0.185 mmol) and 1-methyl-4-(4,4,5,5-tetramethyl-1,3,2-dioxaborolan-2-yl)-1*H*-pyrazole (46 mg, 0.222 mmol). Reaction time: 75 min. The solvents were evaporated and the residue was purified by flash chromatography (hexane/EtOAc, gradient from 70:30 to 0:100). The product was obtained as a white solid (50 mg, 86 % yield).
¹H NMR (500 MHz, Chloroform-*d*) *δ* (ppm) 8.25 (d, *J* = 8.5 Hz, 2H), 8.19 (d, *J* = 8.5 Hz, 1H), 8.03 (s, 1H), 7.97 (s, 1H), 7.94 (s, 1H), 7.79 (d, *J* = 8.5 Hz, 2H), 7.52 (d, *J* = 8.5 Hz, 1H), 4.00 (s, 3H).
¹³C NMR (126 MHz, Chloroform-*d*) *δ* (ppm) 152.65, 149.90, 139.08, 137.80, 134.64, 132.18, 131.62, 131.32, 129.01, 128.87, 123.85, 119.10, 115.91, 110.89, 39.26.
HRMS (APCI): calcd. for C₁₈H₁₃N₄S [M+H]⁺ = 317.0855, found [M+H]⁺ = 317.0852.
FTIR (neat), cm⁻¹: 2975, 2925, 2224, 1604, 1579, 1379, 1214, 993, 852, 829, 817, 791, 560.

### Preparative Example 5: 5-chloro-3-(4-fluorophenyl)thieno[3,2-b]pyridine (intermediate)

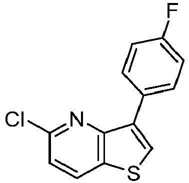

The compound was prepared according to General procedure A using 3-bromo-5-chlorothieno[3,2-b]pyridine (100 mg, 0.402 mmol) and (4-fluorophenyl)boronic acid (67 mg, 0.482 mmol). Reaction time: 45 min. The solvents were evaporated and the residue was purified twice by flash chromatography (hexane/EtOAc, gradient from 100:0 to 85:15; then hexane/EtOAc, gradient from 100:0 to 97:3). The product was obtained as an impure white solid (71 mg, < 67 % yield), which was used without further purification in the next step.
¹H NMR (500 MHz, Chloroform-*d*) *δ* (ppm) 8.14 - 8.10 (m, 2H), 7.98 - 7.92 (m, 1H), 7.81 (s, 1H), 7.24 - 7.13 (m, 3H).
¹³C NMR (126 MHz, Chloroform-*d*) *δ* (ppm) 162.63 (d, *J=* 247.4 Hz), 153.72, 149.04, 132.95, 132.73 (d, *J* = 8.5 Hz), 131.39, 129.99 (d, *J* = 8.1 Hz), 127.90, 126.98, 119.56, 115.51 (d, *J=* 21.4 Hz).
¹⁹F NMR (471 MHz, Chloroform-*d*) *δ* (ppm) -113.9203.
HRMS (APCI): calcd. for C₁₃H₈ClFNS [M+H]⁺ = 264.0045, found [M+H]⁺ = 264.0043.

### Preparative Example 6: 3-(4-fluorophenyl)-5-(1-methyl-1H-pyrazol-4-yl)thieno[3.2-b]pyridine

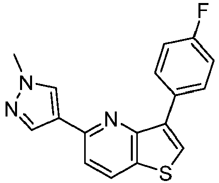

The compound was prepared according to General procedure B using 5-chloro-3-(4-fluorophenyl)thieno[3,2-*b*]pyridine **(Prep. Example 5**) (60 mg, 0.228 mmol) and 1-methyl-4-(4,4,5,5-tetramethyl-1,3,2-dioxaborolan-2-yl)-1*H*-pyrazole (57 mg, 0.273 mmol). After 3 hours, additional potassium phosphate (48 mg, 0.228 mmol), 1-methyl-4-(4,4,5,5-tetramethyl-1,3,2-dioxaborolan-2-yl)-1*H-*pyrazole (24 mg, 0.113 mmol) and [1,1'-bis(diphenylphosphino)ferrocene]dichloropalladium(II) (8.5 mg, 0.011 mmol) were added and the mixture was refluxed for additional 1 hour. The solvents were evaporated and the residue obtained was purified by flash chromatography (hexane/EtOAc, gradient from 50:50 to 0:100). The product was obtained as a white solid (34 mg, 48 % yield).
¹H NMR (500 MHz, Methanol-*d*₄) *δ* (ppm) 8.29 (d, *J* = 8.5 Hz, 1H), 8.19 (s, 1H), 8.18 - 8.12 (m, 2H), 8.09 - 8.06 (m, 1H), 8.05 (s, 1H), 7.63 (d, *J* = 8.5 Hz, 1H), 7.25 - 7.19 (m, 2H), 3.96 (s, 3H).
¹³C NMR (126 MHz, Methanol-*d*₄) *δ* (ppm) 163.80 (d, *J* = 245.4 Hz), 154.15, 150.79, 138.74, 136.42, 133.24, 132.70, 132.41 (d, *J* = 3.5 Hz), 131.32 (d, *J* = 8.0 Hz), 131.03, 128.61, 125.32, 116.77, 116.05 (d, *J* = 21.7 Hz), 39.09.
¹⁹F NMR (471 MHz, Methanol-*d*₄) *δ* (ppm) -117.0295.
HRMS (APCI): calcd. for C₁₇H₁₃FN₃S [M+H]⁺ = 310.0809, found [M+H]⁺ = 310.0810.
FTIR (neat), cm⁻¹: 3097, 2939, 1576, 1542, 1526, 1408, 1397, 1220, 1209, 1159, 1015, 993, 830, 785, 734, 653, 563, 548.

### Preparative Example 7: tert-butyl 4-(5-chlorothieno[3,2-b]pyridin-3-yl)-3,6-dihydropyridine-1(2H)-carboxylate (intermediate)

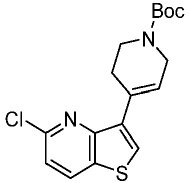

The compound was prepared according to General procedure A using 3-bromo-5-chlorothieno[3,2-b]pyridine (207 mg, 0.833 mmol) and *tert*-butyl 4-(4,4,5,5-tetramethyl-1,3,2-dioxaborolan-2-yl)-3,6-dihydropyridine-1(2*H*)-carboxylate (310 mg, 1.00 mmol). Reaction time: 2.5 hours. The solvents were evaporated and the residue was purified by flash chromatography (hexane/EtOAc, gradient from 100:0 to 80:20). The product was obtained as an impure white solid (215 mg, < 74 % yield), which was used without further purification in the next step.
¹H NMR (500 MHz, Chloroform-*d*) *δ* (ppm) 8.03 (d, *J* = 8.4 Hz, 1H), 7.52 (s, 1H), 7.23 (d, *J* = 8.4 Hz, 1H), 7.16 (br s, 1H), 4.15 (q, *J=* 2.5 Hz, 2H), 3.66 (t, *J* = 5.6 Hz, 2H), 2.77 - 2.47 (m, 2H), 1.48 (s, 9H).
¹³C NMR (126 MHz, Chloroform-*d*) *δ* (ppm) 154.73, 152.83, 148.35, 135.30, 132.79, 132.62, 128.75, 126.44, 124.19, 119.22, 79.50, 31.45, 28.40, 22.52, 13.99.
HRMS (APCI): calcd. for C₁₇H₂₀N₂O₂S [M+H]⁺ = 350.0856, found [M+H]⁺ = not found.
FTIR (neat), cm⁻¹: 2976, 2247, 1681, 1419, 1382, 1364, 1160, 1115, 987, 726, 646.

### Preparative Example 8: tert-butyl 4-(5-(1-methyl-1H-pyrazol-4-yl)thieno[3,2-b]pyridin-3-yl)-3,6-dihydropyridine-1(2H)-carboxylate (intermediate)

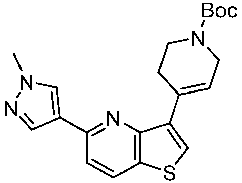

The compound was prepared according to General procedure B using *tert*-butyl 4-(5-chlorothieno[3,2-*b*]pyridin-3-yl)-3,6-dihydropyridine-1(2*H*)-carboxylate (**Prep**. **Example 7**) (126 mg, 0.359 mmol) and 1-methyl-4-(4,4,5,5-tetramethyl-1,3,2-dioxaborolan-2-yl)-1*H*-pyrazole (94 mg, 0.449 mmol). Reaction temperature: 85 °C. Reaction time: 16 hours. The solvents were evaporated and the residue was purified by flash chromatography (hexane/EtOAc, gradient from 90:10 to 50:50). The product was obtained as an impure yellow wax (63 mg, < 44 % yield), which was used without further purification in the next step.
¹H NMR (500 MHz, Chloroform-*d*) *δ* (ppm) 8.11 (d, *J* = 8.3 Hz, 1H), 8.02 (s, 1H), 7.96 (s, 1H), 7.52 (s, 1H), 7.45 (d, *J* = 8.4 Hz, 1H), 7.37 (s, 1H), 4.22 (s, 2H), 4.00 (s, 3H), 3.73 (t, *J* = 5.7 Hz, 2H), 2.73 (s, 2H), 1.51 (s, 9H).
¹³C NMR (126 MHz, Chloroform-*d*) *δ* (ppm) 154.95, 152.98, 148.95, 137.72, 135.91, 131.74, 131.52, 129.80, 129.26, 125.79, 123.71, 115.48, 79.61, 39.25, 28.54, 28.01, 24.86.
HRMS (APCI): calcd. for C₂₁H₂₅N₄O₂S [M+H]⁺ = 397.1693, found [M+H]⁺ = 397.1697.
FTIR (neat), cm⁻¹: 2976, 1685, 1579, 1420, 1402, 1366, 1115, 904, 728, 649.

### Preparative Example 9: 5-(1-methyl-1H-pyrazol-4-yl)-3-(1,2,3,6-tetrahydropyridin-4-yl)-thieno[3.2-b]pyridine

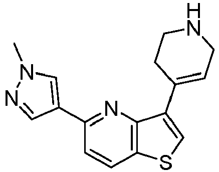

To a solution of *tert*-butyl 4-(5-(1-methyl-1*H*-pyrazol-4-yl)thieno[3,2-*b*]pyridin-3-yl)-3,6-dihydropyridine-1(2*H*)-carboxylate **(Prep. Example 8**) (47 mg, 0.118 mmol) in dichloromethane (2 mL) was added trifluoroacetic acid (144 µL, 1.88 mmol) and the reaction mixture was stirred at 25 °C for 3 hours. The solvents were evaporated and the residue was purified twice by flash chromatography (hexane/EtOAc/methanol/7 M NH₃ in methanol, gradient from 80:20:0:0 to 0:85:13:2; then dichloromethane/methanol, gradient from 95:5 to 80:20). The product was obtained as a white solid (9 mg, 26%).
¹H NMR (500 MHz, Methanol-*d*₄) *δ* (ppm) 8.28 (d, *J=* 8.5 Hz, 1H), 8.26 (s, 1H), 8.10 (s, 1H), 7.93 (s, 1H), 7.64 (d, *J* = 8.5 Hz, 1H), 7.57- 7.54 (m, 1H), 4.00 (br s, 2H), 3.98 (s, 3H), (t, *J* = 6.2 Hz, 2H), 3.04 (br s, 2H).
¹³C NMR (126 MHz, Methanol-*d*₄) *δ* (ppm) 154.30, 150.71, 138.80, 135.04, 133.13, 132.81, 131.28, 131.10, 128.95, 125.20, 119.61, 116.95, 43.52, 42.31, 39.12, 30.73.
HRMS (APCI): calcd. for C₁₆H₁₇N₄S [M+H]⁺ = 297.1168, found [M+H]⁺ = 297.1171.
FTIR (neat), cm⁻¹: 3357, 2955, 2917, 2849, 1703, 1631, 1579, 1463, 1220, 1187, 1080, 967, 831, 820.

### Preparative Example 10: 5-chloro-3-(pyridin-3-yl)thieno[3,2-b]pyridine (intermediate)

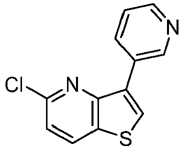

The compound was prepared according to General procedure A using 3-bromo-5-chlorothieno[3,2-*b*]pyridine (200 mg, 0.805 mmol), pyridin-3-ylboronic acid (119 mg, 0.966 mmol) and triethylamine (0.66 mL) instead of potassium phosphate. Reaction time: 3 hours. The solvents were evaporated and the residue was purified by flash chromatography (hexane/EtOAc, gradient from 100:0 to 50:50). The product was obtained as a pale yellow solid (102 mg, 51 % yield).
¹H NMR (500 MHz, Chloroform-*d*) *δ* (ppm) 9.11 (d, *J* = 1.4 Hz, 1H), 8.63 (dd, *J* = 4.8, 1.7 Hz, 1H), 8.41 (dt, *J=* 7.9, 2.0 Hz, 1H), 8.17 (d, *J=* 8.5 Hz, 1H), 7.96 (s, 1H), 7.43 (ddd, *J=* 7.9, 4.8, 0.9 Hz, 1H), 7.35 (d, *J=* 8.5 Hz, 1H).
¹³C NMR (126 MHz, Chloroform-*d*) *δ* (ppm) 152.77, 149.34, 149.03, 148.96, 135.62, 133.22, 133.05, 132.74, 129.74, 128.89, 123.45, 119.92.
HRMS calcd. for C₁₂H₈ClN₂S [M+H]⁺ = 247.0091, found [M+H]⁺ = 247.0090.
FTIR (neat), cm⁻¹: 3095, 3028, 1563, 1533, 1438, 1130, 780, 701, 450.

### Preparative Example 11: 5-(1-methyl-1H-pyrazol-4-yl)-3-(pyridin-3-yl)thieno[3,2-b]pyridine

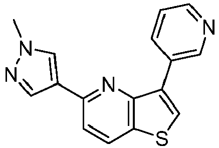

The compound was prepared according to General procedure B using 5-chloro-3-(pyridin-3-yl)thieno[3,2-b]pyridine **(Prep. Example 10)** (45 mg, 0.182 mmol) and 1-methyl-4-(4,4,5,5-tetramethyl-1,3,2-dioxaborolan-2-yl)-1*H*-pyrazole (46 mg, 0.219 mmol). Reaction time: 8 hours. The solvents were evaporated and the residue was purified by flash chromatography (hexane/EtOAc, gradient from 90:10 to 0:100). The product was obtained as a white solid (43 mg, 82 % yield).
¹H NMR (500 MHz, Chloroform-*d*) *δ* (ppm) 9.34 (d, *J* = 2.2 Hz, 1H), 8.63 (dd, *J* = 4.9, 1.7 Hz, 1H), 8.41 (dt, *J=* 7.7, 2.0 Hz, 1H), 8.18 (d, *J* = 8.5 Hz, 1H), 8.02 (s, 2H), 7.91 (s, 1H), 7.52 (d, *J=* 8.4 Hz, 1H), 7.44 (ddd, *J* = 8.0, 4.7, 0.9 Hz, 1H), 3.98 (s, 3H).
¹³C NMR (126 MHz, Chloroform-*d*) *δ* (ppm) 152.93, 149.87, 149.44, 148.51, 137.73, 135.58, 133.43, 131.48, 131.25, 130.59, 129.18, 127.72, 123.89, 123.32, 115.76, 39.22.
HRMS calculated for C₁₆H₁₃N₄S [M+H]⁺ = 293.0855, found [M+H]⁺ = 293.0858.
FTIR (neat), cm⁻¹: 3076, 2923, 1579, 1397, 1369, 1224, 993, 826, 788, 619, 557.

### Preparative Example 12: 5-chloro-3-(pyridin-4-yl)thieno[3.2-b]pyridine (intermediate)

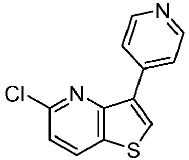

The compound was prepared according to General procedure A using 3-bromo-5-chlorothieno[3,2-b]pyridine (400 mg, 1.61 mmol) and pyridin-4-ylboronic acid (237 mg, 1.93 mmol). Reaction time: 1.5 hours. The solvents were evaporated and the residue was purified by flash chromatography (cyclohexane/EtOAc, gradient from 70:30 to 40:60). The product was obtained as a brown solid (632 mg, 80 % yield).
¹H NMR (500 MHz Chloroform-*d*) *δ* (ppm) 8.72 (dd, *J* = 4.7, 1.4 Hz, 2H), 8.17 (d, *J* = 8.4 Hz, 1H), 8.08 (s, 1H), 7.96 (dd, *J* = 4.5, 1.7 Hz, 2H), 7.36 (d, *J* = 8.5 Hz, 1H).
¹³C NMR (126 MHz, Chloroform-*d*) *δ* (ppm) 152.50, 150.21, 149.39, 140.83, 133.51, 133.07, 132.88, 130.61, 122.40, 120.04.
HRMS calcd. for C₁₂H₈ClN₂S [M+H]⁺ = 247.0091, found [M+H]⁺ = 247.0089.
FTIR (neat), cm⁻¹: 3093, 1597, 1556, 1383, 1150, 1127, 793, 642, 548, 491.

### Preparative Example 13: 5-(1-methyl-1H-pyrazol-4-yl)-3-(pyridin-4-yl)thieno[3.2-b]pyridine

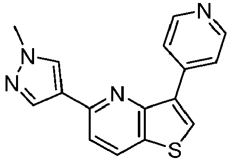

The compound was prepared according to General procedure B using 5-chloro-3-(pyridin-4-yl)thieno[3,2-b]pyridine **(Prep. Example 12**) (160 mg, 0.650 mmol) and 1-methyl-4-(4,4,5,5-tetramethyl-1,3,2-dioxaborolan-2-yl)-1*H*-pyrazole (162 mg, 0.780 mmol). Reaction time: 1 hour. The solvents were evaporated and the residue was purified by flash chromatography (cyclohexane/EtOAc/methanol, gradient from 80:20:0 to 0:95:5). The product was obtained as a white solid (172 mg, 90 % yield).
¹H NMR (500 MHz, Chloroform-*d*) *δ* (ppm) 8.73 (dd, *J* = 4.6, 1.7 Hz, 2H) 8.18 (d, *J* = 8.4 Hz, 1H), 8.08 (dd, *J* = 4.6, 1.7 Hz, 2H). 8.04 (d, *J* = 0.7 Hz, 1H), 8.03 (s, 1H), 8.00 (s, 1H), 7.52 (d, *J* = 8.5 Hz, 1H), 4.00 (s, 3H).
¹³C NMR (126 MHz, Chloroform-*d*) *δ* (ppm) 152.82, 150.00, 149.94, 141.82, 137.84, 133.72, 131.62, 131.27, 129.44, 129.06, 123.90, 122.65, 115.89, 39.25.
HRMS calcd. for C₁₆H₁₃N₄S [M+H]⁺ = 293.0855, found [M+H]⁺ = 293.0857.
FTIR (neat), cm⁻¹: 3076, 2923, 1595, 1579, 15487, 1510, 1397, 1369, 1308, 1224, 1171, 1052, 993, 981, 826, 788, 721, 699, 638, 619, 589, 557.

### Preparative Example 14: 5-chloro-3-(2-methylpyridin-4-yl)thieno[3,2-b]pyridine (intermediate)

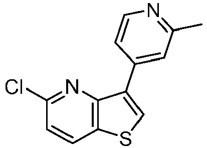

The compound was prepared according to General procedure A using 3-bromo-5-chlorothieno[3,2-*b*]pyridine (250 mg, 1.01 mmol) and (2-methylpyridin-4-yl)boronic acid (166 mg, 1.21 mmol). Reaction time: 48 hours. The solvents were evaporated and the residue was purified by flash chromatography (cyclohexane/EtOAc, gradient from 90:10 to 50:50). The product was obtained as a white solid (39 mg, 15 % yield).
¹H NMR (500 MHz, Chloroform-*d*) *δ* (ppm) 8.61 (d, *J=* 5.1 Hz, 1H), 8.17 (d, *J=* 8.5 Hz, 1H), 8.06 (s, 1H), 7.81 (s, 1H), 7.78 (d, *J* = 5.0 Hz, 1H), 7.36 (d, *J* = 8.54 Hz, 1H), 2.67 (s, 3H).
¹³C NMR (126 MHz, Chloroform-*d*) *δ* (ppm) 158.67, 152.58, 149.40, 149.32, 141.41, 133.83, 133.07, 132.87, 130.55, 122.03, 120.03, 119.77, 24.56.
HRMS (APCI): calcd. for C₁₃H₁₀ClN₂S [M+H]⁺ = 261.0248, found [M+H]⁺ = 261.0251.
FTIR (neat), cm⁻¹: 2954, 2922, 2852, 1602, 1560, 1531, 1380, 1146, 1146, 1124, 967, 847, 833, 794, 707, 658.

### Preparative Example 15: 5-(1-methyl-1H-pyrazol-4-yl)-3-(2-methylpyridin-4-yl)thieno[3.2-b]pyridine

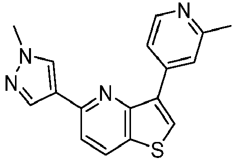

The compound was prepared according to General procedure B using 5-chloro-3-(2-methylpyridin-4-yl)thieno[3,2-b]pyridine **(Prep. Example 14)** (35 mg, 0.134 mmol) and 1-methyl-4-(4,4,5,5-tetramethyl-1,3,2-dioxaborolan-2-yl)-1*H*-pyrazole (35 mg, 0.168 mmol). Reaction temperature: 85 °C. Reaction time: 16 hours. The solvents were evaporated and the residue was purified by flash chromatography (cyclohexane/EtOAc/methanol, gradient from 70:30:0 to 0:90:10). The product was obtained as a white solid (29 mg, 71 % yield).
¹H NMR (500 MHz, Chloroform-*d*) *δ* (ppm) 8.62 (d, *J* = 5.3 Hz, 1H), 8.16 (d, *J* = 8.4 Hz, 1H), 8.04 (s, 1H), 8.00 (s, 1H), 7.97 (s, 1H), 7.95 (s, 1H), 7.89 (d, *J* = 5.3 Hz, 1H), 7.50 (d, *J* = 8.5 Hz, 1H), 3.99 (s, 3H), 2.67 (s, 3H).
¹³C NMR (126 MHz, Chloroform-*d*) *δ* (ppm) 158.37, 152.86, 149.84, 149.08, 142.25, 137.85, 133.88, 131.59, 131.23, 129.32, 128.98, 123.92, 122.22, 119.92, 115.81, 39.24, 24.57.
HRMS (APCI): calcd. for C₁₇H₁₅N₄S [M+H]⁺ = 307.1012, found [M+H]⁺ = 307.1015.
FTIR (neat), cm⁻¹: 3096, 2923, 1603, 1578, 1537, 1376, 1215, 1167, 984, 834, 790, 700, 655.

### Preparative Example 16: 5-chloro-3-(2-methoxypyridin-4-yl)thieno[3,2-b]pyridine (intermediate)

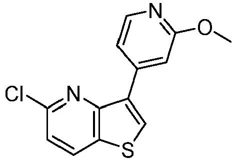

The compound was prepared according to General procedure A using 3-bromo-5-chlorothieno[3,2-*b*]pyridine (250 mg, 1.01 mmol) and 2-methoxy-4-(4,4,5,5-tetramethyl-1,3,2-dioxaborolan-2-yl)pyridine (185 mg, 1.21 mmol). Reaction time: 21 hours. The solvents were evaporated and the residue was purified by flash chromatography (cyclohexane/EtOAc, gradient from 100:0 to 90:10). The product was obtained as a white solid (154 mg, 55 % yield).
¹H NMR (500 MHz, Chloroform-*d*) *δ* (ppm) 8.27 (d, *J=* 5.3 Hz, 1H), 8.16 (d, *J=* 8.5 Hz, 1H), 8.05 (s, 1H), 7.51 (dd, *J* = 5.4, 1.4 Hz, 1H), 7.48 (s, 1H), 7.35 (d, *J=* 8.5 Hz, 1H), 4.01 (s, 3H).
¹³C NMR (126 MHz, Chloroform-*d*) *δ* (ppm) 164.76, 152.57, 149.38, 147.05, 143.56, 133.67, 133.02, 132.84, 130.57, 120.00, 116.05, 109.57, 53.60.
HRMS (APCI): calcd. for C₁₃H₁₀ClN₂OS [M+H]⁺ = 277.0197, found [M+H]⁺ = 277.0199.
FTIR (neat), cm⁻¹: 3085, 1606, 1552, 1479, 1373, 1327, 1307, 1204, 1165, 1152, 1056, 1042, 977, 886, 849, 795, 735, 719, 651, 457, 448.

### Preparative Example 17: 3-(2-methoxypyridin-4-yl)-5-(1-methyl-1H-pyrazol-4-yl)thieno[3.2-b]pyridine

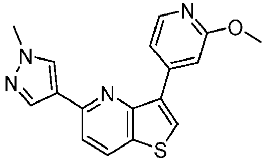

The compound was prepared according to General procedure B using 5-chloro-3-(2-methoxypyridin-4-yl)thieno[3,2-*b*]pyridine (**Prep. Example 16**) (50 mg, 0.181 mmol) and 1-methyl-4-(4,4,5,5-tetramethyl-1,3,2-dioxaborolan-2-yl)-1*H*-pyrazole (47 mg, 0.226 mmol). Reaction temperature: 85 °C. Reaction time: 3.5 hours. The solvents were evaporated and the residue was purified by flash chromatography (cyclohexane/EtOAc, gradient from 80:20 to 50:50). The product was obtained as a white solid (56 mg, 96 % yield).
¹H NMR (500 MHz, Chloroform-*d*) *δ* (ppm) 8.28 (d, *J* = 5.3 Hz, 1H), 8.17 (d, *J* = 8.44, 1H), 8.00 (s, 1H), 8.00 (d, *J* = 2.7 Hz, 2H), 7.66 (s, 1H), 7.61 (d, *J* = 5.2 Hz, 1H), 7.51 (d, *J* = 8.5 Hz, 1H), 4.03 (s, 3H), 4.00 (s, 3H).
¹³C NMR (126 MHz, Chloroform-*d*) *δ* (ppm) 164.76, 152.87, 149.88, 146.67, 144.48, 137.86, 133.72, 131.58, 131.21, 129.44, 129.13, 123.91, 116.26, 115.81, 109.69, 53.58, 39.27.
HRMS (APCI): calcd. for C₁₇H₁₅N₄OS [M+H]⁺ = 323.0961, found [M+H]⁺ = 323.0963.
FTIR (neat), cm⁻¹: 2942, 1604, 1577, 1536, 1481, 1458, 1393, 1374, 1328, 1306, 1281, 1214, 1197, 1164, 1054, 1041, 996, 838, 819, 789, 724, 651, 612.

### Preparative Example 18: 4-(5-(1-methyl-1H-pyrazol-4-yl)thieno[3,2-b]pyridin-3,2-b]pyridin-2-ol

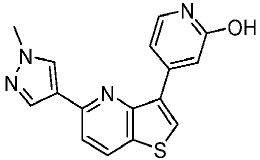

HCl conc. (0.7 mL) was added to 3-(2-methoxypyridin-4-yl)-5-(1-methyl-1*H*-pyrazol-4-yl)thieno[3,2-b]pyridine (**Prep. Example 17**) (16 mg, 0.048 mmol) and the reaction mixture was stirred at 120 °C for 2 hours. The reaction was quenched with 6 M NaOH until pH 7 and extracted with EtOAc (2 × 10 mL). The combined organic extracts were washed with brine (10 mL), dried over MgSO₄, filtered, and the solvent was evaporated *in vacuo.* The solvents were evaporated and the residue was purified by flash chromatography (dichloromethane/methanol, gradient from 100:0 to 85:15). The product was obtained as a white solid (7 mg, 47 %).
¹H NMR (500 MHz, DMSO-*d₆*) *δ* (ppm) 11.51 (br s, 1H), 8.64 (s, 1H), 8.51 (d, *J* = 8.5 Hz, 1H), 8.33 (s, 1H), 8.08 (s, 1H), 7.74 (d, *J* = 8.4 Hz, 1H), 7.49 (s 1H), 7.46 (d, *J* = 6.9 Hz, 1H), 7.01 (dd, *J* = 6.9, 1.8 Hz, 1H), 3.93 (s, 3H).
¹³C NMR (126 MHz, DMSO-*d₆*) *δ* (ppm) 162.79, 152.26, 149.56, 145.31, 137.31, 134.71, 132.45, 132.22, 131.50, 131.04, 129.61, 122.86, 117.23, 115.90, 104.71, 38.78.
HRMS (APCI): calcd. for C₁₆H₁₃N₄OS [M+H]⁺ = 309.0805, found [M+H]⁺ = 309.0803.
FTIR (neat), cm⁻¹: 3383, 3089, 2929, 1641, 1579, 1566, 1541, 1493, 1434, 1216, 990, 982, 808, 786, 509, 429.

### Preparative Example 19: 4-(5-chlorothieno[3,2-b]pyridin-3-yl)picolinonitrile (intermediate)

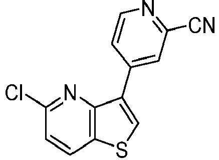

The compound was prepared according to General procedure A using 3-bromo-5-chlorothieno[3,2-*b*]pyridine (25 mg, 0.100 mmol) and 4-(4,4,5,5-tetramethyl-1,3,2-dioxaborolan-2-yl)-picolinonitrile (29 mg, 0.125 mmol). Reaction temperature: 80 °C. Reaction time: 3 hours. The solvents were evaporated and the residue was purified by flash chromatography (cyclohexane/EtOAc, gradient from 100:0 to 70:30). The product was obtained as a white solid (24 mg, 89 % yield).
¹H NMR (500 MHz, Chloroform-*d*) *δ* (ppm) 8.82 (d, *J* = 5.1 Hz, 1H), 8.45 - 8.38 (m, 1H), 8.27 (dd, *J=* 5.2, 1.7 Hz, 1H), 8.22 (s, 1H), 8.20 (s, 1H), 7.42 (d, *J* = 8.5 Hz, 1H).
¹³C NMR (126 MHz, Chloroform-*d*) *δ* (ppm) 152.03, 151.51, 149.87, 142.19, 134.48, 133.27, 132.90, 132.00, 131.21, 127.05, 125.22, 120.61, 117.43.
HRMS (APCI): calcd. for C₁₃H₇ClN₃S [M+H]⁺ = 272.0044, found [M+H]⁺ = 272.0046.
FTIR (neat), cm⁻¹: 3094, 2924, 2239, 1596, 1564, 1550, 1379, 1148, 1115, 992, 868, 849, 664.

### Preparative Example 20: 4-(5-(1-methyl-1H-pyrazol-4-yl)thieno[3,2-b]pyridin-3-yl)picolino-nitrile

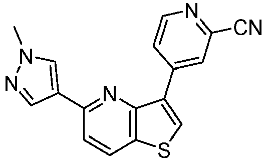

The compound was prepared according to General procedure B using 4-(5-chlorothieno[3,2-*b*]pyridin-3-yl)picolinonitrile (**Prep. Example 19**) (24 mg, 0.086 mmol) and 1-methyl-4-(4,4,5,5-tetramethyl-1,3,2-dioxaborolan-2-yl)-1*H*-pyrazole (22 mg, 0.108 mmol). Reaction temperature: 75 °C. Reaction time: 2 hours. The solvents were evaporated and the residue was purified by flash chromatography (cyclohexane/EtOAc, gradient from 80:20 to 50:50). The product was obtained as a white solid (9 mg, 34 % yield).
¹H NMR (500 MHz, Chloroform-*d*) *δ* (ppm) 8.81 (d, *J* = 5.2 Hz, 1H), 8.67 (dd, *J* = 1.9, 0.8 Hz, 1H), 8.29 (dd, *J* = 5.2, 1.8 Hz, 1H), 8.22 (d, *J* = 8.5 Hz, 1H), 8.14 (s, 1H), 8.05 (s, 1H), 8.02 (s, 1H), 7.57 (d, *J* = 8.5 Hz, 1H), 4.03 (s, 3H).
¹³C NMR (126 MHz, Chloroform-*d*) *δ* (ppm) 152.34, 151.25, 150.34, 143.13, 137.76, 134.27, 131.65, 131.53, 131.31, 130.92, 129.18, 127.46, 125.24, 123.56, 117.63, 116.38, 39.37.
HRMS (APCI): calcd. for C₁₇H₁₂N₅S [M+H]⁺ = 318.0808, found [M+H]⁺ = 318.0810.
FTIR (neat), cm⁻¹: 2924, 2853, 1726, 1595, 1578, 1464, 1426, 1377, 1262, 1129.

### Preparative Example 21: 5-chloro-3-(2-fluoropyridin-4-yl)thieno[3,2-b]pyridine (intermediate)

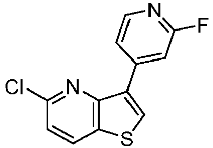

The compound was prepared according to General procedure A using 3-bromo-5-chlorothieno[3,2-*b*]pyridine (130 mg, 0.523 mmol) and 2-fluoro-4-(4,4,5,5-tetramethyl-1,3,2-dioxaborolan-2-yl)pyridine (88 mg, 0.630 mmol). Reaction time: 3 hours. The solvents were evaporated and the residue was purified twice by flash chromatography (cyclohexane/EtOAc, gradient from 80:20 to 60:40; then cyclohexane/EtOAc, gradient from 95:5 to 50:50). The product was obtained as an impure white solid (64 mg, <32 % yield), which was used without further purification in the next step.
¹H NMR (500 MHz, Chloroform-*d*) *δ* (ppm) 8.32 (d, *J* = 5.3 Hz, 1H), 8.19 (d, *J* = 8.5 Hz, 1H), 8.14 (s, 1H), 7.85 (dt, *J* = 5.2, 1.7 Hz, 1H), 7.72 (s, 1H), 7.39 (d, *J* = 8.5 Hz, 1H).
¹³C NMR (126 MHz, Chloroform-*d*) *δ* (ppm) 164.39 (d, *J* = 237.6 Hz), 152.27, 149.62, 148.92 (d, *J* = 15.2 Hz), 147.89 (d, *J=* 15.2 Hz), 133.15, 132.87, 132.35 (d, *J* = 3.6 Hz), 131.46, 120.31, 120.10 (d, *J* = 4.1 Hz), 108.08 (d, *J* = 39.0 Hz).
¹⁹F NMR (471 MHz, Chloroform-*d*) *δ* (ppm) -67.4964
HRMS (APCI): calcd. for C₁₂H₇ClFN₂S [M+H]⁺ = 264.9997, found [M+H]⁺ = 265.0001.
FTIR (neat), cm⁻¹: 3079, 1612, 1562, 1378, 1146, 1123, 992, 850, 835, 812, 652, 564.

### Preparative Example 22: 3-(2-fluoropyridin-4-yl)-5-(1-methyl-1H-pyrazol-4-yl)thieno[3,2-b]pyridine

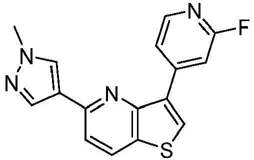

The compound was prepared according to General procedure B using 5-chloro-3-(2-fluoropyridin-4-yl)thieno[3,2-b]pyridine (**Prep. Example 21**) (64 mg, 0.242 mmol) and 1-methyl-4-(4,4,5,5-tetramethyl-1,3,2-dioxaborolan-2-yl)-1*H*-pyrazole (60 mg, 0.290 mmol). Reaction time: 3 hours. The solvents were evaporated and the residue was purified by flash chromatography (cyclohexane/EtOAc, gradient from 90:10 to 50:50). The product was obtained as a white solid (48 mg, 64 % yield).
¹H NMR (500 MHz, Chloroform-*d*) *δ* (ppm) 8.31 (d, *J=* 5.2 Hz, 1H), 8.17 (d, *J=* 8.5 Hz, 1H), 8.07 (s, 1H), 8.03 (s, 1H), 7.99 (s, 1H), 7.94 (s, 1H), 7.88 (d, *J=* 5.2 Hz, 1H), 7.52 (d, *J* = 8.5 Hz, 1H), 4.00 (s, 3H). ¹³C NMR (126 MHz, Chloroform-*d*) *δ* (ppm) 164.37 (d, *J* = 236.9 Hz), 152.55, 150.10, 147.48 (d, *J* = 15.5 Hz), 147.01 (d, *J* = 9.0 Hz), 137.81, 132.36 *(d, J=* 3.6 Hz), 131.56, 131.32, 130.33, 129.10, 123.71, 120.12 (d, *J* = 3.9 Hz), 116.06, 108.13 (d, *J* = 39.2 Hz). 39.28.
¹⁹F NMR (471 MHz, Chloroform-*d*) *δ* (ppm) -68.0315
HRMS (APCI): calcd. for C₁₆H₁₂FN₄S [M+H]⁺ = 311.0761, found [M+H]⁺ = 311.0758.
FTIR (neat), cm⁻¹: 3058, 1607, 1577, 1552, 1513, 1475, 1398, 1377, 1214, 1158, 992, 972, 887, 828, 807, 791, 699, 648, 612, 547, 489, 455, 420.

### Preparative Example 23: tert-butyl (4-(5-chlorothieno[3.2-b]pyridin-3-yl)pyridin-2-yl)-carbamate (intermediate)

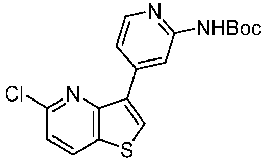

The compound was prepared according to General procedure A using 3-bromo-5-chlorothieno[3,2-b]pyridine (150 mg, 0.603 mmol), *tert*-butyl (4-(4,4,5,5-tetramethyl-1,3,2-dioxaborolan-2-yl)pyridin-2-yl) carbamate (232 mg, 0.724 mmol) and triethylamine (300 µL, 2.20 mmol) instead of potassium phosphate. Reaction time: 1.5 hours. The solvents were evaporated and the residue was purified by flash chromatography (cyclohexane/EtOAc, gradient from 100:0 to 80:20). The product was obtained as a white solid (158 mg, 79 % yield).
¹H NMR (500 MHz, Chloroform-*d*) *δ* (ppm) 8.41 (dd, *J* = 1.6, 0.8 Hz, 1H), 8.37 (dd, *J* = 5.2, 0.8 Hz, 1H), 8.17 - 8.14 (m, 2H), 7.86 (dd, *J* = 5.3, 1.6 Hz, 1H), 7.59 (br s, 1H), 7.35 (d, *J* = 8.5 Hz, 1H), 1.55 (s, 9H).
¹³C NMR (126 MHz, Chloroform-*d*) *δ* (ppm) 152.65, 152.46, 152.39, 149.36, 148.31, 143.12, 133.97, 133.03, 132.84, 131.14, 119.92, 117.96, 110.66, 81.02, 28.33.
HRMS (APCI): calcd. for C₁₇H₁₇ClN₃O₂S [M+H]⁺ = 362.0725, found [M+H]⁺ = 362.0723.
FTIR (neat), cm⁻¹: 2977, 1715, 1608, 1565, 1381, 1293, 1160, 1124, 844, 810, 766, 736, 658.

### Preparative Example 24: tert-butyl (4-(5-(1-methyl-1H-pyrazol-4-yl)thieno[3,2-b]pyridin-3-yl)pyridin-2-yl)carbamate

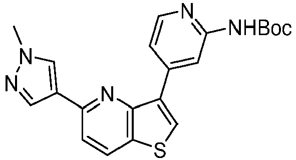

The compound was prepared according to General procedure B using tert-butyl (4-(5-chlorothieno[3,2-*b*]pyridin-3-yl)pyridin-2-yl)carbamate **(Prep. Example 22**) (100 mg, 0.276 mmol) and 1-methyl-4-(4,4,5,5-tetramethyl-1,3,2-dioxaborolan-2-yl)-1*H*-pyrazole (69 mg, 0.331 mmol). Reaction time: 16 hours. The solvents were evaporated and the residue was purified by flash chromatography (cyclohexane/EtOAc, gradient from 70:30 to 0:100). The product was obtained as a white solid (52 mg, 46 % yield).
¹H NMR (500 MHz, Chloroform-*d*) *δ* (ppm) 9.09 (s, 1H), 8.45 (s, 1H), 8.36 - 8.31 (m, 1H), 8.17 (d, *J=* 8.5 Hz, 1H), 8.11 (s, 1H), 8.08 - 8.03 (m, 1H), 7.76 (s, 1H), 7.62 (dd, *J* = 5.3, 1.5 Hz, 1H), 7.54 (d, *J=* 8.5 Hz, 1H), 3.99 (s, 3H), 1.60 (s, 9H).
¹³C NMR (126 MHz, Chloroform-*d*) *δ* (ppm) 152.93, 152.50, 152.44, 150.24, 147.81, 143.87, 137.78, 133.63, 131.56, 131.21, 130.11, 129.80, 124.03, 117.37, 115.87, 111.04, 80.65, 39.22, 28.39.
HRMS (APCI): calcd. for C₂₁H₂₂N₅O₂S [M+H]⁺ = 408.1489, found [M+H]⁺ = 408.1488.
FTIR (neat), cm⁻¹: 2976, 1720, 1606, 1579, 1539, 1271, 1220, 1159, 731.

### Preparative Example 25: 4-(5-(1-methyl-1H-pyrazol-4-yl)thieno[3,2-b]pyridin-3-yl)pyridin-2-amine

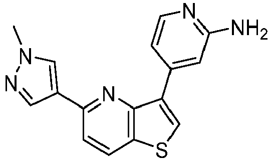

The compound was prepared according to General procedure D using *tert*-butyl (4-(5-(1-methyl-1*H-*pyrazol-4-yl)thieno[3,2-*b*]pyridin-3-yl)pyridin-2-yl)carbatnate (**Prep**. **Example 24**) (40 mg, 0.098 mmol). Reaction time: 6 hours. The solvent was evaporated and the residue was purified by preparative TLC (dichloromethane/methanol/7M NH₃ in methanol, 93:5:2; then EtOAc, 100 %). The product was obtained as a pale yellow solid (13 mg, 43 % yield).
¹H NMR (500 MHz, Methanol-*d*₄) *δ* (ppm) 8.31 (d, *J* = 8.5 Hz, 1H), 8.29 (s, 1H), 8.27 (s, 1H), 8.15 - 8.10 (m, 1H), 7.99 (dd, *J* = 5.6, 0.5 Hz, 1H), 7.66 (d, *J* = 8.5 Hz, 1H), 7.60 - 7.58 (m, 1H), 7.35 (dd, *J* = 5.6, 1.5 Hz, 1H), 3.98 (s, 3H).
¹³C NMR (126 MHz, Methanol-*d*₄) *δ* (ppm) 161.04, 154.09, 151.11, 147.53, 145.51, 138.88, 134.79, 133.28, 132.80, 131.58, 131.25, 125.21, 117.04, 113.66, 109.24, 39.11.
HRMS (APCI): calcd. for C₁₆H₁₄N₅S [M+H]⁺ = 308.0964, found [M+H]⁺ = 308.0964.
FTIR (neat), cm⁻¹: 2284, 2093, 2045, 1606, 1577, 1536, 1421, 1399, 1215, 1169, 992, 865-792, 665.

### Preparative Example 26: tert-butyl ((4-(5-chlorothieno[3,2-b]pyridin-3-yl)pyridin-2-yl)-methyl)carbamate (intermediate)

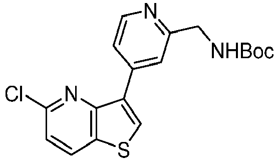

The compound was prepared according to General procedure A using 3-bromo-5-chlorothieno[3,2-*b*]pyridine (496 mg, 1.99 mmol), *tert*-butyl ((4-(4,4,5,5-tetramethyl-1,3,2-dioxaborolan-2-yl)pyridin-2-yl)methyl)carbamate (800 mg, 2.39 mmol) and triethylamine (1.3 mL, 9.33 mmol) instead of potassium phosphate. After 3 hours, additional triethylamine (0.2 mL, 1.44 mmol) was added and the mixture was refluxed for additional 5 hours. The solvents were evaporated and the residue was purified by flash chromatography (cyclohexane/EtOAc, gradient from 50:50 to 0:100). The product was obtained as a pale yellow solid (679 mg, 91 % yield).
¹H NMR (500 MHz, Chloroform-*d*) *δ* (ppm) 8.64 (dd, *J* = 5.2, 0.6 Hz, 1H), 8.17 (d, *J* = 8.5 Hz, 1H), 8.08 (s, 1H), 7.91 (dd, *J* = 5.2, 1.3 Hz, 1H), 7.88 (s, 1H), 7.36 (d, *J =* 8.5 Hz, 1H), 5.66 (s, 1H), 4.53 (d, *J* = 5.3 Hz, 2H), 1.47 (s, 9H).
¹³C NMR (126 MHz, Chloroform-*d*) *δ* (ppm) 157.88, 156.04, 152.50, 149.54, 149.43, 141.67, 133.48, 133.08, 132.85, 130.81, 121.19, 120.41, 120.07, 75.01, 45.95, 28.43.
HRMS (APCI): calcd. for C₁₈H₁₉ClN₃O₂S [M+H]⁺ = 376.0881, found [M+H]⁺ = 376.0878.
FTIR (neat), cm⁻¹: 3343, 2977, 1697, 1604, 1509, 1381, 1366, 1247, 1164, 1146, 1126, 849, 731, 658.

### Preparative Example 27: (4-(5-(1-methyl-1H-pyrazol-4-yl)thieno[3,2-b]pyridin-3-yl)pyridin-2-yl)methanamine

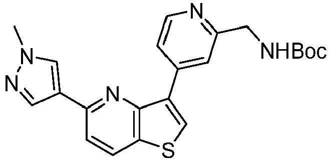

The compound was prepared according to General procedure B using (4-(5-chlorothieno[3,2-*b*]pyridin-3-yl)pyridin-2-yl)methanamine **(Prep. Example 26**) (80 mg (0.213 mmol), 1-methyl-4-(4,4,5,5-tetramethyl-1,3,2-dioxaborolan-2-yl)-1*H*-pyrazole (53 mg, 0.256 mmol) and triethylamine (90 µL, 0.256 mmol) instead of potassium phosphate. After 2 hours, additional triethylamine (1 mL, 7.17 mmol), [1,1'-bis(diphenylphosphino)ferrocene]dichloro-palladium(II) (15 mg, 0.017 mmol) and tripotassium phosphate (45 mg, 0.213 mmol) were added and the reaction mixture was refluxed for additional 7 hours. The solvents were evaporated and the residue was purified by flash chromatography (cyclohexane/EtOAc/methanol, gradient from 80:20:0 to 95:0:5) and preparative TLC (EtOAc - 1 elution, then cyclohexane/EtOAc, 60:40 - 1 elution). The product was obtained as a white solid (32 mg, 36 % yield).
¹H NMR (500 MHz, Chloroform-*d*) *δ* (ppm) 8.65 (dd, *J* = 5.2, 0.8 Hz, 1H), 8.18 (d, *J* = 8.5 Hz, 1H), 8.15 (s, 1H), 8.11 (s, 1H), 8.03 (s, 1H), 8.02 (d, *J* = 0.7 Hz, 1H), 7.94 (dd, *J* = 5.2, 1.7 Hz, 1H), 7.53 (d, *J* = 8.5 Hz, 1H), 5.60 (s, 1H), 4.57 (d, *J* = 5.6 Hz, 2H), 4.02 (s, 3H), 1.45 (s, 9H).
¹³C NMR (126 MHz, Chloroform-*d*) *δ* (ppm) 157.76, 156.05, 152.81, 150.07, 149.27, 142.60, 137.72, 133.59, 131.56, 131.28, 129.57, 129.44, 123.76, 121.29, 120.85, 115.89, 79.45, 46.15, 39.25, 28.41.
HRMS (APCI): calcd. for C₂₂H₂₄N₅O₂S [M+H]⁺ = 422.1645, found [M+H]⁺ = 422.1643.
FTIR (neat), cm⁻¹: 2976, 1698, 1602, 1578, 1365, 1266, 1246, 1163, 1048, 859, 791, 732, 699, 654.

### Preparative Example 28: (4-(5-(1-methyl-1H-pyrazol-4-yl)thieno[3.2-b]pyridin-3-yl)pyridin-2-yl)methanamine

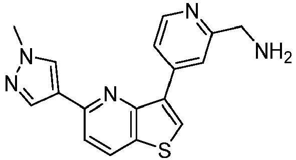

The compound was prepared according to General procedure D using (4-(5-(1-methyl-1*H*-pyrazol-4-yl)thieno[3,2-*b*]pyridin-3-yl)pyridin-2-yl)methanamine (**Prep. Example 27**) (18 mg, 0.047 mmol). After 1 hour at 25 °C, additional amount of 4 N HCl in dioxane (68 µL, 0.188 mmol) and methanol (1 mL) were added and the reaction mixture was stirred for additional 5 hours at 40 °C. The solvents were evaporated and the residue was purified by preparative TLC (dichloromethane/methanol/ 7 M NH₃ in methanol, 88:10:2 - 2 elutions). The product was obtained as a brown solid (11 mg, 73 % yield).
¹H NMR (500 MHz, Methanol-*d₄*) *δ* (ppm) 8.75 (dd, *J* = 5.2, 0.9 Hz, 1H), 8.50 (s, 1H), 8.38 (d, *J* = 8.5 Hz, 1H), 8.35 (s, 1H), 8.32 (dd, *J* = 5.3, 1.8 Hz, 1H), 8.28 (s, 1H), 8.13 (d, *J* = 0.7 Hz, 1H), 7.71 (d, *J* = 8.5 Hz, 1H), 4.41 (s, 2H), 3.99 (s, 3H).
¹³C NMR (126 MHz, Methanol-*d₄*) *δ* (ppm) 153.87, 153.80, 151.33, 150.75, 144.76, 138.91, 133.52, 133.38, 133.05, 132.73, 131.19, 125.12, 123.54, 122.07, 117.40, 44.42, 39.17.
HRMS (APCI): calcd. for C₁₇H₁₆N₅S [M+H]⁺ = 322.1121, found [M+H]⁺ = 322.1123.
FTIR (neat), cm⁻¹: 3608 - 3174, 3058, 1606, 1580, 1445, 1216, 996, 836, 814, 655.

### Preparative Example 29: 5-chloro-3-(2,6-dimethylpyridin-4-yl)thieno[3,2-b]pyridine (intermediate)

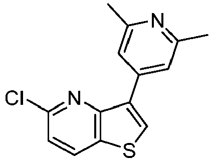

The compound was prepared according to General procedure A using 3-bromo-5-chlorothieno[3,2-b]pyridine (140 mg, 0.563 mmol) and 2,6-dimethyl-4-(4,4,5,5-tetramethyl-1,3,2-dioxaborolan-2-yl)pyridine (158 mg, 0.676 mmol). Reaction time: 4 hours. The solvents were evaporated and the residue was purified by flash chromatography (cyclohexane/EtOAc, gradient from 85:15 to 50:50). The product was obtained as a white solid (80 mg, 52 % yield).
¹H NMR (500 MHz, Chloroform-*d*) *δ* (ppm) 8.16 (d, *J* = 8.4 Hz, 1H), 8.02 (s, 1H), 7.59 (s, 2H), 7.35 (d, *J* = 8.5 Hz, 1H), 2.63 (s, 6H).
¹³C NMR (126 MHz, Chloroform-*d*) *δ* (ppm) 158.02, 152.66, 149.34, 141.64, 134.23, 133.03, 132.83, 130.33, 119.95, 119.24, 24.60.
HRMS (APCI): calcd. for C₁₄H₁₂ClN₂S [M+H]⁺ = 275.0404, found [M+H]⁺ = 275.0405.
FTIR (neat), cm⁻¹: 2919, 1605, 1560, 1378, 1142, 1123, 842, 794, 734, 700, 564, 446.

### Preparative Example 30: 3-(2,6-dimethylpyridin-4-yl)-5-(1-methyl-1H-pyrazol-4-yl)thieno-[3,2-b]pyridine

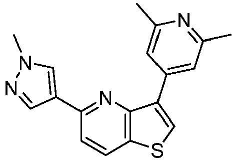

The compound was prepared according to General procedure B using 5-chloro-3-(2,6-dimethylpyridin-4-yl)thieno[3,2-*b*]pyridine **(Prep. Example 29**) (72 mg, 0.260 mmol) and 1-methyl-4-(4,4,5,5-tetramethyl-1,3,2-dioxaborolan-2-yl)-1*H*-pyrazole (67 mg, 0.325 mmol). Reaction temperature: 85 °C. Reaction time: 3 hours. The solvents were evaporated and the residue was purified by flash chromatography (cyclohexane/EtOAc/methanol, gradient from 80:20:0 to 0:90:10). The product was obtained as a white solid (81 mg, 98 % yield).
¹H NMR (500 MHz, Chloroform-*d*) *δ* (ppm) 8.17 (d, *J* = 8.5 Hz, 1H), 8.06 (s, 1H), 8.00 (s, 1H), 7.96 (s, 1H), 7.78 (s, 2H), 7.51 (d, *J=* 8.5 Hz, 1H), 3.99 (s, 3H), 2.66 (s, 6H).
¹³C NMR (126 MHz, Chloroform-*d*) *δ* (ppm) 157.53, 152.93, 149.78, 142.78, 137.91, 134.05, 131.60, 131.25, 129.30, 128.92, 123.97, 119.57, 115.79, 39.27, 24.40.
HRMS (APCI): calcd. for C₁₈H₁₇N₄S [M+H]⁺ = 321.1168, found [M+H]⁺ = 321.1172.
FTIR (neat), cm⁻¹: 1603, 1573, 1538, 1396, 1369, 1210, 1162, 1062, 978, 896, 853, 809, 714, 700, 689, 652, 618, 590, 548, 489, 416.

### Preparative Example 31: 5-chloro-3-(3-methylpyridin-4-yl)thieno[3,2-b]pyridine (intermediate)

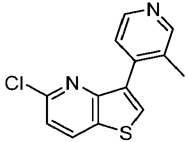

The compound was prepared according to General procedure A using 3-bromo-5-chlorothieno[3,2-b]pyridine (100 mg, 0.402 mmol) and 3-methyl-4-(4,4,5,5-tetramethyl-1,3,2-dioxaborolan-2-yl)pyridine (114 mg, 0.483 mmol). Reaction time: 3 hours. The solvents were evaporated and the residue was purified twice by flash chromatography (cyclohexane/EtOAc, gradient from 90:10 to 30:70). The product was obtained as a brown solid (73 mg, 69 % yield).
¹H NMR (500 MHz, Chloroform-*d*) *δ* (ppm) 8.58 (s, 1H), 8.52 (d, *J* = 4.9 Hz, 1H), 8.18 (d, *J* = 8.5 Hz, 1H), 7.76 (d, *J* = 2.2 Hz, 1H), 7.35 (s, 1H), 7.33 (d, *J* = 2.4 Hz, 1H), 2.28 (s, 3H).
HRMS (APCI): calcd. for C₁₃H₁₀ClN₂S [M+H]⁺ = 261.0248, found [M+H]⁺ = 261.0245.
FTIR (neat), cm⁻¹: 3425, 2978, 2924, 2852, 1599, 1563, 1465, 1379, 1143, 1124, 1111, 948, 883, 829.

### Preparative Example 32: 5-(1-methyl-1H-pyrazol-4-yl)-3-(3-methylpyridin-4-yl)thieno[3.2-b]pyridine

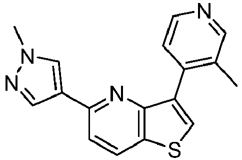

The compound was prepared according to General procedure B using 5-chloro-3-(3-methylpyridin-4-yl)thieno[3,2-b]pyridine (**Prep. Example 31**) (62 mg, 0.237 mmol) and 1-methyl-4-(4,4,5,5-tetramethyl-1,3,2-dioxaborolan-2-yl)-1*H*-pyrazole (59 mg, 0.284 mmol). Reaction time: 5 hours. The solvents were evaporated and the residue was purified by flash chromatography (cyclohexane/EtOAc, gradient from 70:30 to 0:100). The product was obtained as a white solid (20 mg, 28 % yield).
¹H NMR (500 MHz, Chloroform-*d*) *δ* (ppm) 8.61 (s, 1H), 8.55 (s, 1H), 8.19 (d, *J* = 8.5 Hz, 1H), 7.94 (s, 1H), 7.88 (s, 1H), 7.73 (s, 1H), 7.51 (d, *J* = 11.5 Hz, 1H), 7.50 (s, 1H), 3.94 (s, 3H), 2.35 (s, 3H).
¹³C NMR (126 MHz, Chloroform-*d*) *δ* (ppm) 153.13, 150.07, 150.01, 145.90, 137.63, 134.89, 131.14, 130.72, 129.65, 129.12, 125.52, 123.74, 115.80, 39.19, 17.86.
HRMS (APCI): calcd.for C₁₇H₁₅N₄S [M+H]⁺ = 307.1012, found [M+H]⁺ = 307.1012.
FTIR (neat), cm⁻¹: 2924, 1579, 1398, 1361, 1212, 995, 810, 651, 602.

### Preparative Example 33: 4-(5-chlorothieno[3,2-b]pyridin-3-yl)nicotinonitrile (intermediate)

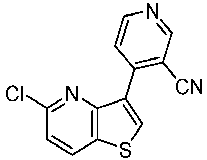

The compound was prepared according to General procedure A using 3-bromo-5-chlorothieno[3,2-*b*]pyridine (250 mg, 1.00 mmol) and 4-(4,4,5,5-tetramethyl-1,3,2-dioxaborolan-2-yl)benzonitrile (276 mg, 1.20 mmol). Reaction time: 2 hours. The solvents were evaporated and the residue was purified by flash chromatography (cyclohexane/EtOAc, gradient from 95:5 to 70:30). The product was obtained as a white solid (69 mg, 25 % yield).
¹H NMR (500 MHz, Chloroform-*d*) *δ* (ppm) 9.01 (s, 1H), 8.91 (d, *J* = 5.2 Hz, 1H), 8.44 (s, 1H), 8.23 (d, *J* = 8.4 Hz, 1H), 8.10 (d, *J* = 5.3 Hz, 1H), 7.42 (d, *J* = 8.5 Hz, 1H).
¹³C NMR (126 MHz, Chloroform-*d*) *δ* (ppm) 153.85, 152.74, 152.13, 149.89, 143.89, 134.18, 133.24, 132.41, 129.91, 124.66, 120.68, 116.85, 108.76.
HRMS (APCI): calcd. for C₁₃H₇ClN₃S [M+H]⁺ = 272.0044, found [M+H]⁺ = 272.0041.
FTIR (neat), cm⁻¹: 2978, 1602, 1587, 1564, 1539, 1507, 1490, 1380, 1229, 1158, 1145, 1125, 949, 835, 810, 792, 701, 563.

### Preparative Example 34: 4-(5-(1-methyl-1H-pyrazol-4-yl)thieno[3,2-b]pyridin-3-yl)nicotino-nitrile

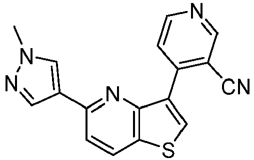

The compound was prepared according to General procedure B using 4-(5-chlorothieno[3,2-*b*]pyridin-3-yl)nicotinonitrile **(Prep. Example 33**) (18 mg, 0.066 mmol), 1-methyl-4-(4,4,5,5-tetramethyl-1,3,2-dioxaborolan-2-yl)-1*H*-pyrazole (16 mg, 0.079 mmol), SPhosPdG3 (7 mg, 0.007 mmol) and Sphos (3 mg, 0.007 mmol) instead of [1,1'-Bis(diphenylphosphino)ferrocene]dichloropalladium(II) in acetonitrile (0.3 mL) instead of dioxane. Reaction time: 16 hours. The solvents were evaporated and the residue was purified by flash chromatography (cyclohexane/EtOAc, gradient from 80:20 to 0:100) and preparative TLC (dichloromethane/methanol, 98:2). The product was obtained as a pale yellow solid (5 mg, 22 % yield).
¹H NMR (500 MHz, Chloroform-*d*) *δ* (ppm) 9.02 (s, 1H), 8.91 (d, *J* = 5.3 Hz, 1H), 8.35 (s, 1H), 8.22 (d, *J* = 8.5 Hz, 1H), 8.17 (d, *J* = 5.2 Hz, 1H), 8.00 (s, 1H), 7.95 (s, 1H), 7.55 (d, *J* = 8.5 Hz, 1H), 3.98 (s, 3H). ¹³C NMR (126 MHz, Chloroform-*d*) *δ* (ppm) 153.93, 152.31, 152.23, 150.29, 144.83, 137.84, 132.93, 131.38, 131.07, 130.39, 129.16, 124.88, 123.55, 117.14, 116.51, 108.99, 39.26.
HRMS (APCI): calcd. for C₁₇H₁₂N₅S [M+H]⁺ = 318.0808, found [M+H]⁺ = 318.0807.
FTIR (neat), cm⁻¹: 1579, 1403, 1215, 996, 822.

### Preparative Example 35: 5-chloro-3-(3-fluoropyridin-4-yl)thieno[3,2-b]pyridine (intermediate)

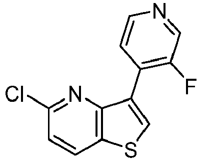

The compound was prepared according to General procedure A using 3-bromo-5-chlorothieno[3,2-b]pyridine (25 mg, 0.100 mmol), (3-fluoropyridin-4-yl)boronic acid (18 mg, 0.125 mmol), trimethylamine (27 µL, 0.200 mmol) instead of potassium phosphate and TPGS/750-M (2 %w) (1.2 mL) in THF (0.8 mL) instead of dioxane. After 6 hours at 40 °C, the reaction was refluxed for 6 hours. The reaction mixture was quenched with water (15 mL) and extracted with EtOAc (2 × 15 mL). The combined organic extracts were washed with brine (10 mL), dried over MgSO4, filtered, and the solvents were evaporated *in vacuo.* The solvents were evaporated and the residue was purified by flash chromatography (hexane/EtOAc, gradient from 100:0 to 80:20). The product was obtained as a pale yellow solid (10 mg, 39 % yield).
¹H NMR (500 MHz, Chloroform-*d*) *δ* (ppm) 8.72 - 8.68 (m, 1H), 8.63 (d, *J =* 15.6 Hz, 2H), 8.45 (d, *J* = 1.01, 1H), 8.22 (d, *J* = 8.4 Hz, 1H), 7.42 (d, *J* = 8.5, 1H).
¹³C NMR (126 MHz, Chloroform-*d*) *δ* (ppm) 157.97 (d, *J* = 237.9 Hz), 152.63, 149.69, 143.67 (d, *J* = 5.3 Hz), 136.42 (d, *J =* 29.2 Hz), 135.77 (d, *J =* 11.4 Hz), 133.17, 132.03, 130.98 (app. s), 125.53, 125.07, 120.47.
¹⁹F NMR (471 MHz, Chloroform-*d*) *δ* -127.3514
HRMS (APCI): calcd. for C₁₂H₇ClFN₂S [M+H]⁺ = 264.9997, found [M+H]⁺ = 264.9999.
FTIR (neat), cm⁻¹: 1606, 1562, 1533, 1380, 1144, 1125, 842, 798, 701.

### Preparative Example 36: 3-(3-fluoropyridin-4-yl)-5-(1-methyl-1H-pyrazol-4-yl)thieno[3,2-b]pyridine

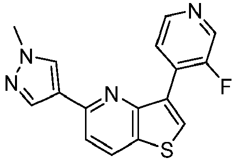

The compound was prepared according to General procedure B using 5-chloro-3-(3-fluoropyridin-4-yl)thieno[3,2-*b*]pyridine (**Prep. Example 35**) (58 mg, 0.219 mmol) and 1-methyl-4-(4,4,5,5-tetramethyl-1,3,2-dioxaborolan-2-yl)-1*H*-pyrazole (57 mg, 0.274 mmol). After 40 min at 25 °C the reaction was stirred at 75 °C for additional 2 hours. The solvents were evaporated and the residue was purified by flash chromatography (cyclohexane/EtOAc, gradient from 80:20 to 20:80). The product was obtained as a white solid (55 mg, 81 % yield).
¹H NMR (500 MHz, Chloroform-*d*) *δ* (ppm) 8.67 - 8.42 (m, 3H), 8.23 (s, 1H), 8.15 (d, *J =* 8.5 Hz, 1H), 8.00 (s, 1H), 7.93 (s, 1H), 7.48 (d, *J* = 8.4 Hz, 1H), 3.96 (s, 3H).
¹³C NMR (126 MHz, Chloroform-*d*) *δ* (ppm) 157.05 (d, *J* = 258.2 Hz), 152.98, 149.82, 145.46 (d, *J* = 5.1 Hz), 138.52 (d, *J* = 26.2 Hz), 137.76, 133.01 (d, *J* = 10.2 Hz), 131.10, 130.61, 129.19 (d, *J* = 9.7 Hz), 128.96, 126.59, 124.66 (br s), 123.73, 115.83,39.18.
¹⁹F NMR (471 MHz, Chloroform-*d*) *δ* (ppm) -130.0085.
HRMS (APCI): calcd. for C₁₆H₁₂FN₄S [M+H]⁺ = 311.0761, found [M+H]⁺ = 311.0764.
FTIR (neat), cm⁻¹: 3380, 2927, 1605, 1579, 1544, 1477, 1401, 1382, 1215, 1061, 1027, 994, 955, 834, 795, 640,582,469.

### Preparative Example 37: 5-chloro-3-(pyrimidin-5-yl)thieno[3,2-b]pyridine (intermediate)

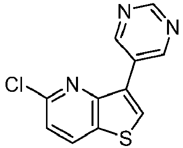

The compound was prepared according to General procedure A using 3-bromo-5-chlorothieno[3,2-*b*]pyridine (224 mg, 0.901 mmol) and pyrimidin-5-ylboronic acid (134 mg, 1.08 mmol). Reaction time: 3 hours. The solvents were evaporated and the residue was purified by flash chromatography (hexane/EtOAc, gradient from 80:20 to 0:100). The product was obtained as a white solid (95 mg, 43 % yield).
¹H NMR (500 MHz, Chloroform-*d*) *δ* (ppm) 9.37 (s, 2H), 9.25 (s, 1H), 8.20 (d, *J =* 8.5 Hz, 1H), 8.05 (s, 1H), 7.39 (d, *J* = 8.5 Hz, 1H).
¹³C NMR (126 MHz, Chloroform-*d*) *δ* (ppm) 157.67, 155.75, 152.38, 149.76, 133.14, 132.62, 129.93, 129.70, 127.92, 120.37.
HRMS calcd. for C₁₁H₇ClN₃S [M+H]⁺248.0044, found [M+H]⁺ = 248.0047.
FTIR (neat), cm⁻¹: 1562, 1530, 1405, 1378, 1347, 1195, 1139, 1126, 1115, 846, 826, 799, 765, 717, 684, 634, 483.

### Preparative Example 38: 5-(1-methyl-1H-pyrazol-4-yl)-3-(pyrimidin-5-yl)thieno[3,2-b]pyridine

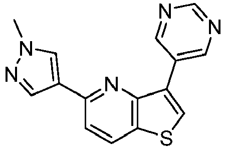

The compound was prepared according to General procedure B using 5-chloro-3-(pyrimidin-5-yl)thieno[3,2-*b*]pyridine (**Prep. Example 37**) (35 mg, 0.141 mmol) and 1-methyl-4-(4,4,5,5-tetramethyl-1,3,2-dioxaborolan-2-yl)-1*H*-pyrazole (35 mg, 0.170 mmol). After 16 hours, triethylamine (0.7 mL, 5.04 mmol) was added and the mixture was refluxed for 36 hours. The solvents were evaporated and the residue was purified twice by flash chromatography (cyclohexane/EtOAc, gradient from 80:20 to 0:100; then dichloromethane/methanol, gradient from 100:0 to 98:2). The product was obtained as a white solid (30 mg, 72 % yield).
¹H NMR (500 MHz, Chloroform-*d*) *δ* (ppm) 9.49 (s, 2H), 9.24 (s, 1H), 8.21 (d, *J* = 8.5 Hz, 1H), 8.03 (d, *J* = 14.3 Hz, 2H), 8.00 (s, 1H), 7.56 (d, *J=* 8.5 Hz, 1H), 3.99 (s, 3H).
¹³C NMR (126 MHz, Chloroform-*d*) *δ* (ppm) 157.28, 155.82, 152.57, 150.24, 137.69, 136.91, 131.37, 131.32, 129.98, 129.29, 128.71, 128.42, 123.58, 116.09, 39.24.
HRMS (APCI): calcd. for C₁₅H₁₂N₅S [M+H]⁺ = 294.0808, found [M+H]⁺ = 294.0806.
FTIR (neat), cm⁻¹: 2920, 2851, 1584, 1555, 1402, 1219, 995, 817, 794, 723, 649, 615.

### Preparative Example 39: 5-chloro-3-(pyridazin-4-yl)thieno[3,2-b]pyridine (intermediate)

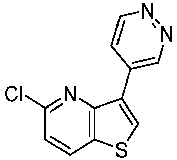

The compound was prepared according to General procedure A using 3-bromo-5-chlorothieno[3,2-b]pyridine (170 mg, 0.684 mmol), 4-(4,4,5,5-tetramethyl-1,3,2-dioxaborolan-2-yl)pyridazine (175 mg, 0.850 mmol) and [1,1'-Bis(di-*tert*-butylphosphino)ferrocene]dichloropalladium(II) (22 mg, 0.034 mmol) instead of [1,1'-Bis(diphenylphosphino)ferrocene]dichloropalladium(II). Reaction temperature: 70 °C. Reaction time: 16 hours. The solvents were evaporated and the residue was purified twice by flash chromatography (cyclohexane/EtOAc, gradient from 80:20 to 20:80; then dicloromethane/methanol, gradient from 100:0 to 95:5). The product was obtained as a white solid (110 mg, 65 % yield).
¹H NMR (500 MHz, Chloroform-*d*) *δ* (ppm) 9.76 (dd, *J* = 2.4, 1.2 Hz, 1H), 9.30 (dd, *J* = 5.4, 1.3 Hz, 1H), 8.42 (dd, *J* = 5.4, 2.4 Hz, 1H), 8.27 (s, 1H), 8.21 (d, *J* = 8.5 Hz, 1H), 7.41 (d, *J* = 8.5 Hz, 1H).
¹³C NMR (126 MHz, Chloroform-*d*) *δ* (ppm) 152.42, 151.55, 149.87, 149.79, 133.23, 132.83, 131.78, 131.64, 129.77, 124.00, 120.57.
HRMS (APCI): calcd. for C₁₁H₇ClN₃S [M+H]⁺ = 248.0044, found [M+H]⁺ = 248.0045.
FTIR (neat), cm⁻¹: 3044, 3012, 2425, 2041, 1583, 1562, 1527, 1494, 1381, 1353, 1148, 1123, 828, 814, 723, 677, 648, 477.

### Preparative Example 40: 5-(1-methyl-1H-pyrazol-4-yl)-3-(pyridazin-4-yl)thieno[3,2-b]-pyridine

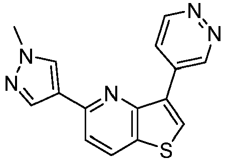

The compound was prepared according to General procedure B using 5-chloro-3-(pyridazin-4-yl)thieno[3,2-b]pyridine (**Prep. Example 39**) (50 mg, 0.202 mmol) and 1-methyl-4-(4,4,5,5-tetramethyl-1,3,2-dioxaborolan-2-yl)-1*H*-pyrazole (53 mg, 0.252 mmol). After 24 hours, additional 1-methyl-4-(4,4,5,5-tetramethyl-1,3,2-dioxaborolan-2-yl)-1*H*-pyrazole (53 mg, 0.252 mmol), tripotassium phosphate (129 mg, 0.606 mmol), [1,1'-Bis(diphenylphosphino)ferrocene]dichloropalladium(II) (7 mg, 0.010 mmol) and H₂O (0.5 mL) were added and the mixture was refluxed for additional 1.5 hours. The solvents were evaporated and the residue was purified by flash chromatography (hexane/EtOAc/methanol, gradient from 20:80:0 to 0:90:10). The product was obtained as a white solid (52 mg, 89 % yield).
¹H NMR (500 MHz, Methanol-*d*₄) *δ* (ppm) 10.07 (s, 1H), 9.24 (d, *J=* 5.5 Hz, 1H), 8.68 (q, *J* = 3.0 Hz, 2H), 8.31 (d, *J=* 8.5 Hz, 1H), 8.24 (s, 1H), 8.09 (s, 1H), 7.64 (d, *J=* 8.5 Hz, 1H), 3.98 (s, 3H).
¹³C NMR (126 MHz, Methanol-*d*₄) *δ* (ppm) 153.75, 152.65, 151.46, 151.26, 138.81, 135.24, 134.37, 133.10, 133.00, 131.26, 129.81, 126.01, 124.89, 117.47, 39.16.
HRMS (APCI): calcd. for C₁₅H₁₂N₅S [M+H]⁺ = 294.0808, found [M+H]⁺ = 294.0809.
FTIR (neat), cm⁻¹: 3071, 1577, 1553, 1400, 1366, 1215, 1189, 1178, 1051, 998, 979, 857, 814, 676, 648, 488.

### Preparative Example 41: 4-(5-(5-(1-methyl-1H-pyrazol-4-yl)thieno[3,2-b]pyridin-3-yl)pyridin-2-vDmorpholine (intermediate)

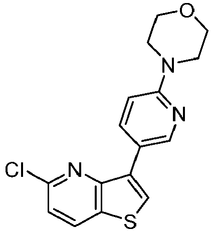

The compound was prepared according to General procedure A using 3-bromo-5-chlorothieno[3,2-*b*]pyridine (300 mg, 1.21 mmol) and 4-(5-(4,4,5,5-tetramethyl-1,3,2-dioxaborolan-2-yl)pyridin-2-yl)morpholine (438 mg, 1.51 mmol). Reaction time: 80 °C. Reaction time: 3.5 hours. The solvents were evaporated and the residue was purified by flash chromatography (hexane/EtOAc, gradient from 90:10 to 60:40). The product was obtained as a white solid (250 mg, 62 % yield).
¹H NMR (500 MHz, Chloroform-*d*) *δ* (ppm) 8.76 (d, *J* = 1.7 Hz, 1H), 8.23 (dd, *J* = 8.9, 2.5 Hz, 1H), 8.14 *(d, J=* 8.5 Hz, 1H), 7.78 (s, 1H), 7.32 (d, *J* = 8.5 Hz, 1H), 6.77 (d, *J* = 8.8 Hz, 1H), 3.85 (t, *J* = 5.1 Hz, 4H), 3.59 (t, *J* = 5.0 Hz, 4H).
¹³C NMR (126 MHz, Chloroform-*d*) *δ* (ppm) 158.84, 153.04, 148.95, 147.22, 137.32, 133.72, 132.91, 132.67, 126.06, 120.06, 119.51, 106.43, 66.73, 45.60.
HRMS (APCI): calcd. for C₁₆H₁₅ClN₃OS [M+H]⁺ = 332.0619, found [M+H]⁺ 332.0616.
FTIR (neat), cm⁻¹: 3061, 2958, 2867, 2850, 1606, 1562, 1530, 1516, 1484, 1444, 1372, 1323, 1244, 1141, 1112, 942, 794, 649, 595, 460.

### Preparative Example 42: 4-(5-(5-(1-methyl-1H-pyrazol-4-yl)thieno[3.2-b]pyridin-3-yl)pyridin-2-yl)morpholine

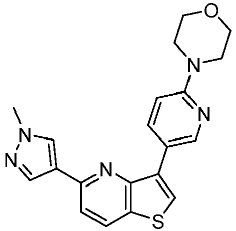

The compound was prepared according to General procedure B using 4-(5-(5-chlorothieno[3,2-*b*]pyridin-3-yl)pyridin-2-yl)morpholine (**Prep. Example 41**) (45 mg, 0.136 mmol) and 1-methyl-4-(4,4,5,5-tetramethyl-1,3,2-dioxaborolan-2-yl)-1*H*-pyrazole (35 mg, 0.170 mmol). Reaction temperature: 85 °C. Reaction time: 16 hours. The solvents were evaporated and the residue was purified by flash chromatography (hexane/EtOAc, gradient from 80:20 to 40:60). The product was obtained as a white solid (36 mg, 70 % yield).
¹H NMR (500 MHz, Chloroform-*d*) *δ* (ppm) 8.94 (br s, 1H), 8.30 (dd, *J=* 8.8, 2.3, 1H), 8.15 (d, *J* = 8.4 Hz, 1H), 8.02 (s, 2H), 7.73 (s, 1H), 7.48 (d, *J* = 8.4 Hz, 1H), 6.79 (d, *J* = 8.9 Hz, 1H), 3.98 (s, 3H), 3.88 (t, *J* = 4.7 Hz, 4H), 3.88 (d, *J* = 4.3 Hz, 4H).
¹³C NMR (126 MHz, Chloroform-*d*) *δ* (ppm) 158.74, 153.23, 149.49, 147.43, 137.77, 137.50, 133.88, 131.46, 131.10, 129.07, 124.88, 124.05, 121.12, 115.46, 106.39, 66.77, 45.77, 39.15.
HRMS (APCI): calcd.for C₂₀H₂₀N₅OS [M+H]⁺ = 378.1383, found [M+H]⁺ = 378.1386.
FTIR (neat), cm⁻¹: 2968, 2848, 1601, 1577, 1549, 1537, 1518, 1482, 1441, 1402, 1372, 1312, 1254, 1210, 1115, 980, 941, 820, 786, 638, 549, 468.

### Preparative Example 43: 5-chloro-3-(1-tosyl-1H-pyrrolo[2.3-b]pyridin-4-yl)thieno[3.2-b]pyridine (intermediate)

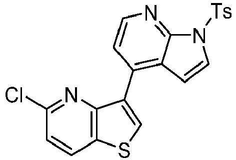

The compound was prepared according to General procedure A using 3-bromo-5-chlorothieno[3,2-*b*]pyridine (114 mg, 0.460 mmol) and 4-(4,4,5,5-tetramethyl-1,3,2-dioxaborolan-2-yl)-1-tosyl-1*H-*pyrrolo[2,3-*b*]pyridine (220 mg, 0.055 mmol). Reaction time: 2 hours. The solvents were evaporated and the residue was purified twice by flash chromatography (cyclohexane/EtOAc, gradient from 90:10 to 70:30; then cyclohexane/EtOAc, gradient from 100:0 to 80:20). The product was obtained as a pale yellow solid (96 mg, 48 % yield).
¹H NMR (500 MHz, Chloroform-*d*) *δ* (ppm) 8.56 (d, *J* = 5.0 Hz, 1H), 8.19 (d, *J* = 8.5 Hz, 1H), 8.11 (d, *J* = 8.3 Hz, 2H), 8.04 (s, 1H), 7.78 (d, *J* = 4.0 Hz, 1H), 7.71 (d, *J* = 4.9 Hz, 1H), 7.36 (d, *J=* 8.5 Hz, 1H), 7.28 (d, *J* = 8.1 Hz, 2H), 6.73 (d, *J* = 4.0 Hz, 1H), 2.38 (s, 3H).
¹³C NMR (126 MHz, Chloroform-*d*) *δ* (ppm) 152.66, 149.56, 147.66, 145.24, 144.93, 135.35, 135.06, 133.11, 132.62, 131.86, 129.66, 128.17, 126.58, 121.43, 120.21, 119.08, 104.89, 21.62.
HRMS (APCI): calcd.for C₂₁H₁₅ClN₃O₂S₂ [M+H]⁺ = 440.0289, found [M+H]⁺ = 440.0291.
FTIR (neat), cm⁻¹: 1592, 1383, 1365, 1176, 1161, 1147, 811, 730, 681, 580.

### Preparative Example 44: 5-(1-methyl-1H-pyrazol-4-yl)-3-(1-tosyl-1H-pyrrolo[2,3-b]pyridin-4-yl)thieno[3,2-b]pyridine

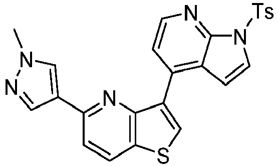

The compound was prepared according to General procedure B using 5-chloro-3-(1-tosyl-1*H*-pyrrolo[2,3-*b*]pyridin-4-yl)thieno[3,2-*b*]pyridine **(Prep. Example 43**) (90 mg, 0.204 mmol) and 1-methyl-4-(4,4,5,5-tetramethyl-1,3,2-dioxaborolan-2-yl)-1*H*-pyrazole (50 mg, 0.204 mmol). Reaction time: 2 hours. The solvents were evaporated and the residue was purified by flash chromatography (cyclohexane/EtOAc, gradient from 70:30 to 40:60). The product was obtained as a pale yellow wax. (54 mg, 55 % yield).
¹H NMR (500 MHz, Chloroform-*d*) *δ* (ppm) 8.55 (d, *J=* 5.0 Hz, 1H), 8.20 (d, *J=* 8.5 Hz, 1H), 8.12 *(d, J=* 8.4 Hz, 2H), 7.97 (s, 1H), 7.95 (s, 1H), 7.87 (s, 1H), 7.77 (d, *J=* 1.7 Hz, 1H), 7.76 (s, 1H), 7.52 (d, *J=* 8.5 Hz, 1H), 7.29 (d, *J=* 8.1 Hz, 2H), 6.80 (d, *J=* 4.1 Hz, 1H), 3.95 (s, 3H), 2.38 (s, 3H).
¹³C NMR (126 MHz, Chloroform-*d*) *δ* (ppm) 152.84, 150.01, 148.00, 145.11, 144.82, 137.76, 136.04, 135.55, 133.61, 131.27, 131.24, 130.53, 129.62, 129.10, 128.11, 126.02, 123.77, 121.61, 119.27, 115.98, 105.81, 39.23, 21.63.
HRMS (APCI): calcd. for C₂₅H₂₀N₅O₂S₂ [M+H]⁺ = 486.1053, found [M+H]⁺ = 486.1056.
FTIR (neat), cm⁻¹: 2920, 2851, 1632, 1600, 1557, 1434, 1397, 1355, 1155, 1112, 828, 632.

### Preparative Example 45: 5-(1-methyl-1H-pyrazol-4-yl)-3-(1H-pyrrolo[2,3-b]pyridin-4-yl)thieno[3,2-b]pyridine

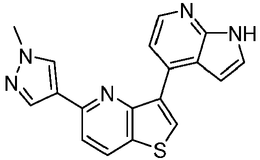

To a solution of 5-(1-methyl-1*H*-pyrazol-4-yl)-3-(1-tosyl-1*H*-pyrrolo[2,3-*b*]pyridin-4-yl)-thieno[3,2-*b*]pyridine **(Prep. Example 44**) (36 mg, 0.074 mmol) in THF (2 mL) and methanol (2 mL) was added 5 M NaOH (0.7 mL). Reaction temperature: 60 °C. Reaction time: 30 min. The solvents were evaporated and the residue was purified by flash chromatography (cyclohexane/EtOAc/methanol, gradient from 50:50:0 to 0:95:5). The product was obtained as a colorless wax (14 mg, 58 % yield).
¹H NMR (500 MHz, Chloroform-*d*) *δ* (ppm) 10.03 (s, 1H), 8.47 (d, *J* = 5.1 Hz, 1H), 8.21 (d, *J* = 8.5 Hz, 1H), 8.14 (s, 1H), 8.01 (s, 1H), 7.95 (s, 1H), 7.87 (d, *J* = 5.0 Hz, 1H), 7.53 (d, *J* = 8.4 Hz, 1H), 7.41 (d, *J* = 3.6 Hz, 1H), 6.76 (d, *J=* 3.5 Hz, 1H), 3.95 (s, 3H).
¹³C NMR (126 MHz, Chloroform-*d*) *δ* (ppm) 153.31, 149.82, 149.20, 142.66, 137.79, 135.28, 134.47, 131.36, 131.14, 130.28, 129.20, 124.86, 124.03, 119.04, 116.48, 115.76, 101.37, 39.18.
HRMS (APCI): calcd. for C₁₈H₁₄N₅S [M+H]⁺ = 332.0964, found [M+H]⁺ = 332.0965.
FTIR (neat), cm⁻¹: 3133, 1579, 1542, 1376, 1342, 1276, 985, 831, 797, 758, 657, 644.

### Preparative Example 46: 5-chloro-3-(quinazolin-6-yl)thieno[3,2-b]pyridine (intermediate)

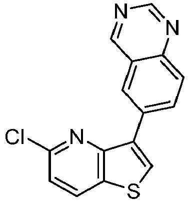

To a degassed solution of 6-bromoquinazoline (111 mg, 0.530 mmol) in dioxane (3.5 mL) were added bis(pinacolato)diboron (134 mg, 0.530 mmol), tris(dibenzylidenaceton)dipalladium(0) (19 mg, 0.021 mmol), XPhos (32 mg, 0.070 mmol), potassium acetate (166 mg, 1.70 mmol) and the reaction mixture was refluxed for 6 hours. Then, 3-bromo-5-chlorothieno[3,2-*b*]pyridine (100 mg, 0.424 mmol), tripotassium phosphate (88 mg, 0.417 mmol) and H₂O (0.3 mL) were added and the reaction mixture was stirred at reflux for 16 hours. The solvents were evaporated and the residue was purified by flash chromatography (cyclohexane/EtOAc, gradient from 50:50 to 30:70). The product was obtained as a pale yellow solid (29 mg, 23 %).
¹H NMR (500 MHz, Chloroform-*d*) *δ* (ppm) 9.52 (s, 1H), 9.35 (s, 1H), 8.75 (d, *J=* 1.9 Hz, 1H), 8.48 (dd, *J* = 8.8, 2.0 Hz, 1H), 8.21 (d, *J* = 8.5 Hz, 1H), 8.16 *(d, J=* 8.8 Hz, 1H), 8.08 (s, 1H), 7.39 (d, *J* = 8.5 Hz, 1H). ¹³C NMR (126 MHz, Chloroform-*d*) *δ* (ppm) 160.71, 155.42, 152.68, 149.66, 149.45, 134.61, 134.50, 133.34, 133.18, 132.92, 129.77, 128.66, 126.31, 125.31, 120.08.
HRMS (APCI): calcd. for C₁₅H₉ClN₃S [M+H]⁺ = 298.0199, found [M+H]⁺ = 298.0199.
FTIR (neat), cm⁻¹: 3076, 1562, 1489, 1380, 1355, 1143,124, 1076, 927, 841, 797, 780, 583,550.

### Preparative Example 47: 5-(1-methyl-1H-pyrazol-4-yl)-3-(quinazolin-6-yl)thieno[3,2-b]pyridine

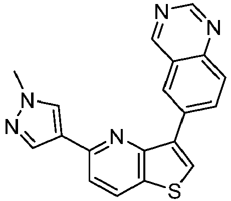

The compound was prepared according to General procedure B using 5-chloro-3-(quinazolin-6-yl)thieno[3,2-*b*]pyridine **(Prep. Example 46**) (35 mg, 0.117 mmol) and 1-methyl-4-(4,4,5,5-tetramethyl-1,3,2-dioxaborolan-2-yl)-1*H*-pyrazole (28 mg, 0.130 mmol). Reaction time: 2 hours. The solvents were evaporated and the residue was purified by flash chromatography (cyclohexane/EtOAc/methanol, gradient from 50:50:0 to 95:0:5). The product was obtained as a pale yellow solid (6 mg, 18 % yield).
¹H NMR (500 MHz, Chloroform-*d*) *δ* (ppm) 9.52 (s, 1H), 9.37 (s, 1H), 8.87 (d, *J=* 2.0 Hz, 1H), 8.61 (dd, *J* = 8.7, 2.0 Hz, 1H), 8.22 (d, *J* = 8.4 Hz, 1H), 8.17 (s, 1H), 8.06 (s, 1H), 8.03 (s, 1H), 7.99 (s, 1H), 7.55 (d, *J* = 8.5 Hz, 1H), 4.00 (s, 3H).
¹³C NMR (126 MHz, Chloroform-*d*) *δ* (ppm) 160.50, 155.29, 152.92, 149.94, 149.58, 137.87, 134.99, 134.79, 134.40, 131.68, 131.38, 128.96, 128.56, 128.25, 126.26, 125.37, 123.95, 115.94, 39.30.
HRMS (APCI): calcd.for C₁₉H₁₄N₅S [M+H]⁺ = 344.0964, found [M+H]⁺ = 344.0965
FTIR (neat), cm⁻¹: 1568, 1381, 1367, 1220, 1142, 995, 982, 833, 788, 648, 555, 489, 451.

### Preparative Example 48: 5-chloro-3-(quinoxalin-6-yl)thieno[3,2-b]pyridine (intermediate)

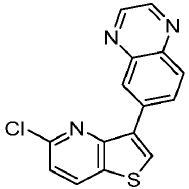

To a solution of 6-bromoquinoxaline (157 mg, 0.753 mmol) in dioxane (3.5 mL) was added bis(pinacolato)diboron (191 mg, 0.753 mmol), tris(dibenzylidenaceton)dipalladium(0) (28 mg, 0.030 mmol), XPhos (46 mg, 0.096 mmol) and potassium acetate (237 mg, 2.41 mmol) and the reaction mixture was refluxed for 8 hours. Then, 3-bromo-5-chlorothieno[3,2-*b*]pyridine (150 mg, 0.603 mmol), tripotassium phosphate (384 mg, 1.81 mmol) and H₂O (0.4 mL) were added and the reaction mixture was refluxed for 16 hours. The solvents were evaporated and the residue was purified by flash chromatography (cyclohexane/EtOAc, gradient from 80:20 to 40:60). The product was obtained as a white solid (79 mg, 44%).
¹H NMR (500 MHz, Chloroform-*d*) *δ* (ppm) 8.88 (dd, *J* = 16.8, 1.8 Hz, 2H), 8.78 (d, *J* = 2.0 Hz, 1H), 8.45 (dd, *J* = 8.8, 2.0 Hz, 1H), 8.21 (dd, *J=* 12.9, 8.6 Hz, 2H), 8.11 (s, 1H), 7.38 (d, *J* = 8.5 Hz, 1H).
¹³C NMR (126 MHz, Chloroform-*d*) *δ* (ppm) 152.78, 149.46, 145.35, 144.94, 143.26, 142.74, 135.61, 135.05, 133.09, 132.91, 130.65, 130.02, 129.62, 128.49, 120.02.
HRMS (APCI): calcd. for C₁₅H₉ClN₃S [M+H]⁺ = 298.0199, found [M+H]⁺ = 298.0202.
FTIR (neat), cm⁻¹: 1635, 1564, 1382, 1153, 1123, 1028, 951, 902, 844, 832, 788, 734, 408.

### Preparative Example 49: 5-(1-methyl-1H-pyrazol-4-yl)-3-(quinoxalin-6-yl)thieno[3,2-b]pyridine

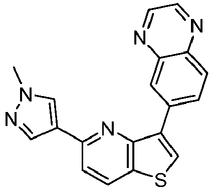

The compound was prepared according to General procedure B using 5-chloro-3-(quinoxalin-6-yl)thieno[3,2-b]pyridine (**Prep. Example 48**) (40 mg, 0.134 mmol) and 1-methyl-4-(4,4,5,5-tetramethyl-1,3,2-dioxaborolan-2-yl)-1*H*-pyrazole (32 mg, 0.154 mmol). Reaction time: 4 hours. The solvents were evaporated and the residue was purified by flash chromatography (cyclohexane/EtOAc/methanol, gradient from 50:50:0 to 95:0:5). The product was obtained as a white solid (22 mg, 48 % yield).
¹H NMR (500 MHz, Chloroform-*d*) *δ* (ppm) 8.95 - 8.83 (m, 3H), 8.56 (dd, *J=* 8.8, 2.0 Hz, 1H), 8.22 (dd, *J=* 8.6, 6.6 Hz, 2H), 8.06 (s, 1H), 8.05 (d, *J* = 4.1 Hz, 2H), 7.55 (d, *J* = 8.4 Hz, 1H), 3.99 (s, 3H).
¹³C NMR (126 MHz, Chloroform-*d*) *δ* (ppm) 153.01, 149.97, 145.25, 144.72, 143.37, 142.64, 137.84, 136.54, 135.17, 131.65, 131.29, 131.04, 129.22, 129.18, 128.84, 128.51, 123.94, 115.83, 39.26.
HRMS (APCI): calcd. for C₁₉H₁₄N₅S [M+H]⁺ = 344.0964, found [M+H]⁺ = 344.0962.
FTIR (neat), cm⁻¹: 3443, 3091, 2939, 1614, 1578, 1541, 1400, 1372, 1216, 1159, 1026, 864, 784.

### Preparative Example 50: 5-chloro-3-(2H-indazol-5-yl)thieno[3.2-b]pyridine (intermediate)

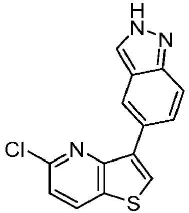

The compound was prepared according to General procedure A using 3-bromo-5-chlorothieno[3,2-*b*]pyridine (200 mg, 0.805 mmol) and 5-(4,4,5,5-tetramethyl-1,3,2-dioxaborolan-2-yl)-2*H*-indazole (216 mg, 0.885 mmol). Reaction time: 4.5 hours. The solvents were evaporated and the residue was purified by two flash chromatography (cyclohexane/EtOAc, gradient from 100:0 to 40:60). The product was obtained as a white solid (146 mg, 63 % yield).
¹H NMR (500 MHz, Methanol-*d*₄) *δ* (ppm) 8.49 (s, 1H), 8.38 (d, *J=* 8.5 Hz, 1H), 8.17 (s, 1H), 8.14 (s, 1H), 7.99 (dd, *J* = 8.7, 1.5 Hz, 1H), 7.64 (d, *J* = 8.7 Hz, 1H), 7.41 (d, *J* = 8.4 Hz, 1H).
¹³C NMR (126 MHz, Methanol-*d₄*) *δ* (ppm) 154.32, 150.03, 141.16, 137.48, 135.60, 134.97, 134.64, 129.87, 128.90, 128.29, 124.59, 121.60, 120.52, 111.04.
HRMS (APCI): calcd. for C₁₄H₉N₃ClS [M+H]⁺ = 286.0200, found [M+H]⁺ = 286.0202.
FTIR (neat), cm⁻¹: 3503 - 2632, 1541, 1381, 1125, 949, 786.

### Preparative Example 51: 3-(2H-indazol-5-yl)-5-(1-methyl-1H-pyrazol-4-yl)thieno[3,2-b]-pyridine

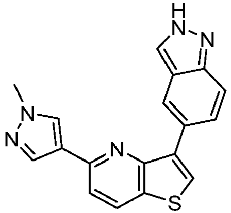

The compound was prepared according to General procedure B using 5-chloro-3-(2*H*-indazol-5-yl)thieno[3,2-b]pyridine (**Prep. Example 50**) (45 mg, 0.157 mmol) and 1-methyl-4-(4,4,5,5-tetramethyl-1,3,2-dioxaborolan-2-yl)-1*H*-pyrazole (39 mg, 0.550 mmol). Reaction time: 16 hours. The solvents were evaporated and the residue was purified by flash chromatography (cyclohexane/EtOAc, gradient from 50:50 to 0:100). The product was obtained as a white solid (42 mg, 81 % yield).
¹H NMR (500 MHz, Methanol-*d₄*) *δ* (ppm) 8.60 (dd, *J =* 1.6, 0.9 Hz, 1H), 8.31 (d, *J =* 8.5 Hz, 1H), 8.20 (s, 1H), 8.16 (d, *J* = 1.1 Hz, 1H), 8.11 (dd, *J* = 8.8, 1.6 Hz, 1H), 8.09 (d, *J* = 0.9 Hz, 1H), 8.06 (s, 1H), 7.65 (t, *J=* 8.8 Hz, 2H), 3.96 (s, 3H).
¹³CNMR (126 MHz, Methanol-*d₄*) *δ* (ppm) 154.48, 150.76, 138.80, 137.87, 135.54, 133.36, 132.75, 131.04, 129.25, 129.22, 128.15, 125.42, 124.64, 121.61, 116.79, 110.92, 39.12.
HRMS (APCI): calcd. for C₁₈H₁₄N₅S [M+H]⁺ = 332.0964, found [M+H]⁺ = 332.0965.
FTIR (neat), cm⁻¹: 1579, 1544, 1224, 1213, 908, 824, 778, 770, 701, 451, 428.

### Preparative Example 52: 3-([1,2,4]triazolo[1,5-a]pyridin-6-yl)-5-chlorothieno[3,2-b]pyridine (intermediate)

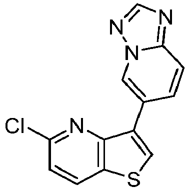

The compound was prepared according to General procedure A using 3-bromo-5-chlorothieno[3,2-*b*]pyridine (109 mg, 0.438 mmol) and 6-(4,4,5,5-tetramethyl-1,3,2-dioxaborolan-2-yl)-[1,2,4]triazolo[1,5-*a*]pyridine (129 mg, 0.526 mmol). Reaction time: 75 min. The solvents were evaporated and the residue was purified by flash chromatography (cyclohexane/EtOAc, gradient from 70:30 to 0:100). The product was obtained as a white solid (75 mg, 60 % yield).
¹H NMR (500 MHz, Chloroform-*d*) *δ* (ppm) 9.71 (s, 1H), 8.42 (s, 1H), 8.20 (d, *J* = 8.5 Hz, 1H), 8.04 (d, *J* = 10.5 Hz, 2H), 7.88 (d, *J* = 9.5 Hz, 1H), 7.40 (d, *J* = 8.5 Hz, 1H).
¹³C NMR (126 MHz, Chloroform-*d*) *δ* (ppm) 154.09, 152.20, 149.55, 133.22, 132.82, 130.88, 130.35, 129.12, 127.79, 121.17, 120.30, 116.33.
HRMS (APCI): calcd. for C₁₃H₈ClN₄S [M+H]⁺ = 287.0153, found [M+H]⁺ = 287.0153.
FTIR (neat), cm⁻¹: 3058, 1637, 1539, 1499, 1381, 1315, 1255, 1187, 1150, 1121, 910, 832, 805, 729, 655, 578, 422.

### Preparative Example 53: 3-([1,2,4]triazolo[1,5-a]pyridin-6-yl)-5-(1-methyl-1H-pyrazol-4-yl)thieno[3,2-b]ridine

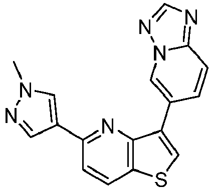

The compound was prepared according to General procedure B using 3-([1,2,4]triazolo[1,5-*a*]pyridin-6-yl)-5-chlorothieno[3,2-*b*]pyridine **(Prep. Example 52)** (65 mg, 0.227 mmol) and 1-methyl-4-(4,4,5,5-tetramethyl-1,3,2-dioxaborolan-2-yl)-1*H*-pyrazole (57 mg, 0.272 mmol). Reaction time: 1.5 hours. The solvents were evaporated and the residue was purified by flash chromatography (EtOAc/methanol, gradient from 100:0 to 90:10). The product was obtained as a white solid (62 mg, 83 % yield).
¹H NMR (500 MHz, DMSO-*d₆*) *δ* (ppm) 10.11 (s, 1H), 8.69 (s, 1H), 8.57 (s, 1H), 8.56 (d, *J =* 8.4 Hz, 1H), 8.45 (dd, *J =* 9.4, 1.8 Hz, 1H), 8.38 (s, 1H), 8.13 (s, 1H), 8.00 (d, *J =* 9.3 Hz, 1H), 7.78 (d, *J =* 8.5 Hz, 1H), 3.95 (s, 3H).
¹³C NMR (126 MHz, DMSO-*d₆*) *δ* (ppm) 154.32, 151.98, 149.61, 148.99, 137.26, 132.38, 131.01, 130.46, 129.92, 129.67, 129.38, 126.87, 122.79, 121.50, 116.10, 115.77, 38.81.
HRMS (APCI): calcd. for C₁₇H₁₃N₆S [M+H]⁺ = 333.0917, found [M+H]⁺ = 333.0920.
FTIR (neat), cm⁻¹: 2924, 1590, 1578, 1436, 1190, 1165, 1118, 1100, 1028, 833, 750, 723, 699, 565, 531, 492.

### Preparative Example 54: 5-chloro-3-(pyrazolo[1,5-a]pyrimidin-6-yl)thieno[3,2-b]pyridine (intermediate)

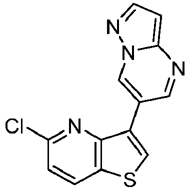

The compound was prepared according to General procedure A using 3-bromo-5-chlorothieno[3,2-*b*]pyridine (80 mg, 0.322 mmol) and 6-(4,4,5,5-tetramethyl-1,3,2-dioxaborolan-2-yl)imidazo[1,2-*a*]pyridine (95 mg, 0.386 mmol). Reaction time: 2 hours. The solvents were evaporated and the residue was purified by flash chromatography (cyclohexane/EtOAc, gradient from 80:20 to 0:100). The product was obtained as a pale yellow solid (39 mg, 42 % yield).
¹H NMR (500 MHz, Chloroform-*d*) *δ* (ppm) 9.82 (d, *J =* 2.6 Hz, 1H), 8.95 (d, *J* = 2.3 Hz, 1H), 8.22 - 8.17 (m, 2H), 8.07 (s, 1H), 7.40 (d, *J* = 8.4 Hz, 1H), 6.76 (d, *J* = 1.8 Hz, 1H).
¹³C NMR (126 MHz, Chloroform-*d*) *δ* (ppm) 152.31, 149.61, 149.07, 147.56, 145.68, 133.81, 133.19, 132.63, 128.91, 128.33, 120.39, 115.15, 97.11.
HRMS (APCI): calcd.for C₁₃H₈ClN₄S [M+H]⁺ = 287.0153, found [M+H]⁺ = 287.0152.

### Preparative Example 55: 5-(1-methyl-1H-pyrazol-4-yl)-3-(pyrazolo[1,5-a]pyrimidin-6-yl)thieno-(3,2-b]ridine

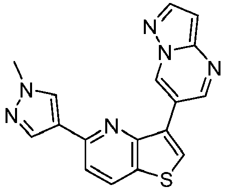

The compound was prepared according to General procedure B using 5-chloro-3-(pyrazolo[1,5-*a*]pyrimidin-6-yl)thieno[3,2-*b*]pyridine **(Prep. Example 54)** (50 mg, 0.174 mmol) and 1-methyl-4-(4,4,5,5-tetramethyl-1,3,2-dioxaborolan-2-yl)-1*H*-pyrazole (40 mg, 0.191 mmol). Reaction time: 5 hours. The solvents were evaporated and the residue was purified by flash chromatography (cyclohexane/EtOAc, gradient from 60:40 to 0:100) and preparative TLC (toluene:methanol, 95:5 - 5 elutions). The product was obtained as a white solid (5 mg, 9 % yield).
¹H NMR (500 MHz, Chloroform-*d*) *δ* (ppm) 9.88 (d, *J* = 1.2 Hz, 1H), 9.07 (d, *J* = 2.1 Hz, 1H), 8.21 (d, *J* = 8.5 Hz, 1H), 8.19 (d, *J=* 2.4 Hz, 1H), 8.07 (s, 1H), 8.03 (s, 1H), 8.00 (s, 1H), 7.56 (d, *J=* 8.4 Hz, 1H), 6.76 (d, *J=* 1.5 Hz, 1H), 4.00 (s, 3H).
¹³C NMR (126 MHz, Chloroform-*d*) *δ* (ppm) 152.52, 150.13, 149.62, 145.42, 137.74, 133.57, 131.43, 131.35, 129.25, 129.00, 127.11, 123.50, 116.18, 116.10, 96.96, 39.32.
HRMS (APCI): calcd. for C₁₇H₁₃N₆S [M+H]⁺ = 333.0917, found [M+H]⁺ = 333.0915.
FTIR (neat), cm⁻¹ 3422, 3084, 2925, 1619, 1580, 1556, 1444, 1220, 980, 809, 723.

### Preparative Example 56: 5-chloro-3-(imidazo[1,2-a]pyridin-6-yl)thieno(3,2-b]pyridine (intermediate)

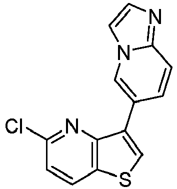

The compound was prepared according to General procedure A using 3-bromo-5-chlorothieno[3,2-b]pyridine (80 mg, 0.322 mmol) and 6-(4,4,5,5-tetramethyl-1,3,2-dioxaborolan-2-yl)imidazo[1,2-*a*]pyridine (94 mg, 0.386 mmol). Reaction time: 2 hours. The solvents were evaporated and the residue was purified by flash chromatography (cyclohexane/EtOAc, gradient from 80:20 to 0:100). The product was obtained as a brown solid (41 mg, 45 % yield).
¹H NMR (500 MHz, Chloroform-*d*) *δ* (ppm) 9.46 (s, 1H), 8.19 (d, *J* = 8.5 Hz, 1H), 8.01 (s, 1H), 7.82 (d, *J* = 9.5 Hz, 1H), 7.76 (s, 2H), 7.72 - 7.66 (m, 1H), 7.39 (d, *J* = 8.5 Hz, 1H).
¹³C NMR (126 MHz, Chloroform-*d*) *δ* (ppm) 152.30, 149.21, 143.68, 133.26, 132.92, 132.26, 131.30, 128.61, 126.08, 125.37, 120.05, 119.85, 117.10, 113.63.
HRMS (APCI): calcd. for C₁₄H₉ClN₃S [M+H]⁺ = 286.0200, found [M+H]⁺ = 286.0200.
FTIR (neat), cm⁻¹: 2917, 2850, 1694, 1563, 1522, 1366, 1146, 1127, 837, 803.

### Preparative Example 57: 3-(imidazo[1,2-a]pyridin-6-yl)-5-(1-methyl-1H-pyrazol-4-yl)thieno[3,2-b]pyridine

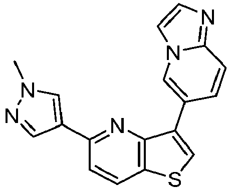

The compound was prepared according to General procedure B using 5-chloro-3-(imidazo[1,2-*a*]pyridin-6-yl)thieno[3,2-b]pyridine **(Prep. Example 56)** (50 mg, 0.175 mmol) and 1-methyl-4-(4,4,5,5-tetramethyl-1,3,2-dioxaborolan-2-yl)-1*H*-pyrazole (40 mg, 0.192 mmol). Reaction time: 4 hours. The solvents were evaporated and the residue was purified by flash chromatography (EtOAc/methanol, gradient from 100:0 to 95:5) and preparative TLC (toluene:methanol, 95:5 - 5 elutions). The product was obtained as a white solid (15 mg, 26 % yield).
¹H NMR (500 MHz, Chloroform-*d*) *δ* (ppm) 9.50 (s, 1H), 8.18 (d, *J* = 8.4 Hz, 1H), 8.07 (s, 1H), 7.96 (s, 1H), 7.89 (d, *J* = 1.6 Hz, 1H), 7.76 - 7.66 (m, 4H), 7.52 (d, *J* = 8.5 Hz, 1H), 4.00 (s, 3H).
¹³C NMR (126 MHz, Chloroform-*d*) *δ* (ppm) 152.75, 149.68, 144.67, 137.89, 133.82, 132.04, 131.67, 131.39, 128.84, 126.86, 125.53, 125.29, 123.94, 120.08, 117.34, 115.91, 113.10, 39.29.
HRMS (APCI): calcd. for C₁₈H₁₄N₅S [M+H]⁺ = 332.0964, found [M+H]⁺ = 332.0961.
FTIR (neat), cm⁻¹: 3396, 3088, 2925, 1579, 1549, 1400, 1312, 1217, 1143, 857.

### Preparative Example 58: 3-([1,2,4]triazolo[4,3-a]pyridin-6-yl)-5-chlorothieno[3,2-b]yridine (intermediate)

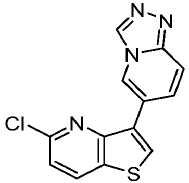

The compound was prepared according to General procedure A using 3-bromo-5-chlorothieno[3,2-*b*]pyridine (150 mg, 0.603 mmol) and 6-(4,4,5,5-tetramethyl-1,3,2-dioxaborolan-2-yl)-[1,2,4]triazolo[4,3-*a*]pyridine (177 mg, 0.724 mmol). Reaction time: 4 hours. The solvents were evaporated and the residue was purified by flash chromatography (cyclohexane/EtOAc, gradient from 100:0 to 70:30). The product was obtained as a brown solid (107 mg, 62 % yield).
¹H NMR (500 MHz, Methanol-*d₄*) *δ* (ppm) 9.59 (s, 1H), 9.33 (s, 1H), 8.50 (s, 1H), 8.45 (d, *J =* 8.5 Hz, 1H), 8.06 (d, *J =* 9.4 Hz, 1H), 7.85 (d, *J =* 9.6 Hz, 1H), 7.50 (d, *J =* 8.5 Hz, 1H).
¹³CNMR (126 MHz, Methanol-*d₄*) *δ* (ppm) 153.50, 150.37, 149.72, 138.60, 135.33, 134.67, 132.44, 131.43, 131.03, 124.26, 122.79, 121.19, 115.60.
HRMS (APCI): calcd. for C₁₃H₈ClN₄S [M+H]⁺ = 287.0153, found [M+H]⁺ = 287.0154.
FTIR (neat), cm⁻¹: 3402, 3064, 2005, 1584, 1564, 1529, 1487, 1437, 1146, 1127, 799.

### Preparative Example 59: 3-([1,2,4]triazolo[4,3-a]pyridin-6-yl)-5-(1-methyl-1H-pyrazol-4-yl)thieno[3,2-b]ridine

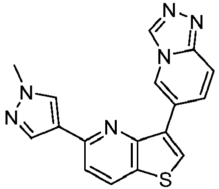

The compound was prepared according to General procedure B using 3-([1,2,4]triazolo[4,3-*a*]pyridin-6-yl)-5-chlorothieno[3,2-*b*]pyridine **(Prep. Example 58)** (40 mg, 0.139 mmol) and 1-methyl-4-(4,4,5,5-tetramethyl-1,3,2-dioxaborolan-2-yl)-1*H*-pyrazole (34 mg, 0.167 mmol). Reaction time: 4 hours. The solvents were evaporated and the residue was purified by flash chromatography (cyclohexane/EtOAc/methanol, gradient from 50:50:0 to 0:95:5) and preparative TLC (EtOAc/methanol, 95:5). The product was obtained as a white solid (10 mg, 22 % yield).
¹H NMR (500 MHz, DMSO-*d₆*) *δ* (ppm) 9.90 (s, 1H), 9.57 (s, 1H), 8.64 (s, 1H), 8.55 (d, *J* = 8.5 Hz, 1H), 8.49 (s, 1H), 8.24 (s, 1H), 8.06 (dd, *J* = 9.6, 1.6 Hz, 1H), 7.92 (d, *J=* 9.6 Hz, 1H), 7.79 (d, *J* = 8.5 Hz, 1H), 3.96 (s, 3H).
¹³C NMR (126 MHz, DMSO-*d₆*) *δ* (ppm) 151.99, 149.76, 147.60, 137.70, 137.67, 132.37, 130.97, 130.11, 129.82, 129.48, 128.68, 122.66, 122.63, 120.39, 116.07, 114.86, 38.80.
HRMS (APCI): calcd. for C₁₇H₁₃N₆S [M+H]⁺ = 333.0917, found [M+H]⁺ = 333.0914.
FTIR (neat), cm⁻¹: 1576, 1549, 1402, 1220, 994, 874, 771, 655, 492.

### Preparative Example 60: 5-chloro-3-(pyrazolo[1,5-a]pyrimidin-2-yl)thieno[3,2-b]pyridine (intermediate)

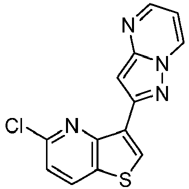

To a solutiom of 3-bromo-5-chlorothieno[3,2-*b*]pyridine (50 mg, 0.021 mmol) in dioxane (1 mL) were added potassium carbonate (87 mg, 0.063 mmol), SPhosPdG3 (16 mg, 0.002 mmol), SPhos (4 mg, 0.011 mmol), 6-(4,4,5,5-tetramethyl-1,3,2-dioxaborolan-2-yl)-[1,2,4]triazolo[1,5-*a*]pyridine (59 mg, 0.024 mmol) and H₂O (0.3 mL) and the reaction mixture was refluxed for 4 hours. The solvents were evaporated and the residue was purified by flash chromatography (cyclohexane/EtOAc, gradient from 70:30 to 40:60). The product was obtained as a white solid (42 mg, 72 %).
¹H NMR (500 MHz, Chloroform-*d*) *δ* 9.54 (s, 1H), 8.80 - 8.76 (m, 2H), 8.60 (dd, *J* = 4.0, 1.7 Hz, 1H), 8.15 (d, *J* = 8.4 Hz, 1H), 7.33 (d, *J* = 8.4 Hz, 1H), 6.90 (dd, *J* = 7.0, 4.0 Hz, 1H).
¹³C NMR (126 MHz, Chloroform-*d*) *δ* 153.54, 149.28, 148.78, 145.65, 143.97, 135.39, 132.80, 131.92, 126.78, 126.58, 119.30, 108.18, 104.67.
HRMS (APCI): calcd. for C₁₃H₈ClN₄S [M+H]⁺ = 287.0153, found [M+H]⁺ = 287.0150.
FTIR (neat), cm⁻¹: 1614, 1561, 1520, 1387, 1297, 1144, 1121, 839, 778, 752, 716, 578, 453.

### Preparative Example 61: 5-(1-methyl-1H-pyrazol-4-yl)-3-(pyrazolo[1,5-a]pyrimidin-2-yl)thieno[3,2-b]pyridine

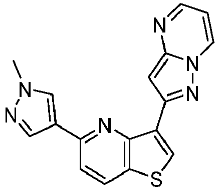

The compound was prepared according to General procedure B using 5-chloro-3-(pyrazolo[1,5-*a*]pyrimidin-2-yl)thieno[3,2-*b*]pyridine **(Prep. Example 60)** (42 mg, 0.146 mmol) and 1-methyl-4-(4,4,5,5-tetramethyl-1,3,2-dioxaborolan-2-yl)-1*H*-pyrazole (37 mg, 0.176 mmol). Reaction time: 4 hours. The solvents were evaporated and the residue was purified by flash chromatography (cyclohexane/EtOAc, gradient from 20:80 to 0:100). The product was obtained as a white solid (15 mg, 31 % yield).
¹H NMR (500 MHz, Chloroform-*d*) *δ* (ppm) 9.72 (s, 1H), 8.77 (dd, *J* = 6.9, 1.8 Hz, 1H), 8.72 (s, 1H), 8.61 (dd, *J* = 4.1, 1.7 Hz, 1H), 8.18 (d, *J* = 8.4 Hz, 1H), 8.10 (d, *J=* 7.8 Hz, 2H), 7.51 (d, *J* = 8.4 Hz, 1H), 6.90 (dd, *J* = 7.0, 4.0 Hz, 1H), 4.02 (s, 3H).
¹³C NMR (126 MHz, Chloroform-*d*) *δ* (ppm) 153.89, 149.37, 149.12, 145.69, 144.20, 137.78, 135.30, 131.05, 130.78, 129.05, 126.92, 125.44, 124.28, 115.34, 108.10, 105.55, 39.25.
HRMS (APCI): calcd. for C₁₇H₁₃N₆S [M+H]⁺ = 333.0917, found [M+H]⁺ = 333.0919.
FTIR (neat), cm⁻¹: 2924, 2854, 1733, 1523, 1436, 1419, 1271, 1262, 1066, 971, 842, 801, 652, 599.

### Preparative Example 62: 5-chloro-3-(1-methyl-1H-pyrazol-4-yl)thieno[3,2-b]pyridine (intermediate)

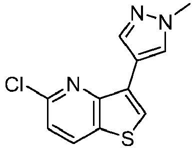

The compound was prepared according to General procedure A using 3-bromo-5-chlorothieno[3,2-*b*]pyridine (205 mg, 0.825 mmol) and 1-methyl-4-(4,4,5,5-tetramethyl-1,3,2-dioxaborolan-2-yl)-1*H-*pyrazole (206 mg, 0.990 mmol). Reaction time: 48 hours. The solvents were evaporated and the residue was purified by flash chromatography (cyclohexane/EtOAc, gradient from 100:0 to 70:30). The product was obtained as a white solid (30 mg, 15 % yield).
¹H NMR (500 MHz, Chloroform-*d*) *δ* (ppm) 8.44 (s, 1H), 8.11 (d, *J* = 8.4 Hz, 1H), 7.98 (s, 1H), 7.75 (s, 1H), 7.31 (d, *J* = 8.4 Hz, 1H), 4.01 (s, 3H).
¹³C NMR (126 MHz, Chloroform-*d*) *δ* (ppm) 152.87, 148.88, 137.31, 132.88, 132.38, 128.76, 128.56, 124.01, 119.47, 115.25, 39.13.
HRMS calcd. for C₁₁H₉ClN₃S [M+H]⁺250.020, found [M+H]⁺= 250.0202.
FTIR (neat), cm⁻¹: 2922, 2851, 1560, 1531, 1382, 1142, 1123, 981, 839, 795, 719.

### Preparative Example 63: 3,5-bis(1-methyl-1H-pyrazol-4-yl)thieno[3,2-b]pyridine

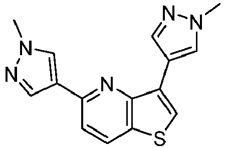

The compound was prepared according to General procedure B using 5-chloro-3-(1-methyl-1*H*-pyrazol-4-yl)thieno[3,2-b]pyridine **(Prep. Example 62)** (26 mg, 0.104 mmol) and 1-methyl-4-(4,4,5,5-tetramethyl-1,3,2-dioxaborolan-2-yl)-1H-pyrazole (26 mg, 0.125 mmol). Reaction temperature: 95 °C. Reaction time: 16 hours. The solvents were evaporated and the residue was purified by flash chromatography (cyclohexane/EtOAc, gradient from 80:20 to 0:100). The product was obtained as a white solid (13 mg, 45 % yield).
¹H NMR (500 MHz, Chloroform-*d*) *δ* (ppm) 8.39 (s, 1H), 8.15 (s, 1H), 8.13 (d, *J=* 8.4 Hz, 1H), 8.07 (s, 1H), 7.99 (s, 1H), 7.68 (s, 1H), 7.47 (d, *J=* 8.4 Hz, 1H), 4.03 (s, 3H), 4.01 (s, 3H).
¹³C NMR (126 MHz, Chloroform-*d*) *δ* (ppm) 153.18, 149.42, 137.91, 137.78, 131.23, 131.06, 128.83, 128.76, 128.50, 124.23, 122.85, 116.22, 115.62, 39.26, 39.15.
HRMS calcd. for C₁₅H₁₄N₅S [M+H]⁺296.0964, found [M+H]⁺ = 296.0967.
FTIR (neat), cm⁻¹: 3098, 2936, 1578, 1541, 1406, 1370, 1217, 1184, 1168, 1057, 980, 866, 833, 807, 778, 702, 645, 612, 593.

### Preparative Example 64: 3-(1-benzyl-1H-pyrazol-4-yl)-5-chlorothieno[3,2-b]pyridine (intermediate)

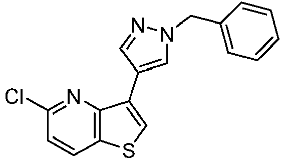

The compound was prepared according to General procedure A using 3-bromo-5-chlorothieno[3,2-b]pyridine **(Prep. Example 64)** (200 mg, 0.804 mmol) and 1-benzyl-4-(4,4,5,5-tetramethyl-1,3,2-dioxaborolan-2-yl)-1*H*-pyrazole (251 mg, 0.884 mmol). Reaction temperature: 80 °C. Reaction time: 3 hours. The solvents were evaporated and the residue was purified by flash chromatography (hexane/EtOAc, gradient from 100:0 to 50:50). The product was obtained as a white solid (111 mg, 42 % yield).
¹H NMR (500 MHz, Methanol-*d*₄) *δ* (ppm) 8.53 (s, 1H), 8.30 (d, *J* = 8.5 Hz, 1H), 8.21 (s, 1H), 8.08 (s, 1H), 7.39 - 7.34 (m, 3H), 7.31 (t, *J* = 3.2 Hz, 2H), 7.30 - 7.28 (m, 1H), 5.42 (s, 2H).
¹³CNMR (126 MHz, Methanol-*d*₄) *δ* (ppm) 153.99, 149.95, 138.85, 138.22, 134.70, 133.99, 129.80, 129.03, 129.00, 128.65, 126.64, 120.46, 117.16, 56.68.
HRMS calcd. for C₁₇H₁₃ClN₃S [M+H]⁺326.0513, found [M+H]⁺= 326.0514.
FTIR (neat), cm⁻¹: 3042, 1529, 1438, 1163, 1055, 832, 762, 719, 703, 474.

### Preparative Example 65: 3-(1-benzyl-1H-pyrazol-4-yl)-5-chlorothieno[3,2-b]pyridine

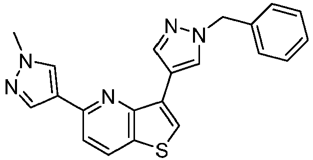

The compound was prepared according to General procedure B using 5-chloro-3-phenylthieno[3,2-*b*]pyridine **(Prep. Example 64)** (43 mg, 0.132 mmol), 1-methyl-4-(4,4,5,5-tetramethyl-1,3,2-dioxaborolan-2-yl)-1*H*-pyrazole (35 mg, 0.168 mmol) and triethylamine (0.1 mL, 0.396 mmol) instead of potassium phosphate. After 2 hours, additional triethylamine (0.2 mL, 0.343 mmol) was added and the mixture was refluxed for additional 6 hours. The solvents were evaporated and the residue was purified by preparative TLC (hexane/EtOAc, 25:75). The product was obtained as a white solid (13 mg, 26 % yield).
¹H NMR (500 MHz, Chloroform-*d*) *δ* (ppm) 8.40 (s, 1H), 8.21 (s, 1H), 8.11 (d, *J* = 8.4 Hz, 1H), 7.97 (s, 1H), 7.84 (s, 1H), 7.68 (s, 1H), 7.45 (d, *J* = 8.4 Hz, 1H), 7.43 - 7.38 (m, 2H), 7.36 (t, *J* = 6.1 Hz, 3H), 5.43 (s, 2H), 3.98 (s, 3H).
¹³C NMR (126 MHz, Chloroform-*d*) *δ* (ppm) 153.12, 149.36, 137.94, 137.81, 136.41, 131.16, 131.02, 128.91, 128.76, 128.65, 128.19, 128.08, 127.77, 124.14, 122.87, 116.43, 115.50, 56.32, 39.23.
HRMS calcd. for C₂₁H₁₈N₅S [M+H]⁺= 372.1277, found [M+H]⁺= 372.1277.
FTIR (neat), cm⁻¹: 3147, 2922, 2852, 1574, 1374, 1179, 1052, 980, 850, 782, 709, 615, 576.

### Preparative Example 66: 5-chloro-3-(isothiazol-5-yl)thieno[3,2-b]pyridine (intermediate)

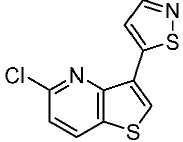

The compound was prepared according to General procedure A using 3-bromo-5-chlorothieno[3,2-b]pyridine (400 mg, 1.61 mmol) and 5-(4,4,5,5-tetramethyl-1,3,2-dioxaborolan-2-yl)isothiazole (408 mg, 1.93 mmol). Reaction time: 5 hours. The solvents were evaporated and the residue was purified by flash chromatography (hexane/EtOAc, gradient from 90:10 to 80:20). The product was obtained as a white solid (246 mg, 40 % yield).
¹H NMR (500 MHz, Chloroform-*d*) *δ* (ppm) 8.53 *(d, J=* 1.8 Hz, 1H), 8.16 *(d, J=* 8.4 Hz, 1H), 8.11 (s, 1H), 7.84 (d, *J* = 1.8 Hz, 1H), 7.38 (d, *J* = 8.5 Hz, 1H).
¹³C NMR (126 MHz, Chloroform-*d*) *δ* (ppm) 156.99, 156.58, 151.79, 149.66, 133.07, 131.99, 128.38, 127.57, 120.44, 120.42.
HRMS (APCI): calcd. for C₁₀H₆ClN₂S₂ [M+H]⁺ = 252.9655, found [M+H]⁺ = 252.9656.
FTIR (neat), cm⁻¹: 3086, 3052, 1561, 1543, 1410, 1390, 1327, 1161, 1125, 838, 796, 757.

### Preparative Example 67: 3-(isothiazol-5-yl)-5-(1-methyl-1H-pyrazol-4-yl)thieno[3,2-b]pyridine

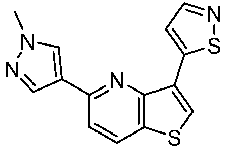

The compound was prepared according to General procedure B using 5-chloro-3-(isothiazol-5-yl)thieno[3,2-*b*]pyridine **(Prep. Example 66)** (45 mg, 0.178 mmol) and 1-methyl-4-(4,4,5,5-tetramethyl-1,3,2-dioxaborolan-2-yl)-1*H*-pyrazole (44 mg, 0.214 mmol). Reaction time: 45 min. The solvents were evaporated and the residue was purified by flash chromatography (hexane/EtOAc, gradient from 100:0 to 50:50). The product was obtained as awhite solid (32 mg, 70 % yield).
¹H NMR (500 MHz, Chloroform-*d*) *δ* (ppm) 8.52 (d, *J* = 1.8 Hz, 1H), 8.18 (s, 1H), 8.14 (d, *J* = 8.5 Hz, 1H), 8.08 (s, 1H), 8.07 (s, 1H), 7.73 (d, *J=* 1.8 Hz, 1H), 7.53 (d, *J=* 8.5 Hz, 1H), 4.03 (s, 3H).
¹³C NMR (126 MHz, Chloroform-*d*) *δ* (ppm) 157.44, 155.77, 151.96, 150.09, 137.83, 131.26, 130.29, 129.75, 128.03, 126.53, 123.49, 119.35, 116.04, 39.33.
HRMS (APCI): calcd. for C₁₄H₁₁N₄S₂ [M+H]⁺ = 299.0420, found [M+H]⁺ = 299.0424.
FTIR (neat), cm⁻¹: 1608, 1546, 1400, 1255, 1167, 993, 828, 808, 784,746, 649, 551, 490, 432.

### Preparative Example 68: 5-chloro-3-(thiazol-5-yl)thieno[3,2-b]pyridine (intermediate)

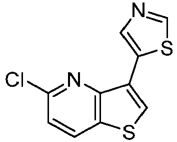

The compound was prepared according to General procedure A using 3-bromo-5-chlorothieno[3,2-*b*]pyridine (250 mg, 1.01 mmol) and 5-(4,4,5,5-tetramethyl-1,3,2-dioxaborolan-2-yl)thiazole (256 mg, 1.21 mmol). Reaction time: 4 hours. The solvents were evaporated and the residue was purified by flash chromatography (hexane/EtOAc, gradient from 70:30 to 30:70). The product was obtained as a white solid (47 mg, 19 % yield).
¹H NMR (500 MHz, Chloroform-*d*) *δ* (ppm) 8.85 (s, 1H), 8.66 (s, 1H), 8.15 (d, *J =* 8.4 Hz, 1H), 7.97 (s, 1H), 7.37 (d, *J* = 8.4 Hz, 1H).
¹³C NMR (126 MHz, Chloroform-*d*) *δ* (ppm) 152.75, 151.91, 149.45, 141.13, 133.03, 132.25, 129.96, 127.66, 127.32, 120.26.
HRMS (APCI): calcd. for C₁₀H₆ClN₂S₂ [M+H]⁺ = 252.9655, found [M+H]⁺ = 252.9657.
FTIR (neat), cm⁻¹: 3071, 1561, 1526, 1387, 1149, 1124, 1107, 871, 845, 786, 762, 708, 600, 471.

### Preparative Example 69: 5-(1-methyl-1H-pyrazol-4-yl)-3-(thiazol-5-yl)thieno[3,2-b]pyridine

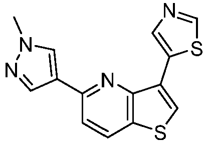

The compound was prepared according to General procedure B using 5-chloro-3-(thiazol-5-yl)thieno[3,2-b]pyridine **(Prep. Example 68)** (40 mg, 0.158 mmol) and 1-methyl-4-(4,4,5,5-tetramethyl-1,3,2-dioxaborolan-2-yl)-1*H*-pyrazole (39 mg (0.189 mmol). Reaction time: 16 hours. The solvents were evaporated and the residue was purified by flash chromatography (hexane/EtOAc, gradient from 40:60 to 0:100). The product was obtained as a brown solid (29 mg, 62 % yield).
¹H NMR (500 MHz, Chloroform-*d*) *δ* (ppm) 8.86 (s, 1H), 8.70 (s, 1H), 8.15 (d, *J=* 8.5 Hz, 1H), 8.09 (d, *J* = 4.0 Hz, 2H), 7.94 (s, 1H), 7.52 (d, *J* = 8.5 Hz, 1H), 4.01 (s, 3H).
¹³C NMR (126 MHz, Chloroform-*d*) *δ* (ppm) 152.58, 151.98, 149.87, 140.55, 137.87, 131.22, 130.85, 130.68, 129.31, 127.40, 126.13, 123.76, 115.97, 39.27.
HRMS (APCI): calcd. for C₁₄H₁₁N₄S₂ [M+H]⁺ = 299.0420, found [M+H]⁺ = 299.0421.
FTIR (neat), cm⁻¹: 3080, 1579, 1404, 1215, 990, 872, 836, 813, 605.

### Preparative Example 70: 5-chloro-3-(3-methyl-1,2,4-thiadiazol-5-yl)thieno[3,2-b]pyridine (intermediate)

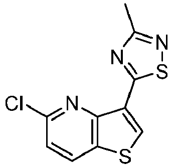

To a solution of 3-bromo-5-chlorothieno[3,2-b]pyridine (100 mg, 0.402 mmol) in dioxane (4 mL) was added 3-methyl-5-(tributylstannyl)-1,2,4-thiadiazole (157 µL, 0.482 mmol) and the reaction mixture was refluxed for 16 hours. The solvents were evaporated and the residue was purified by flash chromatography (hexane/EtOAc, gradient from 100:0 to 70:30). The product was obtained as a white solid (37 mg, 34 % yield).
¹H NMR (500 MHz, Chloroform-*d*) *δ* (ppm) 8.74 (s, 1H), 8.20 (d, *J=* 8.5 Hz, 1H), 7.42 (d, *J=* 8.4 Hz, 1H), 2.76 (s, 3H).
¹³C NMR (126 MHz, Chloroform-*d*) *δ* (ppm) 177.45, 171.89, 151.86, 149.90, 133.24, 132.41, 132.00, 127.79, 120.70, 18.74.
HRMS (APCI): calcd. for C₁₀H₇ClN₃S₂ [M+H]⁺ = 267.9764, found [M+H]⁺ = 267.9766.
FTIR (neat), cm⁻¹: 2977, 1714, 1609, 1578, 1565, 1381, 1263, 1164, 1124, 845, 790, 737, 659.

### Preparative Example 71: 3-(3-methyl-1,2,4-thiadiazol-5-yl)-5-(1-methyl-1H-pyrazol-4-yl)thieno[3,2-b]pyridine

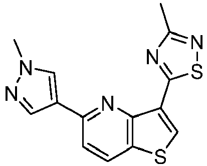

The compound was prepared according to General procedure B using 5-chloro-3-(3-methyl-1,2,4-thiadiazol-5-yl)thieno[3,2-b]pyridine **(Prep. Example 70)** (40 mg, 0.149 mmol) and 1-methyl-4-(4,4,5,5-tetramethyl-1,3,2-dioxaborolan-2-yl)-1*H*-pyrazole (37 mg, 0.178 mmol). Reaction time: 2 hours. The solvents were evaporated and the residue was purified by flash chromatography (cyclohexane/EtOAc, gradient from 80:20 to 0:100). The product was obtained as a brown solid (20 mg, 44 % yield).
¹H NMR (500 MHz, DMSO-*d₆*) *δ* (ppm) 9.00 (s, 1H), 8.60 (d, *J* = 8.4 Hz, 1H), 8.51 (s, 1H), 8.22 (s, 1H), 7.85 (d, *J* = 8.5 Hz, 1H), 3.97 (s, 3H), 2.68 (s, 3H).
¹³C NMR (126 MHz, DMSO-*d₆*) *δ* (ppm) 177.36, 171.12, 151.14, 150.15, 137.65, 133.55, 132.83, 130.22, 126.35, 122.16, 116.65, 38.83, 18.49.
HRMS (APCI): calcd. for C₁₄H₁₂N₅S₂ [M+H]⁺ = 314.0529, found [M+H]⁺ = 314.0527.
FTIR (neat), cm⁻¹: 1581, 1546, 1366, 1290, 982, 819, 806, 694.

### Preparative Example 72: (5-chlorothieno[3,2-b]pyridin-3-yl)(phenyl)methanone (intermediate)

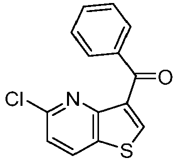

To a solution of 3-bromo-5-chlorothieno[3,2-b]pyridine (500 mg, 2.01 mmol) in hexamethylphosphoramide (8 mL) were added tributyl(phenyl)stannane (659 µL, 2.01 mmol) and bis(triphenylphosphine) palladium(II) dichloride (113 mg, 0.161 mmol) and the reaction mixture was stirred at 75 °C under 10 atm in pressence of carbon monoxide for 60 hours. The reaction mixture was quenched with water (15 mL) and extracted with EtOAc (2 × 15 mL). The combined organic extracts were washed water (2 x 15 mL) and brine (10 mL), dried over MgSO4, filtered, and the solvents were evaporated *in vacuo.* The solvents were evaporated and the residue was purified by flash chromatography (cyclohexane/EtOAc, gradient from 98:2 to 80:20). The product was obtained as a yellow wax (95 mg, 17 %).
¹H NMR (500 MHz, Chloroform-*d*) *δ* (ppm) 8.21 (s, 1H), 8.17 (d, *J =* 8.5 Hz, 1H), 7.91 (dd, *J* = 8.2, 1.1 Hz, 2H), 7.63 - 7.58 (m, 1H), 7.48 (t, *J* = 7.8 Hz, 2H), 7.36 (d, *J* = 8.5 Hz, 1H).
¹³C NMR (126 MHz, Chloroform-*d δ* (ppm) 189.59, 152.99, 150.18, 137.69, 137.66, 135.75, 133.27, 132.72, 132.16, 130.17, 128.35, 120.55.
HRMS (APCI): calcd. for C₁₄H₉ClNOS [M+H]⁺ = 274.0088, found [M+H]⁺ = 274.0088.
FTIR (neat), cm⁻¹: 3093, 3065, 1655, 1563, 1385, 1323, 1237, 1148, 1125, 846, 724, 696, 671.

### Preparative Example 73: (5-(1-methyl-1H-pyrazol-4-yl)thieno[3,2-b]pyridin-3-yl)(phenyl)-methanone

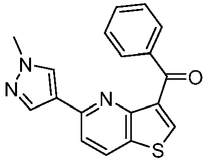

The compound was prepared according to General procedure B using (5-chlorothieno[3,2-b]pyridin-3-yl)(phenyl)methanone **(Prep. Example 72)** (30 mg, 0.109 mmol) and 1-methyl-4-(4,4,5,5-tetramethyl-1,3,2-dioxaborolan-2-yl)-1*H*-pyrazole (27 mg, 0.131 mmol). Reaction temperature: 110 °C. Reaction time: 1.5 hours. The solvents were evaporated and the residue was purified by flash chromatography (cyclohexane/EtOAc, gradient from 70:30 to 50:50). The product was obtained as a pale yellow wax (27 mg, 77 %).
¹H NMR (500 MHz, Chloroform-*d*) δ (ppm) 8.29 (s, 1H), 8.17 (d, *J* = 8.4 Hz, 1H), 7.92 (dd, *J* = 8.3, 1.1 Hz, 2H), 7.81 (s, 1H), 7.73 (s, 1H), 7.61 (t, *J =* 7.4 Hz, 1H), 7.48 (t, *J* = 7.7 Hz, 3H), 3.89 (s, 3H).
¹³C NMR (126 MHz, Chloroform-*d*) δ (ppm) 190.69, 153.02, 150.31, 138.53, 137.75, 137.71, 136.17, 132.67, 131.06, 130.92, 130.23, 129.54, 128.04, 123.56, 116.02, 39.12.
HRMS (APCI): calcd. for C₁₈H₁₄N₃OS [M+H]⁺ = 320.0852, found [M+H]⁺ = 320.0853.
FTIR (neat), cm⁻¹: 1652, 1578, 1538, 1400, 1214, 1177, 985, 839, 726, 694, 661.

### Preparative Example 74: (5-(1-methyl-1H-pyrazol-4-yl)thieno[3,2-b]pyridin-3-yl)(phenyl)methanone

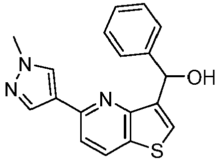

To a solution of (5-(1-methyl-1*H*-pyrazol-4-yl)thieno[3,2-*b*]pyridin-3-yl)(phenyl)methanone (18 mg, 0.056 mmol) **(Prep. Example 73)** in methanol (1.5 mL) was added sodium tetrahydridoborate (3 mg, 1.3 mmol) and the reaction mixture was stirred at 25 °C for 30 min. Additional sodium tetrahydridoborate (3 mg, 1.3 mmol) was added and the reaction mixture was warmed to 30 °C for 2 hours and then stirred at 25 °C for 16 hours. NMR analysis of the crude mixture showed full conversion. The solvents were evaporated and the residue was purified by flash chromatography (cyclohexane/EtOAc, gradient from 20:80 to 0:100). The product was obtained as a white solid (13 mg, 72 %).
¹H NMR (500 MHz, Chloroform-*d*) *δ* (ppm) 8.13 (d, *J* = 8.5 Hz, 1H), 8.02 (s, 1H), 8.00 (s, 1H), 7.56 (d, *J* = 7.4 Hz, 2H), 7.48 (d, *J* = 8.5 Hz, 1H), 7.40 (t, *J* = 7.6 Hz, 2H), 7.32 (t, *J* = 7.3 Hz, 1H), 7.13 (s, 1H), 6.28 (s, 1H), 6.13 (s, 1H), 3.99 (s, 3H).
¹³C NMR (126 MHz, Chloroform-*d*) *δ* (ppm) 148.67, 142.31, 138.71, 137.78, 131.72, 131.63, 129.05, 128.44, 127.74, 127.17, 126.86, 115.82, 72.96, 39.27.
HRMS (APCI): calcd. for C₁₈H₁₆N₃OS [M+H]⁺ 322.1009, found [M+H]⁺ = 322.1008.
FTIR (neat), cm⁻¹: 3550 - 3087, 2923, 2853, 1717, 1654, 1580, 1493, 1454, 1215, 982, 824, 700.

### Preparative Example 75: 5-chloro-N-phenylthieno[3,2-b]pyridin-3-amine (intermediate)

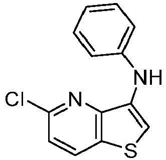

To a solution of 3-bromo-5-chlorothieno[3,2-*b*]pyridine (200 mg, 0.804 mmol) in toluene (4 mL) were added aniline (87 µL, 0.960 mmol), sodium tert-butoxide (85 mg, 0.884 mmol) and Bis(tri-tert-butylphosphine)palladium(0) (21 mg, 0.040 mmol) and the reaction mixture was refluxed for 16 hours. The solvents were evaporated and the residue was purified by flash chromatography (cyclohexane/EtOAc, 90:10). The product was obtained as a pale yellow solid (149 mg, 71 %).
¹H NMR (500 MHz, Chloroform-*d*) *δ* 8.07 (d, *J* = 8.4 Hz, 1H), 7.38 - 7.31 (m, 3H), 7.29 - 7.26 (m, 2H), 7.07 (br s, 1H), 7.00 - 6.95 (m, 1H).
¹³C NMR (126 MHz, Chloroform-*d*) *δ* 148.11, 148.04, 142.26, 134.36, 133.17, 130.57, 129.47, 121.20, 120.25, 117.06, 102.12.
HRMS (APCI): calcd. for C₁₃H₁₀ClN₂S [M+H]⁺ = 261.0248, found [M+H]⁺ = 261.0251.
FTIR (neat), cm⁻¹: 3381, 1593, 1570, 1532, 1391, 1156, 1119, 750, 738, 692.

### Preparative Example 76: 5-(1-methyl-1H-pyrazol-4-yl)-N-phenylthieno[3,2-b]pyridin-3-amine

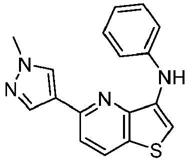

The compound was prepared according to General procedure B using 5-chloro-N-phenylthieno[3,2-*b*]pyridin-3-amine (**Prep. Example 75)** (50 mg, 0.191 mmol) and 1-methyl-4-(4,4,5,5-tetramethyl-1,3,2-dioxaborolan-2-yl)-1*H*-pyrazole (48 mg, 0.229 mmol). Reaction time: 1.5 hours. The solvents were evaporated and the residue was purified by flash chromatography (cyclohexane/EtOAc, gradient from 50:50 to 40:60). The product was obtained as a pale yellow solid (44 mg, 76 %).
¹H NMR (500 MHz, Chloroform-d) *δ* 8.10 (d, *J* = 8.3 Hz, 1H), 8.06 (s, 2H), 7.50 (d, *J* = 8.4 Hz, 1H), 7.39 - 7.34 (m, 2H), 7.33 - 7.29 (m, 2H), 7.01 (br s, 1H), 6.99 - 6.95 (m, 1H), 4.01 (s, 3H).
¹³C NMR (126 MHz, Chloroform-d) *δ* 148.51, 142.62, 137.86, 136.62, 134.48, 131.40, 129.44, 128.97, 120.83, 116.89, 116.63, 100.67, 39.25.
HRMS (APCI): calcd. for C₁₇H₁₅N₄S [M+H]⁺ 307.1012, found [M+H]⁺ = 307.1011
FTIR (neat), cm⁻¹: 3372, 1602, 1568, 1497, 1402, 1214, 984, 820, 777, 692.

### Preparative Example 77: 5-(1-ethyl-1H-gyrazol-4-yl)-3-(pyridin-4-yl)thieno[3,2-b]pyridine

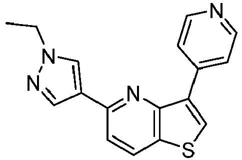

The compound was prepared according to General procedure B using 5-chloro-3-(pyridin-4-yl)thieno[3,2-b]pyridine **(Prep. Example 12)** (60 mg, 0.243 mmol) and 1-ethyl-4-(4,4,5,5-tetramethyl-1,3,2-dioxaborolan-2-yl)-1*H*-pyrazole (65 mg, 0.291 mmol). Reaction time: 1.5 hours. The solvents were evaporated and the residue was purified by flash chromatography (cyclohexane/EtOAc, gradient from 100:0 to 0:100). The product was obtained as a white solid (60 mg, 81 % yield).
¹H NMR (500 MHz, Chloroform-*d*) *δ* (ppm) 8.72 (d, *J* = 5.2 Hz, 2H), 8.16 (d, *J* = 8.5 Hz, 1H), 8.07 (d, *J* = 5.9 Hz, 2H), 8.05 (s, 1H), 8.01 (s, 2H), 7.51 (d, *J =* 8.5 Hz, 1H), 4.25 (q, *J* = 7.3 Hz, 2H), 1.56 (t, *J* = 7.3 Hz, 3H).
¹³C NMR (126 MHz, Chloroform-*d*) *δ* (ppm) 152.74, 150.02, 149.84, 141.88, 137.66, 133.60, 131.52, 131.22, 129.46, 127.41, 123.47, 122.65, 115.88, 47.34, 15.52.
HRMS (APCI): calcd. for C₁₇H₁₅N₄S [M+H]⁺ = 307.1012, found [M+H]⁺ = 307.1013.
FTIR (neat), cm⁻¹: 2925, 1712, 1598, 1577, 1444, 1385, 1209, 993, 958, 828, 812, 791, 643, 561.

### Preparative Example 78: 3-(pyridin-4-yl)-5-(1-(trifluoromethyl)-1H-pyrazol-4-yl)thieno[3,2-b]pyridine

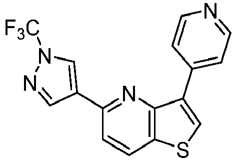

The compound was prepared according to General procedure B using 5-chloro-3-(pyridin-4-yl)thieno[3,2-*b*]pyridine **(Prep. Example 12)** (45 mg, 0.183 mmol) and 4-(4,4,5,5-tetramethyl-1,3,2-dioxaborolan-2-yl)-1-(trifluoromethyl)-1*H*-pyrazole (57 mg, 0.220 mmol). After 1 hour, additional [1,1'-Bis(diphenyl-phosphino)ferrocene]dichloropalladium(II) (6 mg, 0.05 mmol) was added and the mixture was refluxed for additional 5 hours. The solvents were evaporated and the residue was purified by flash chromatography (cyclohexane/EtOAc, gradient from 100:0 to 70:30). The product was obtained as an orange solid (44 mg, 70 % yield).
¹H NMR (500 MHz, Chloroform-*d*) *δ* (ppm) 8.76 (dd, *J* = 4.7, 1.8 Hz, 2H), 8.39 (s, 1H), 8.34 (s, 1H), 8.28 (d, *J* = 8.4 Hz, 1H), 8.13 (s, 1H), 8.11 (dd, *J* = 4.7, 1.8 Hz, 2H), 7.60 (d, *J* = 8.4 Hz, 1H).
¹³C NMR (126 MHz, Chloroform-*d*) *δ* (ppm) 152.89, 149.18, 147.80, 142.40, 142.05, 133.54, 133.02, 131.78, 130.74, 126.38, 126.12, 122.84, 119.07, 118.03 (apparent quartet, found doublet, *J* = 263.5 Hz). ¹⁹F NMR (471 MHz, Chloroform-*d*) *δ* (ppm) -60.4582.
HRMS (APCI): calcd. for C₁₆H₁₀F₃N₄S [M+H]⁺ = 347.0573, found [M+H]⁺ = 347.0570,
FTIR (neat), cm⁻¹: 3070, 1587, 1556, 1449, 1405, 1382, 1273, 1261, 1205, 1162, 1112, 1066, 993, 951, 886, 822, 761, 553.

### Preparative Example 79: 5-(1-isoprogyl-1H-gyrazol-4-yl)-3-(pyridin-4-yl)thieno[3,2-b]pyridine

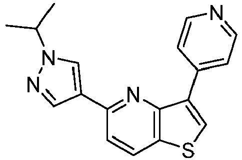

The compound was prepared according to General procedure B using 5-chloro-3-(pyridin-4-yl)thieno[3,2-b]pyridine **(Prep. Example 12)** (50 mg, 0.203 mmol) and 1-isopropyl-4-(4,4,5,5-tetramethyl-1,3,2-dioxaborolan-2-yl)-1*H*-pyrazole (60 mg, 0.254 mmol). Reaction time: 1.5 hours. The solvents were evaporated and the residue was purified by flash chromatography (cyclohexane/EtOAc, gradient from 80:20 to 50:50). The product was obtained as a white solid (43 mg, 66 % yield).
¹H NMR (500 MHz, Chloroform-*d*) *δ* (ppm) 8.74 (s, 2H), 8.18 (d, *J* = 8.1 Hz, 3H), 8.07 (s, 1H), 8.05 (d, *J* = 13.1 Hz, 2H), 7.54 (d, *J=* 8.5 Hz, 1H), 4.59 (p, *J=* 6.7 Hz, 1H), 1.59 (s, 3H), 1.58 (s, 3H).
¹³C NMR (126 MHz, Chloroform-*d*) *δ* (ppm) 152.67, 150.31, 148.68, 143.00, 137.39, 133.17, 131.50, 131.30, 130.13, 125.65, 122.99, 122.95, 116.06, 54.19, 22.91.
HRMS (APCI): calcd. for C₁₈H₁₇N₄S [M+H]⁺ = 321.1168, found [M+H]⁺ = 321.1171.
FTIR (neat), cm⁻¹: 2977, 1598, 1577, 1537, 1385, 1370, 1257, 1220, 994, 981, 828, 811, 791, 641, 561.

### Preparative Example 80: 5-(1-(tert-butyl)-1H-pyrazol-3-yl)-3-(pyridin-4-yl)thieno[3,2-b]pyridine

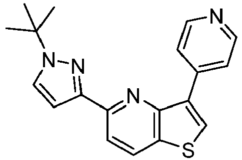

To a solution of 3-bromo-1-(*tert*-butyl)-1*H*-pyrazole (122 mg, 0.600 mmol) in dioxane (3.8 mL) were added bis(pinacolato)diboron (190 mg, 0.675 mmol), potassium acetate (236 mg, 2.40 mmol), tris(dibenzylideneacetone)dipalladium (0) (27 mg, 0.030 mmol) and 2-dicyclohexylphosphino-2',4',6'-triisopropylbiphenyl (46 mg, 0.096 mmol) and the reaction mixture was refluxed for 16 hours. Then, 5-chloro-3-(pyridin-4-yl)thieno[3,2-*b*]pyridine (184 mg, 0.675 mmol) **(Prep. Example 12),** tripotassium phosphate (381 mg, 1.80 mmol), dioxane (3 mL) and H₂O (0.9 mL) were added and the reaction mixture was refluxed for 16 hours. Then, [1,1'-Bis(diphenylphosphino)ferrocene]dichloropalladium(II) (44 mg, 0.060 mmol) and additional tripotassium phosphate (381 mg, 1.80 mmol) were added and the reaction mixture was refluxed for additional 2 hours. The solvents were evaporated and the residue was purified by flash chromatography (hexane/EtOAc, 40:60) and preparative TLC (hexane/EtOAc, 80:20). The product was obtained as a brown solid (92 mg, 46 %).
¹H NMR (500 MHz, Chloroform-*d*) *δ* (ppm) 8.74 (s, 2H), 8.37 (s, 2H), 8.25 (d, *J* = 8.5 Hz, 1H), 8.18 (d, *J* = 8.5 Hz, 1H), 8.14 (s, 1H), 7.62 (d, *J* = 2.3 Hz, 1H), 6.99 (d, *J=* 2.4 Hz, 1H), 1.67 (s, 9H).
¹³C NMR (126 MHz, Chloroform-*d*) *δ* (ppm) 152.27, 151.31, 150.92, 147.51, 144.16, 132.72, 132.66, 131.13, 130.44, 127.17, 123.14, 116.66, 103.97, 58.88, 29.92.
HRMS (APCI): calcd. for C₁₉H₁₉N₄S [M+H]⁺ = 335.1325, found [M+H]⁺ = 335.1326.
FTIR (neat), cm⁻¹: 2976, 1634, 1597, 1572, 1524, 1368, 1239, 1185, 833, 775, 647, 491.

### Preparative Example 81: 5-(1-benzyl-1H-pyrazol-4-yl)-3-(pyridin-4-yl)thieno[3,2-b]pyridine

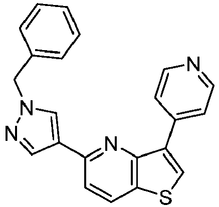

The compound was prepared according to General procedure B using 5-chloro-3-(pyridin-4-yl)thieno[3,2-b]pyridine **(Prep. Example 12)** (48 mg, 0.195 mmol) and 1-benzyl-4-(4,4,5,5-tetramethyl-1,3,2-dioxaborolan-2-yl)-1*H*-pyrazole (58 mg, 0.234 mmol). Reaction time: 1.5 hours. The solvents were evaporated and the residue was purified by flash chromatography (cyclohexane/EtOAc, gradient from 90:10 to 50:50). The product was obtained as a white solid (60 mg, 84 % yield).
¹H NMR (500 MHz, Chloroform-*d*) *δ* (ppm) 8.72 (d, *J =* 4.5 Hz, 2H), 8.26 (d, *J =* 5.7 Hz, 2H), 8.19 (d, *J =* 8.5 Hz, 1H), 8.13 (s, 1H), 8.11 (s, 1H), 7.99 (s, 1H), 7.53 (d, *J =* 8.5 Hz, 1H), 7.41 - 7.34 (m, 3H), 7.33 - 7.29 (m, 2H), 5.40 (s, 2H).
¹³C NMR (126 MHz, Chloroform-*d*) *δ* (ppm) 152.58, 150.12, 147.47, 144.05, 138.17, 136.04, 132.64, 131.67, 131.40, 130.90, 128.94, 128.36, 128.32, 127.87, 123.94, 123.08, 116.23, 56.46.
HRMS (APCI): calcd. for C₂₂H₁₇N₄S [M+H]⁺ = 369.1168, found [M+H]⁺ = 369.1166.
FTIR (neat), cm⁻¹: 1595, 1573, 1531, 1454, 1397, 1228, 1172, 991, 873, 791, 727, 694, 641, 627, 558.

### Preparative Example 82: 5-(5-(cyclopropylmethyl)-1-methyl-1H-pyrazol-4-yl)-3-(pyridin-4-yl)thieno[3,2-b]pyridine

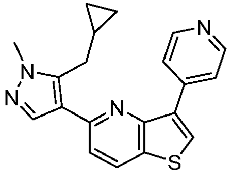

The compound was prepared according to General procedure B using 5-chloro-3-(pyridin-4-yl)thieno[3,2-b]pyridine **(Prep. Example 12)** (45 mg, 0.183 mmol) and 5-(cyclopropylmethyl)-1-methyl-4-(4,4,5,5-tetramethyl-1,3,2-dioxaborolan-2-yl)-1*H*-pyrazole (58 mg, 0.234 mmol). Reaction temperature: 85 °C. After 16 hours, additional tripotassium phosphate (116 mg, 0.549 mmol), 1,1'-Bis(di- *tert-*phosphino)ferrocene]dichloropalladium(II) (6 mg, 0.009 mmol) and H₂O (0.5 mL) were added and the mixture was refluxed for additional 2.5 hours. The solvents were evaporated and the residue was purified by flash chromatography (cyclohexane/EtOAc, gradient from 80:20 to 20:80). The product was obtained as a yellow wax (23 mg, 37 % yield).
¹H NMR (500 MHz, Chloroform-*d*) *δ* (ppm) 8.73 (s, 2H), 8.20 (d, *J =* 8.5 Hz, 1H), 8.13 - 8.07 (m, 2H), 8.04 (s, 1H), 7.91 (s, 1H), 7.61 (d, *J=* 8.5 Hz, 1H), 3.92 (s, 3H), 3.34 - 3.19 (m, 2H), 1.04 (s, 1H), 0.38 (d, *J=* 7.6 Hz, 2H), 0.09 *(d, J=* 5.1 Hz, 2H).
¹³C NMR (126 MHz, Chloroform-*d*) *δ* (ppm) 152.40, 151.92, 148.07, 143.86, 141.37, 138.09, 133.77, 131.18, 131.06, 130.18, 123.45, 119.21, 117.39, 36.93, 28.29, 10.24, 4.27.
HRMS (APCI): calcd.for C₂₀H₁₉N₄S [M+H]⁺ = 347.1325, found [M+H]⁺ = 347.1327.
FTIR (neat), cm⁻¹: 1571, 1264, 827, 730, 702.

### Preparative Example 83: tert-butyl(1-methyl-4-(3-(pyridin-4-yl)thieno[3,2-b]pyridin-5-yl)-1H-pyrazol-3-yl)carbamate

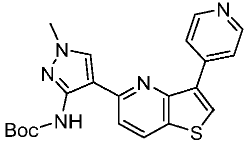

The compound was prepared according to General procedure B using 5-chloro-3-(pyridin-4-yl)thieno[3,2-*b*]pyridine **(Prep. Example 12)** (100 mg, 0.406 mmol) and *tert*-butyl (1-methyl-4-(4,4,5,5-tetramethyl-1,3,2-dioxaborolan-2-yl)-1*H*-pyrazol-3-yl)carbamate (157 mg, 0.487 mmol). After 16 hours, additional tripotassium phosphate (125 mg (0.560 mmol) and [1,1'-Bis(di-*tert-*butylphosphino)ferrocene]dichloropalladium(II) (13 mg, 0.020 mmol) were added and the mixture was refluxed for additional 4 hours. Then, bis(tri-tert-butylphosphine)palladium(0) (10 mg, 0.020 mmol) was added and the mixture was refluxed for additional 2 hours. The solvents were evaporated and the residue was purified by flash chromatography (EtOAc/methanol, gradient from 100:0 to 95:5). The product was obtained as a pale yellow wax (59 mg, 36 % yield).
¹HNMR (500 MHz, Chloroform-*d*) *δ* (ppm) 9.66 (s, 1H), 8.75 (d, *J* = 4.7 Hz, 2H), 8.18 *(d, J=* 8.6 Hz, 1H), 7.98 (s, 1H), 7.92 (d, *J=* 5.0 Hz, 2H), 7.72 (s, 1H), 7.43 (d, *J* = 8.6 Hz, 1H), 3.94 (s, 3H), 1.38 (s, 9H).
¹³C NMR (126 MHz, Chloroform-*d*) *δ* (ppm) 151.72, 151.45, 150.94, 149.77, 146.62, 142.47, 134.16, 131.67, 130.97, 130.14, 128.72, 122.74, 116.40, 108.70, 80.56, 39.59, 28.28.
HRMS (APCI): calcd.for C₂₁H₂₂N₅O₂S [M+H]⁺ = 408.1489, found [M+H]⁺ = 408.1487.
FTIR (neat), cm⁻¹: 3289, 2925, 1726, 1580, 1556, 1534, 1388, 1365, 1222, 1153, 1089, 1052, 1052, 1016, 814, 641, 557.

### Preparative Example 84: 1-methyl-4-(3-(pyridin-4-yl)thieno[3,2-b]pyridin-5-yl)-1H-pyrazol-3-amine

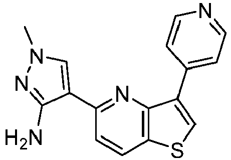

To a solution of *tert*-butyl(1-methyl-4-(3-(pyridin-4-yl)thieno[3,2-*b*]pyridin-5-yl)-1*H*-pyrazol-3-yl)carbamate (25 mg, 0.061 mmol) in dichloromethane (0.2 mL) was added trifluoroacetic acid (50 µL) and the reaction mixture was stirred at 25 °C for 2 hours. Then, additional trifluoroacetic acid (0.1 mL) was added and the reaction mixture was stirred at 25 °C for 16 hours. The solvents were evaporated and the residue was purified by flash chromatography (EtOAc/methanol/7 M NH₃ in methanol, gradient from 100:0:0 to 79:19:2). The product was obtained as a yellow wax (14 mg, 72 %).
¹HNMR(500 MHz, Methanol-*d*₄) *δ* (ppm) 8.65 (dd, *J* = 4.7, 1.4 Hz, 2H), 8.32 (s, 1H), 8.28 (d, *J=* 8.6 Hz, 1H), 8.06 (dd, *J* = 4.7, 1.5 Hz, 2H), 8.01 (s, 1H), 7.60 (d, *J* = 8.6 Hz, 1H), 3.76 (s, 3H).
¹³CNMR(126 MHz, Methanol-*d*₄) *δ* (ppm) 155.69, 153.35, 152.73, 150.10, 145.31, 134.53, 132.68, 132.14, 131.82, 131.59, 124.62, 117.07, 107.67, 38.66.
HRMS (APCI): calcd. for C₁₆H₁₄N₅S [M+H]⁺ = 308.0964, found [M+H]⁺ = 308.0962.
FTIR (neat), cm⁻¹: 3656 - 2258, 1673, 1556, 1388, 1173, 1122, 826, 799, 643.

### Preparative Example 85: 3-(pyridin-4-yl)-5-(1,3,5-trimethyl-1H-pyrazol-4-yl)thieno[3,2-b]pyridine

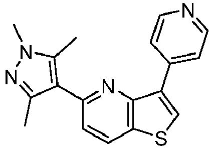

To a solution of 5-chloro-3-(pyridin-4-yl)thieno[3,2-*b*]pyridine **(Prep. Example 12)** (155 mg, 0.629 mmol) in dioxane (3 mL) were added (1,3,5-trimethyl-1*H*-pyrazol-4-yl)boronic acid (92 mg, 0.755 mmol), potassium carbonate (261 mg, 1.88 mmol), SPhosPdG3 (0.063 mmol, 49 mg), Sphos (13 mg, 0.032 mmol) and H₂O (1 mL) and) and the reaction mixture was refluxed for 16 hours. Then, additional SPhosPdG3 (0.063 mmol, 49 mg) and potassium carbonate (125 mg, 0.904 mmol) were added and the reaction mixture was refluxed for additional 24 hours. The solvents were evaporated and the residue was purified twice by (dichloromethane/methanol, gradient from 100:0 to 95:5; then hexane/EtOAc, gradient from 70:30 to 50:50). The product was obtained as a white solid (69 mg, 34 %).
¹H NMR (500 MHz, Chloroform-*d*) *δ* (ppm) 8.69 (dd, *J* = 4.6, 1.6 Hz, 2H), 8.24 (d, *J* = 8.5 Hz, 1H), 8.08 - 8.07 (dd, *J=* 4.6, 1.5 Hz, 2H), 8.06 (s, 1H), 7.40 (d, *J=* 8.5 Hz, 1H), 3.81 (s, 3H), 2.49 (s, 3H), 2.45 (s, 3H).
¹³C NMR (126 MHz, Chloroform-*d*) *δ* (ppm) 152.84, 151.99, 149.59, 145.86, 142.15, 138.29, 133.96, 131.12, 130.90, 129.47, 122.80, 119.35, 118.45, 35.99, 13.58, 11.07.
HRMS (APCI): calcd. for C₁₈H₁₇N₄S [M+H]⁺ = 321.1168, found [M+H]⁺ = 321.1167.
FTIR (neat), cm⁻¹: 3045, 3196, 3046, 2925, 2853, 1662, 1598, 1570, 1433, 1385, 1249, 1083, 993, 858, 813, 640, 587, 573, 553, 480, 453.

### Preparative Example 86: 5-(1-methyl-1H-pyrazol-3-yl)-3-(pyridin-4-yl)thieno[3,2-b]pyridine

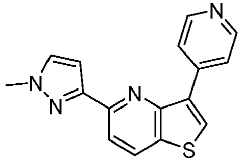

The compound was prepared according to General procedure B using 5-chloro-3-(pyridin-4-yl)thieno[3,2-b]pyridine **(Prep. Example 12)** (50 mg, 0.203 mmol) and 1-methyl-3-(4,4,5,5-tetramethyl-1,3,2-dioxaborolan-2-yl)-1*H*-pyrazole (51 mg, 0.243 mmol). Reaction time: 1 hour. The solvents were evaporated and the residue was purified by flash chromatography (hexane/EtOAc, gradient from 40:60 to 0:100). The product was obtained as an orange solid (53 mg, 89 % yield).
¹H NMR (500 MHz, Chloroform-*d*) *δ* (ppm) 8.73 (d, *J=* 5.0 Hz, 2H), 8.24 (d, *J=* 8.5 Hz, 1H), 8.18 (d, *J* = 5.6 Hz, 2H), 8.09 (d, *J* = 8.5 Hz, 1H), 8.07 (s, 1H), 7.45 (d, *J* = 2.2 Hz, 1H), 7.03 (d, *J* = 2.2 Hz, 1H), 4.01 (s, 3H).
¹³C NMR (126 MHz, Chloroform-*d*) *δ* (ppm) 152.50, 152.14, 150.42, 149.55, 142.21, 133.62, 132.90, 131.64, 131.21, 129.46, 122.73, 116.06, 104.97, 39.27.
HRMS (APCI): calcd. for C₁₆H₁₃N₄S [M+H]⁺ = 293.0855, found [M+H]⁺ = 293.0854.
FTIR (neat), cm⁻¹: 3098, 1594, 1571, 1558, 1511, 1383, 1266, 1236, 992, 844, 795, 776, 765, 723, 639, 591, 557, 477, 439.

### Preparative Example 87: 5-(1-ethyl-1H-pyrazol-3-yl)-3-(pyridin-4-yl)thieno[3,2-b]pyridine

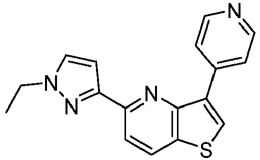

The compound was prepared according to General procedure B using 5-chloro-3-(pyridin-4-yl)thieno[3,2-b]pyridine **(Prep. Example 12)** (50 mg, 0.203 mmol) and 1-ethyl-3-(4,4,5,5-tetramethyl-1,3,2-dioxaborolan-2-yl)-1*H*-pyrazole (50 mg, 0.223 mmol). Reaction time: 2.5 hours. The solvents were evaporated and the residue was purified by flash chromatography (hexane/EtOAc, gradient from 40:60 to 20:80) and preparative TLC (cyclohexane/EtOAc, 90:10 - 2 elutions). The product was obtained as a yellow wax (37 mg, 62 % yield).
¹H NMR (500 MHz, Chloroform-*d*) *δ* (ppm) 8.74 (s, 2H), 8.23 *(d, J=* 8.5 Hz, 1H), 8.16 (s, 2H), 8.10 (d, *J* = 8.5 Hz, 1H), 8.04 (s, 1H), 7.48 (d, *J=* 1.9 Hz, 1H), 7.03 (d, *J=* 1.9 Hz, 1H), 4.27 (q, *J=* 7.3 Hz, 2H), 1.57 (t, *J* = 7.3 Hz, 3H).
¹³C NMR (126 MHz, Chloroform-*d*) *δ* (ppm) 152.50, 151.85, 150.57, 149.86, 141.89, 133.72, 132.82, 131.12, 129.82, 129.19, 122.71, 116.11, 104.68, 47.34, 15.62.
HRMS (APCI): calcd.for C₁₇H₁₅N₄S [M+H]⁺ = 307.1012, found [M+H]⁺ = 307.1011.
FTIR (neat), cm⁻¹: 2979, 2933, 1596, 1570, 1557, 1432, 1384, 1355, 1324, 1263, 1266, 1039, 994, 828, 775, 761, 733, 641, 592.

### Preparative Example 88: 5-(1-phenyl-1H-pyrazol-3-yl-3-(pyridin-4-yl)thieno[3,2-b]pyridine

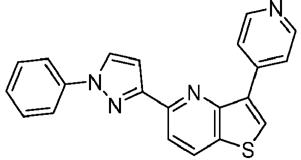

The compound was prepared according to General procedure B using 5-chloro-3-(pyridin-4-yl)thieno[3,2-b]pyridine **(Prep. Example 12)** (50 mg, 0.203 mmol) and 1-phenyl-3-(4,4,5,5-tetramethyl-1,3,2-dioxaborolan-2-yl)-1*H*-pyrazole (60 mg, 0.223 mmol). Reaction time: 2.5 hours. The solvents were evaporated and the residue was purified by flash chromatography (hexane/EtOAc, gradient from 70:30 to 50:50). The product was obtained as a white solid (57 mg, 79 % yield).
¹H NMR (500 MHz, Chloroform-*d*) *δ* (ppm) 8.68 (dd, *J* = 4.6, 1.5 Hz, 2H), 8.29 (s, 2H), 8.12 (dd, *J* = 4.5, 1.5 Hz, 2H), 8.07 (s, 1H), 8.03 (d, *J* = 2.4 Hz, 1H), 7.82 (dd, *J* = 8.8, 1.1 Hz, 2H), 7.52 - 7.48 (m, 2H), 7.34 - 7.31 (m, 1H), 7.27 (d, *J* = 2.5 Hz, 1H).
¹³C NMR (126 MHz, Chloroform-*d*) *δ* (ppm) 153.59, 152.55, 150.04, 141.77, 140.19, 133.84, 133.32, 131.24, 129.49, 129.32, 128.27, 126.66, 122.67, 119.22, 116.35, 106.90.
HRMS (APCI): calcd. for C₂₁H₁₅N₄S [M+H]⁺ = 355.1012, found [M+H]⁺ = 355.1009.
FTIR (neat), cm⁻¹: 3039, 1598, 1570, 1518, 1382, 1273, 830, 756, 690, 643.

### Preparative Example 89: 5-(1-benzyl-1H-pyrazol-3-yl)-3-(pyridin-4-yl)thieno[3,2-b]pyridine

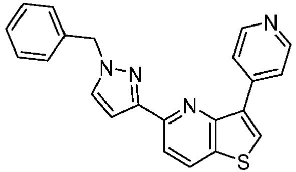

To a solution of 1-benzyl-3-bromo-1*H*-pyrazole (100 mg, 0.421 mmol) in dioxane (3.5 mL) were added bis(pinacolato)diboron (133 mg, 0.526 mmol), potassium acetate (165 mg, 1.68 mmol), tris(dibenzylideneacetone)dipalladium(0) (19 mg, 0.021 mmol) and Xphos (32 mg, 0.067 mmol) and the reaction mixture was refluxed for 5 hours. Then, 5-chloro-3-(pyridin-4-yl)thieno[3,2-*b*]pyridine (129 mg, 0.526 mmol) **(Prep. Example 12),** tripotassium phosphate (268 mg, 1.26 mmol), dioxane (3 mL) and H₂O (0.9 mL) were added and the reaction mixture was refluxed for 16 hours. The solvents were evaporated and the residue was purified by flash chromatography (cyclohexane/EtOAc/methanol, gradient from 100:0:0 to 0:90:10). The product was obtained as a white solid (87 mg, 56 %).
¹HNMR (500 MHz, Chloroform-*d*) *δ* (ppm) 8.68 (d, *J* = 6.1 Hz, 2H), 8.19 (d, *J=* 8.5 Hz, 1H), 8.13 - 8.11 (m, 2H), 8.09 (d, *J* = 8.5 Hz, 1H), 8.01 (s, 1H), 7.41 (d, *J* = 2.3 Hz, 1H), 7.36 - 7.25 (m, 3H), 7.24 (d, *J* = 1.4 Hz, 1H), 7.22 (d, *J =* 2.5 Hz, 1H), 7.03 (d, *J* = 2.3 Hz, 1H), 5.37 (s, 2H).
¹³C NMR (126 MHz, Chloroform-*d*) *δ* (ppm) 152.43, 152.18, 150.39, 149.57, 142.09, 136.38, 133.54, 132.91, 131.13, 130.96, 129.40, 128.81, 128.08, 127.61, 122.66, 116.24, 105.32, 56.31.
HRMS (APCI): calcd. for C₂₂H₁₇N₄S [M+H]⁺ = 369.1168, found [M+H]⁺ = 369.1167.
FTIR (neat), cm⁻¹: 1596, 1572, 1382, 1231, 1053, 1043, 838, 789, 774, 717, 694, 641.

### Preparative Example 90: 5-(1-methyl-1H-pyrazol-5-yl)-3-(pyridin-4-yl)thieno[3,2-b]pyridine

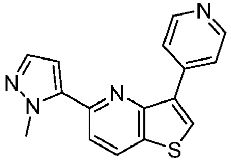

The compound was prepared according to General procedure B using 5-chloro-3-(pyridin-4-yl)thieno[3,2-b]pyridine **(Prep. Example 12)** (45 mg, 0.183 mmol) and 1-methyl-5-(4,4,5,5-tetramethyl-1,3,2-dioxaborolan-2-yl)-1*H*-pyrazole (46 mg, 0.220 mmol). Reaction temperature: 85 °C. After 16 hours, additional tripotassium phosphate (116 mg, 0.549 mmol), 1,1'-Bis(di-tert-phosphino)ferrocene]dichloropalladium(II) (6 mg, 0.009 mmol) and H₂O (0.5 mL) were added and the mixture was refluxed for additional 2.5 hours. The solvents were evaporated and the residue was purified by flash chromatography (hexane/EtOAc, gradient from 70:30 to 20:80) and preparative TLC (hexane/EtOAc, 20:80 - 1 elution and 50:50 - 1 elution). The product was obtained as a white solid (11 mg, 21 % yield).
¹H NMR (500 MHz, Chloroform-*d*) *δ* (ppm) 8.75 (d, *J* = 4.9 Hz, 2H), 8.33 (d, *J* = 8.5 Hz, 1H), 8.23 (s, 1H), 8.22 (s, 2H), 7.70 (d, *J* = 8.5 Hz, 1H), 7.70 (d, *J* = 8.5 Hz, 1H), 6.71 (d, *J* = 1.6 Hz, 1H), 4.26 (s, 3H).
¹³C NMR (126 MHz, Chloroform-*d*) *δ* (ppm) 152.02, 148.06, 146.92, 144.57, 140.89, 138.29, 133.12, 132.99, 131.93, 131.85, 123.43, 119.13, 107.38, 39.89.
HRMS (APCI): calcd. for C₁₆H₁₃N₄S [M+H]⁺ = 293.0855, found [M+H]⁺ = 293.0858.
FTIR (neat), cm⁻¹: 3398, 3097, 2924, 1598, 1571, 1556, 1390, 999, 928, 828, 782, 641.

### Preparative Example 91: 5-(1-methyl-1H-imidazol-5-yl)-3-(pyridin-4-yl)thieno[3,2-b]pyridine

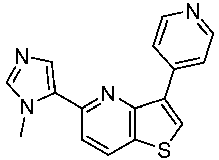

The compound was prepared according to General procedure B using 5-chloro-3-(pyridin-4-yl)thieno[3,2-b]pyridine **(Prep. Example 12)** (60 mg, 0.244 mmol) and 1-methyl-5-(4,4,5,5-tetramethyl-1,3,2-dioxaborolan-2-yl)-1*H*-imidazole (61 mg, 0.293 mmol). After 6 hours, additional [1,1'-Bis(diphenylphosphino)ferrocene]dichloropalladium(II) (14 mg, 0.019 mmol) and triethylamine (102 µL, 0.732 mmol) instead of potassium phosphate were added and the mixture was refluxed for additional 16 hours. The solvents were evaporated and the residue was purified by flash chromatography (hexane/EtOAc/methanol, gradient from 30:70:0 to 0:95:5) and preparative TLC (dichloromethane/methanol, 90:10). The product was obtained as a brown solid (18 mg, 13 % yield).
¹H NMR (500 MHz, Chloroform-*d*) *δ* (ppm) 8.71 (d, *J* = 6.0 Hz, 2H), 8.23 (d, *J* = 8.6 Hz, 1H), 8.01 (s, 1H), 7.91 (d, *J=* 6.0 Hz, 2H), 7.66 (d, *J* = 8.6 Hz, 1H), 7.58 (s, 1H), 7.55 (s, 1H), 4.04 (s, 3H).
¹³C NMR (126 MHz, Chloroform-*d*) *δ* (ppm) 152.17, 149.92, 148.22, 141.98, 141.35, 134.48, 131.96, 131.63, 131.51, 131.19, 129.67, 122.98, 117.61, 35.07.
HRMS (APCI): calcd. for C₁₆H₁₃N₄S [M+H]⁺ = 293.0855, found [M+H]⁺ = 293.0853.
FTIR (neat), cm⁻¹: 3380, 3096, 1716, 1598, 1572, 1393, 1231, 1177, 1129, 1075, 827, 658, 641.

### Preparative Example 92: 5-(isothiazol-5-yl)-3-(pyridin-4-yl)thieno[3,2-b]pyridine

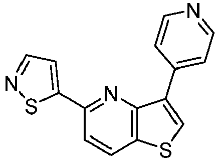

The compound was prepared according to General procedure B using 5-chloro-3-(pyridin-4-yl)thieno[3,2-b]pyridine **(Prep. Example 12)** (45 mg, 0.182 mmol), 5-(4,4,5,5-tetramethyl-1,3,2-dioxaborolan-2-yl)isothiazole (46 mg, 0.218 mmol) and XPhosPdG3 (15 mg, 0.018 mmol) instead of [1,1'-Bis(diphenylphosphino)ferrocene]dichloropalladium(II). Reaction time: 16 hours. The solvents were evaporated and the residue was purified by flash chromatography (hexane/EtOAc, gradient from 70:30 to 30:70). The product was obtained as a pale yellow solid (12 mg, 22 % yield).
¹H NMR (500 MHz, Methanol-*d₄*) *δ* (ppm) 8.66 (d, *J* = 5.3 Hz, 2H), 8.60 (s, 1H), 8.56 (d, *J* = 1.8 Hz, 1H), 8.52 (d, *J* = 8.4 Hz, 1H), 8.28 (dd, *J* = 4.6, 1.7 Hz, 2H), 7.95 (d, *J* = 8.5 Hz, 1H), 7.92 (d, *J* = 1.9 Hz, 1H).
¹³CNMR (126 MHz, Methanol-*d₄*) *δ* (ppm) 169.64, 160.02, 153.91, 150.25, 148.77, 143.77, 136.30, 134.11, 133.88, 133.54, 124.06, 121.98, 116.86.
HRMS (APCI): calcd. for C₁₅H₁₀N₃S₂ [M+H]⁺ = 296.0311, found [M+H]⁺ = 296.0311.
FTIR (neat), cm⁻¹: 3045, 2957, 2853, 1599, 1557, 1387, 812, 784, 637.

### Preparative Example 93: 5-(1-methyl-1H-1,2,3-triazol-4-yl)-3-(pyridin-4-yl)thieno[3,2-b]pyridine

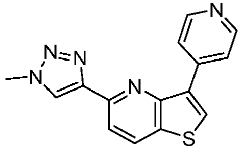

To a solution of 5-chloro-3-(pyridin-4-yl)thieno[3,2-*b*]pyridine **(Prep. Example 12)** (57 mg, 0.232 mmol) in dioxane (4.5 mL) were added 1-methyl-4-(4,4,5,5-tetramethyl-1,3,2-dioxaborolan-2-yl)-1*H*-1,2,3-triazole (58 mg, 0.278 mmol), potassium carbonate (96 mg, 0.696 mmol), SPhosPdG3 (0.028 mmol, 22 mg), Sphos (7 mg, 0.009 mmol) and H₂O (1.1 mL) and the reaction mixture was refluxed for 4.5 hours. Then, additional tripotassium phosphate (116 g, 0.546 mmol) was added and the reaction mixture was refluxed for additional 5 hours. The solvents were evaporated and the residue was purified by flash chromatography (hexane: EtOAc, gradient from 50:50 to 0:100) and preparative TLC (hexane/ EtOAc, 20:80). The product was obtained as a pale yellow solid (14 mg, 20 %).
¹H NMR (500 MHz, Chloroform-*d*) *δ* (ppm) 8.75 (d, *J* = 4.9 Hz, 2H), 8.32 (d, *J* = 8.5 Hz, 1H), 8.29 (d, *J*= 8.5 Hz, 1H), 8.19 (s, 1H), 8.08 (s, 1H), 8.06 *(d, J=* 6.1 Hz, 2H), 4.22 (s, 3H).
¹³C NMR (126 MHz, Chloroform-*d*) *δ* (ppm) 152.49, 149.66, 148.94, 148.42, 142.11, 133.59, 133.46, 131.66, 130.03, 123.23, 122.79, 116.39, 100.00, 36.91.
HRMS (APCI): calcd. for C₁₅H₁₂N₅S [M+H]⁺ = 294.0808, found [M+H]⁺ = 294.0806.
FTIR (neat), cm⁻¹: 3401, 3080, 1627, 1597, 1519, 1389, 1220, 1199, 1056, 832, 803.

### Preparative Example 94: 5-(2-methyl-2H-1,2,3-triazol-4-yl)-3-(pyridin-4-yl)thieno[3,2-b]pyridine

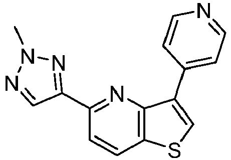

The compound was prepared according to General procedure B using 5-chloro-3-(pyridin-4-yl)thieno[3,2-b]pyridine **(Prep. Example 12)** (55 mg, 0.223 mmol) and 2-methyl-4-(4,4,5,5-tetramethyl-1,3,2-dioxaborolan-2-yl)-2*H*-1,2,3-triazole (56 mg, 0.267 mmol). Reaction time: 2 hours. The solvents were evaporated and the residue was purified by flash chromatography (hexane/EtOAc, gradient from 60:40 to 0:100). The product was obtained as a pale yellow solid (59 mg, 91 % yield).
¹H NMR (500 MHz, Chloroform-*d*) *δ* (ppm) 8.75 (s, 2H), 8.29 (d, *J* = 8.5 Hz, 1H), 8.23 (s, 1H), 8.11 (d, *J* = 5.9 Hz, 2H), 8.09 (s, 1H), 8.01 (d, *J* = 8.5 Hz, 1H), 4.29 (s, 3H).
¹³C NMR (126 MHz, Chloroform-*d*) *δ* (ppm) 152.60, 149.87, 148.34, 147.88, 141.73, 133.74, 133.58, 133.17, 131.60, 129.93, 122.64, 116.46, 41.93.
HRMS (APCI): calcd. for C₁₅H₁₂N₅S [M+H]⁺ = 294.0808, found [M+H]⁺ = 294.0807.
FTIR (neat), cm⁻¹: 3076, 1602, 1573, 1388, 1370, 1012, 991, 827, 800, 712, 642, 593, 555.

### Preparative Example 95: 5-(1-methyl-1H-1,2,3-triazol-4-yl)-3-(pyridin-4-yl)thieno[3,2-b]pyridine

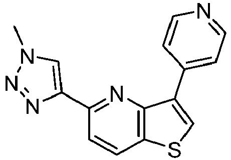

The compound was prepared according to General procedure B using 5-chloro-3-(pyridin-4-yl)thieno[3,2-b]pyridine **(Prep. Example 12)** (50 mg, 0.203 mmol) and 1-methyl-4-(4,4,5,5-tetramethyl-1,3,2-dioxaborolan-2-yl)-1*H*-1,2,3-triazole (51 mg, 0.244 mmol). Reaction time: 2 hours. The solvents were evaporated and the residue was purified by flash chromatography (cyclohexane/EtOAc, gradient from 80:20 to 0:100). The product was obtained as a pale yellow solid (43 mg, 73 % yield).
¹H NMR (500 MHz, Chloroform-*d*) *δ* (ppm) 8.76 (s, 2H), 8.32 (d, *J* = 8.5 Hz, 1H), 8.28 (d, *J* = 8.5 Hz, 1H), 8.19 (s, 1H), 8.08 (m, 3H), 4.21 (s, 3H).
¹³C NMR (126 MHz, Chloroform-*d*) *δ* (ppm) 152.47, 149.53, 148.92, 148.42, 142.18, 133.52, 133.45, 131.65, 130.09, 123.24, 122.85, 116.38, 36.90.
HRMS (APCI): calcd. for C₁₅H₁₂N₅S [M+H]⁺ = 294.0808, found [M+H]⁺ = 294.0811.
FTIR (neat), cm⁻¹: 1593, 1578, 1388, 1234, 1198, 1068, 870, 855, 832, 791, 641, 591, 482.

### Preparative Example 96: 5-(1-methyl-1H-1,2,4-triazol-5-yl)-3-(pyridin-4-yl)thieno[3,2-b]pyridine

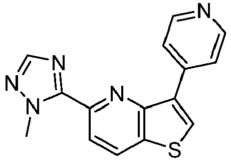

To a solution of 5-chloro-3-(pyridin-4-yl)thieno[3,2-*b*]pyridine (60 mg, 0.243 mmol) in dioxane (3 mL) were added 1-methyl-5-(tributylstannyl)-1*H*-1,2,4-triazole (102 µL, 0.316 mmol) and bis *(tri-tert-*butylphosphine)palladium (0) (10 mg, 0.019 mmol) and the reaction mixture was refluxed for 16 hours. The solvents were evaporated and the residue was purified by flash chromatography (EtOAc/cyclohexane/methanol, gradient from 70:30:0 to 95:0:5). The product was obtained as a white solid (31 mg, 44%).
¹H NMR (500 MHz, Methanol-*d₄*) *δ* (ppm) 8.66 (dd, *J* = 4.6, 1.6 Hz, 2H), 8.62 (d, *J* = 8.5 Hz, 1H), 8.56 (s, 1H), 8.22 (d, *J=* 8.5 Hz, 1H), 8.14 (dd, *J* = 4.7, 1.5 Hz, 2H), 8.03 (s, 1H), 4.38 (s, 3H).
¹³CNMR (126 MHz, Methanol-*d₄*) *δ* (ppm) 153.36, 153.30, 150.95, 150.38, 146.44, 144.09, 136.76, 134.76, 134.08, 133.89, 124.59, 120.26, 39.68.
HRMS (APCI): calcd. for C₁₅H₁₂N₅S[M+H]⁺ = 294.0808, found [M+H]⁺ = 294.0810.
FTIR (neat), cm⁻¹: 1601, 1470, 1386, 1360, 1272, 1262, 1169, 1080, 1061, 873, 774, 741, 698, 639, 512, 488.

### Preparative Example 97: N-(1-methyl-1H-pyrazol-4-yl)-3-(pyridin-4-yl)thieno[3,2-b]pyridin-5-amine

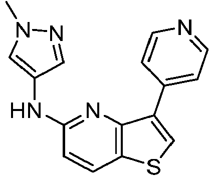

The compound was prepared according to General procedure C using 5-chloro-3-(pyridin-4-yl)thieno[3,2-*b*]pyridine **(Prep. Example 12)** (50 mg, 0.203 mmol) and 1-methyl-1*H*-pyrazol-4-amine (24 mg, 0.244 mmol). Reaction time: 16 hours. The solvents were evaporated and the residue was purified by flash chromatography (cyclohexane/EtOAc/methanol, gradient from 80:20:0 to 0:98:2). The product was obtained as an orange solid (29 mg, 47 % yield).
¹H NMR (500 MHz, Chloroform-*d*) *δ* (ppm) 8.71 (dd, *J* = 4.5, 1.7 Hz, 2H), 7.97 (dd, *J* = 4.5, 1.6 Hz, 2H), 7.94 (d, *J* = 8.7 Hz, 1H), 7.89 (s, 2H), 7.46 (s, 1H), 6.68 (d, *J* = 8.9 Hz, 1H), 6.29 (s, 1H), 3.89 (s, 3H).
¹³C NMR (126 MHz, Chloroform-*d*) *δ* (ppm) 154.52, 151.62, 149.85, 142.55, 133.28, 132.08, 132.00, 128.66, 124.86, 123.26, 122.96, 122.90, 107.23, 39.26.
HRMS (APCI): calcd. for C₁₆H₁₄N₅S [M+H]⁺ = 308.0964, found [M+H]⁺ = 308.0962.
FTIR (neat), cm⁻¹: 1603, 1577, 1452, 1406, 1389, 1349, 999, 757, 638, 563, 451.

### Preparative Example 98: N-(1H-pyrazol-4-yl)-3-(pyridin-4-yl)thieno[3,2-b]pyridin-5-amine

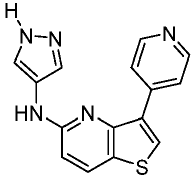

To a solution of 5-chloro-3-(pyridin-4-yl)thieno[3,2-*b*]pyridine (70 mg, 0.284 mmol) in tert-butanol (2.4 mL) were added potassium carbonate (78 mg, 0.568 mmol), tert-butyl 4-amino-1*H*-pyrazole-1-carboxylate (62 mg, 0.342 mmol) and BrettPhosPdG3 (25 mg, 0.028 mmol) and the reaction mixture was stirred 85 °C for 16 hours. The solvents were evaporated and the residue was purified by flash chromatography (cyclohexane/EtOAc/methanol, gradient from 80:20:0 to 0:95:5) and preparative TLC (EtOAc/methanol, 95:5). The product was obtained as a white solid (7 mg, 11 %).
¹H NMR (500 MHz, Methanol-*d₄*) *δ* (ppm) 8.63 (d, *J* = 4.1 Hz, 2H), 8.20 (s, 1H), 8.18 (d, *J* = 6.3 Hz, 2H), 8.03 (d, *J* = 8.9 Hz, 1H), 7.69 (s, 2H), 6.80 (d, *J* = 8.9 Hz, 1H).
¹³CNMR (126 MHz, Methanol-*d₄*) *δ* (ppm) 155.86, 152.63, 150.09, 145.40, 133.80, 132.75, 130.49, 125.51, 124.44, 109.87.
HRMS (APCI): calcd. for C₁₅H₁₂N₅S [M+H]⁺ = 294.0808, found [M+H]⁺ = 294.0809.
FTIR (neat), cm⁻¹: 2920, 2852, 2027, 1980, 1606, 1576, 1454, 1386, 1285, 1009, 789, 777, 693.

### Preparative Example 99: N-(1-methyl-1H-pyrazol-3-yl)-3-(pyridin-4-yl)thieno[3,2-b]pyridin-5-amine

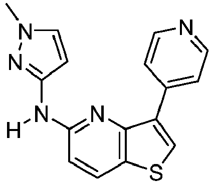

The compound was prepared according to General procedure B using 5-chloro-3-(pyridin-4-yl)thieno[3,2-*b*]pyridine **(Prep. Example 12)** (50 mg, 0.203 mmol) and 1-methyl-1*H*-pyrazol-3-amine (21 µL, 0.244 mmol). Reaction time: 16 hours. The solvents were evaporated and the residue was purified by flash chromatography (cyclohexane/EtOAc/methanol, gradient from 50:50:0 to 0:98:2). The product was obtained as a pale yellow solid (38 mg, 61 % yield).
¹H NMR (500 MHz, Chloroform-*d*) *δ* (ppm) 8.70 (d, *J* = 6.3 Hz, 2H), 8.04 (d, *J* = 4.7 Hz, 2H), 8.02 (d, *J* = 8.9 Hz, 1H), 7.94 (s, 1H), 7.28 (d, *J* = 2.3 Hz, 1H), 7.16 (s, 1H), 6.47 (d, *J* = 2.3 Hz, 1H), 3.84 (s, 3H).
¹³C NMR (126 MHz, Chloroform-*d*) *δ* (ppm) 166.29, 160.42, 153.24, 149.40, 149.16, 142.69, 132.32, 130.77, 129.08, 125.61, 122.84, 108.25, 96.41, 38.83.
HRMS (APCI): calcd. for C₁₆H₁₄N₅S [M+H]⁺ = 308.0964, found [M+H]⁺ = 308.096.
FTIR (neat), cm⁻¹: 3098, 2923, 1596, 1572, 1536, 1518, 1481, 1406, 1390, 1351, 1312, 1293, 779, 737, 697, 640.

### Preparative Example 100: N-methyl-N-(1-methyl-1H-pyrazol-3-yl)-3-(pyridin-4-yl)thieno[3,2-b]pyridin-5-amine

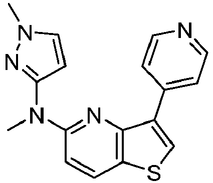

The compound was prepared according to General procedure C using 5-chloro-3-(pyridin-4-yl)thieno[3,2-*b*]pyridine **(Prep. Example 12)** (60 mg, 0.243 mmol) and *N*,1-dimethyl-1*H*-pyrazol-3-amine hydrochloride (43 mg, 0.292 mmol). Reaction time: 16 hours. The solvents were evaporated and the residue was purified by flash chromatography (cyclohexane/EtOAc, gradient from 50:50:0 to 0:100:0). The product was obtained as a pale yellow solid (19 mg, 24 % yield).
¹H NMR (500 MHz, Chloroform-*d*) *δ* (ppm) 8.68 (d, *J=* 5.4 Hz, 2H), 8.12 (dd, *J* = 4.6, 1.6 Hz, 2H) 7.95 (s, 1H), 7.89 (d, *J* = 9.0 Hz, 1H), 7.33 (d, *J* = 2.3 Hz, 1H), 7.15 (d, *J* = 9.1 Hz, 1H), 6.17 (d, *J* = 2.3 Hz, 1H), 3.89 (s, 3H), 3.60 (s, 3H).
¹³C NMR (126 MHz, Chloroform-*d*) *δ* (ppm) 156.28, 153.94, 151.28, 149.84, 142.25, 132.81, 131.23, 130.88, 128.29, 124.73, 122.33, 108.32, 99.53, 39.16, 37.44.
HRMS (APCI): calcd. for C₁₇H₁₆N₅S [M+H]⁺ = 322.1121, found [M+H]⁺ = 322.1124.
FTIR (neat), cm⁻¹: 3402, 3097, 2927, 1597, 1579, 1525, 1449, 1389, 1343, 827, 783, 756, 686, 591.

### Preparative Example 101: N-(1H-pyrazol-3-yl)-3-(pyridin-4-yl)thieno[3,2-b]pyridin-5-amine

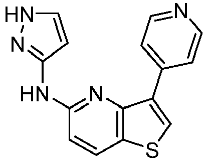

The compound was prepared according to General procedure C using 5-chloro-3-(pyridin-4-yl)thieno[3,2-*b*]pyridine **(Prep. Example 12)** (70 mg, 0.284 mmol) and *tert*-butyl 3-amino-1*H*-pyrazole-1-carboxylate (62 mg, 0.308 mmol). Reaction time: 16 hours. The solvents were evaporated and the residue was purified by flash chromatography (EtOAc/methanol, gradient from 100:0 to 95:5). The product was obtained as a white solid (17 mg, 20 % yield).
¹H NMR (500 MHz, DMSO-*d₆*) *δ* (ppm) 12.14 (s, 1H), 9.54 (s, 1H), 8.68 - 8.65 (m, 2H), 8.48 (s, 1H), 8.22 (d, *J* = 8.9 Hz, 1H), 8.18 (d, *J* = 3.5 Hz, 1H), 7.63 (s, 1H), 7.22 (d, *J* = 8.9 Hz, 1H), 6.65 (s, 1H).
¹³C NMR (126 MHz, DMSO-*d*₆) *δ* (ppm) 153.71, 150.84, 149.65, 141.67, 132.16, 131.72, 129.98, 128.36, 123.91, 122.22, 108.76, 95.22, 79.19, 79.13, 78.92, 78.66.
HRMS (APCI): calcd. for C₁₅H₁₂N₅S [M+H]⁺ = 294.0808, found [M+H]⁺ = 294.0806.
FTIR (neat), cm⁻¹: 3270 - 2928, 1602, 1580, 1484, 1390, 778, 638.

### Preparative Example 102: N-(1,5-dimethyl-1H-pyrazol-3-yl)-3-(pyridin-4-yl)thieno[3,2-b]pyridin-5-amine

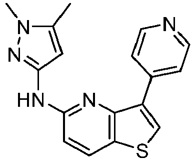

The compound was prepared according to General procedure C using 5-chloro-3-(pyridin-4-yl)thieno[3,2-*b*]pyridine **(Prep. Example 12)** (80 mg, 0.325 mmol) and 1,5-dimethyl-1*H*-pyrazol-3-amine (43 mg, 0.390 mmol). Reaction time: 16 hours. The solvents were evaporated and the residue was purified by flash chromatography (cyclohexane/EtOAc/methanol, gradient from 50:50:0 to 0:95:5). The product was obtained as a white solid (43 mg, 41 % yield).
¹H NMR (500 MHz, Methanol-*d₄*) *δ* (ppm) 8.57 (dd, *J* = 4.7, 1.6 Hz, 2H), 8.25 (s, 1H), 8.23 (dd, *J* = 4.7, 1.7 Hz, 2H), 8.06 (d, *J* = 8.9 Hz, 1H), 7.05 (d, *J* = 8.9 Hz, 1H), 6.43 (s, 1H), 3.70 (s, 3H), 2.28 (s, 3H).
¹³CNMR (126 MHz, Methanol-*d₄*) *δ* (ppm) 155.33, 152.55, 149.93, 149.65, 144.95, 140.98, 133.36, 133.09, 130.88, 126.44, 124.33, 109.64, 97.98, 35.52, 11.18.
HRMS (APCI): calcd. for C₁₇H₁₆N₅S [M+H]⁺ = 322.1121, found [M+H]⁺ = 322.1124.
FTIR (neat), cm⁻¹: 3263, 3065, 3037, 2935, 1600, 1580, 1484, 1390, 851, 780, 734, 637.

### Preparative Example 103: tert-butyl (1-methyl-3-((3-(pyridin-4-yl)thieno[3,2-b]pyridin-5-yl)-amino)-1H-pyrazol-5 -yl)carbamate

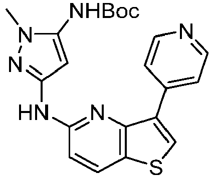

The compound was prepared according to General procedure C using 5-chloro-3-(pyridin-4-yl)thieno[3,2-b]pyridine **(Prep. Example 12)** (70 mg, 0.284 mmol) and tert-butyl (3-amino-1-methyl-1*H*-pyrazol-5-yl)carbamate (72 mg, 0.341 mmol). Reaction time: 16 hours. The solvents were evaporated and the residue was purified by flash chromatography (cyclohexane/EtOAc, gradient from 70:30 to 0:100). The product was obtained as a white solid (13 mg, 13 % yield).
¹H NMR (500 MHz, Chloroform-*d*) *δ* (ppm) 8.69 (dd, *J* = 4.7, 1.4 Hz, 2H), 7.99 (dd, *J* = 4.6, 1.6 Hz, 2H), 7.97 (d, *J* = 8.8 Hz, 1H), 7.89 (s, 1H), 7.08 (d, *J* = 9.8 Hz, 1H), 6.48 (s, 2H), 3.67 (s, 3H), 1.54 (s, 9H).
¹³C NMR (126 MHz, Chloroform-*d*) *δ* (ppm) 153.16, 151.42, 149.95, 147.61, 142.25, 135.94, 133.22, 132.14, 128.70, 125.65, 122.72, 108.11, 90.66, 81.79, 34.93, 28.23.
HRMS (APCI): calcd. for C₂₁H₂₃N₆O₂S [M+H]⁺ = 423.1598, found [M+H]⁺ = 423.1598.
FTIR (neat), cm⁻¹: 2976, 2928, 1716, 1601, 1578, 1389, 1246, 1156, 907, 779, 729, 641.

### Preparative Example 104: 1-methyl-N3-(3-(pyridin-4-yl)thieno[3,2-b]pyridin-5-yl)-1H-pyrazole-3,5-diamine

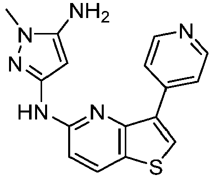

The compound was prepared according to General procedure D using *tert*-butyl (1-methyl-3-((3-(pyridin-4-yl)thieno[3,2-*b*]pyridin-5-yl)amino)-1*H*-pyrazol-5-yl)carbamate **(Prep. Example 103)** (14 mg, 0.033 mmol). After 5 hours, additional 4 N HCl in dioxane (100 µL, 0.792 mmol) was added and the mixture was refluxed for additional 5 hours. The solvents were evaporated and the residue was purified by preparative TLC (dichloromethane/methanol/7 M NH₃ in methanol, 90:5:5). The product was obtained as a pale yellow solid (8 mg, 71 % yield).
¹H NMR (500 MHz, DMSO-*d₆*) *δ* (ppm) 9.15 (s, 1H), 8.69 (dd, *J* = 4.3, 1.4 Hz, 2H), 8.51 (s, 1H), 8.25 (dd, *J* = 4.8, 1.5 Hz, 2H), 8.18 (d, *J* = 9.0 Hz, 1H), 7.30 (d, *J=* 8.9 Hz, 1H), 5.17 (d, *J* = 2.4 Hz, 2H), 3.46 (s, 3H).
¹³C NMR (126 MHz, DMSO-*d₆*) *δ* (ppm) 153.64, 150.94, 149.79, 147.24, 146.68, 146.61, 141.46, 132.01, 131.37, 129.89, 123.72, 121.88, 108.73, 79.66, 33.61.
HRMS (APCI): calcd. for C₁₆H₁₅N₆S [M+H]⁺ = 323.1073, found [M+H]⁺ = 323.1076.
FTIR (neat), cm⁻¹: 3564 - 2999, 1634, 1604, 1582, 1563, 1507, 1485, 1393, 783, 640.

### Preparative Example 105: Ethyl 1-methyl-3-((3-(pyridin-4-yl)thieno[3,2-b]pyridin-5-yl)amino)-1H-pyrazole-5-carboxylate

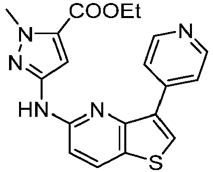

To a solution of ethyl 3-amino-1-methyl-1*H*-pyrazole-5-carboxylate hydrochloride (111 mg, 0.537 mmol) in dioxane (1 mL) was added cesium carbonate (120 mg, 0.366 mmol) and the reaction mixture was stirred at room temperature for 30 min. Then, 5-chloro-3-(pyridin-4-yl)thieno[3,2-*b*]pyridine **(Prep. Example 12)** (60 mg, 0.244 mmol), cesium carbonate (198 mg, 0.610 mmol), bis(dibenzylideneacetone)palladium (0) (7 mg, 0.0081 mmol), 4,5-Bis(diphenylphosphino)-9,9-dimethylxanthene (9 mg, 0.016 mmol) and dioxane (2 mL) were added and the mixture was refluxed stirred 16 hours. Then, triethylamine (0.5 mL) was added and the reaction was refluxed for additional 24 hours. The solvents were evaporated and the residue was purified by flash chromatography (cyclohexane/ EtOAc, gradient from 90:10 to 50:50). The product was obtained as a pale yellow solid (35 mg, 38 %).
¹H NMR (500 MHz, Chloroform-*d₃*) *δ* (ppm) 8.71 (dd, *J* = 4.5, 1.5 Hz, 2H), 8.01 (d, *J* = 6.1 Hz, 1H), 7.99 (dd, *J* = 3.1, 1.3 Hz, 2H), 7.92 (s, 1H), 7.27 (s, 1H), 7.11 (s, 1H), 6.91 (d, *J =* 8.8 Hz, 1H), 4.41 (q, *J* = 7.1 Hz, 2H), 4.12 (s, 3H), 1.42 (t*, J* = 7.1 Hz, 3H).
¹³C NMR (126 MHz, Chloroform-*d₃*) *δ* (ppm) 159.92, 152.62, 151.42, 150.00, 147.42, 142.20, 133.45, 132.72, 132.22, 128.76, 125.82, 122.77, 108.22, 100.88, 61.17, 38.90, 14.42.
HRMS (APCI): calcd. for C₁₉H₁₈N₅O₂S₂ [M+H]⁺ = 380.1176, found [M+H]⁺ = 380.1174.
FTIR (neat), cm⁻¹: 2956, 2922, 2850, 1717, 1599, 1581, 1550, 1482, 1454, 1389, 1258, 1098, 801, 763.

### Preparative Example 106: N-(1-methyl-4-(methylthio)-1H-pyrazol-3-yl)-3-(pyridin-4-yl)thieno[3,2-b]pyridin-5-amine

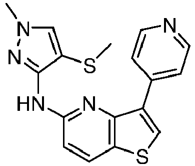

The compound was prepared according to General procedure B using 5-chloro-3-(pyridin-4-yl)thieno[3,2-*b*]pyridine **(Prep. Example 12)** (60 mg, 0.244 mmol) and 1-methyl-4-(methylthio)-1*H*-pyrazol-3-amine (42 mg, 0.295 mmol). Reaction time: 16 hours. The solvents were evaporated and the residue was purified by flash chromatography (cyclohexane/EtOAc, gradient from 90:10 to 30:70). The product was obtained as a orange solid (20 mg, 23 % yield).
¹H NMR (500 MHz, Chloroform-*d*) *δ* (ppm) 8.71 (dd, *J* = 4.5, 1.5 Hz, 2H), 8.11 (d, *J* = 1.2 Hz, 2H), 8.06 (dd, *J =* 4.5, 1.5 Hz, 2H), 7.95 (s, 1H), 7.38 (s, 1H), 7.35 (s, 1H), 3.86 (s, 3H), 2.25 (s, 3H).
¹³C NMR (126 MHz, Chloroform-*d*) *δ* (ppm) 152.72, 151.35, 151.06, 150.03, 141.96, 134.45, 132.81, 132.50, 129.03, 126.42, 122.52, 108.03, 99.17, 39.25, 21.28.
HRMS (APCI): calcd. for C₁₇H₁₆N₅S₂ [M+H]⁺ = 354.0842, found [M+H]⁺ = 354.0841.
FTIR (neat), cm⁻¹: 3397, 3048, 3028, 2915, 1601, 1578, 1542, 1392, 1159, 994, 963, 817, 806, 714, 678, 516, 494.

### Preparative Example 107: Ethyl 1-methyl-3-((3-(pyridin-4-yl)thieno[3.2-b]pyridin-5-yl)amino)-1H-pyrazole-4-carboxylate

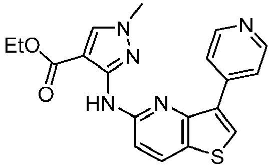

The compound was prepared according to General procedure C using 5-chloro-3-(pyridin-4-yl)thieno[3,2-b]pyridine **(Prep. Example 12)** (150 mg, 0.609 mmol) and ethyl 3-amino-1-methyl-1*H*-pyrazole-4-carboxylate (124 mg, 0.730 mmol). Reaction time: 1 hour. The solvents were evaporated and the residue was purified by flash chromatography (cyclohexane/EtOAc, gradient from 20:80 to 0:100). The product was obtained as a white solid (220 mg, 95 % yield).
¹H NMR (500 MHz, Chloroform-*d*) *δ* (ppm) 8.85 (s, 1H), 8.70 (dd, *J* = 4.6, 1.8 Hz, 2H), 8.14 (s, 2H), 8.12 (dd, *J* = 4.7, 1.6 Hz, 2H), 7.99 (s, 1H), 7.73 (s, 1H), 4.35 (q, *J = 7.1* Hz, 2H), 3.89 (s, 3H), 1.41 (t, *J = 7.1* Hz, 3H).
¹³C NMR (126 MHz, Chloroform-*d*) *δ* (ppm) 163.93, 151.99, 151.50, 151.46, 150.00, 141.83, 132.91, 132.76, 132.44, 129.00, 126.76, 122.38, 108.76, 100.09, 60.19, 39.44, 14.37.
HRMS (APCI): calcd. for C₁₉H₁₈N₅O₂S [M+H]⁺ 380.1176, found [M+H]⁺ = 380.1175.
FTIR (neat), cm⁻¹: 3411, 1705, 1577, 1557, 1396, 1250, 1106, 810, 767, 558.

### Preparative Example 108: N-((1-methyl-1H-pyrazol-3-yl)methyl)-3-(pyridin-4-yl)thieno[3,2-b]pyridin-5-amine

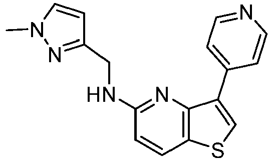

The compound was prepared according to General procedure C using 5-chloro-3-(pyridin-4-yl)thieno[3,2-*b*]pyridine **(Prep. Example 12)** (50 mg, 0.203 mmol) and (1-methyl-1*H*-pyrazol-3-yl)methanamine (27 mg, 0.243 mmol). After 7 hours, additional cesium carbonate (102 mg, 0.312 mmol), tris(dibenzylidenacetone)dipalladium (7 mg, 0.008 mmol) and 4,5-bis(diphenylphosphino)-9,9-dimethylxanthene (9 mg, 0.008 mmol) were added and the mixture was refluxed for additional 16 hours. The solvents were evaporated and the residue was purified by flash chromatography (EtOAc/methanol, gradient from 100:0 to 95:5) and preparative TLC (EtOAc/methanol, 100:0 - 1 elution and 95:5 - 1 elution). The product was obtained as a pale yellow solid (7 mg, 11 % yield).
¹H NMR (500 MHz, Chloroform-*d*) *δ* (ppm) 8.66 (d, *J* = 6.3 Hz, 2H), 8.09 (d, *J* = 6.3 Hz, 2H), 7.90 (s, 1H), 7.86 (d, *J* = 8.8 Hz, 1H), 7.28 (d, *J* = 2.2 Hz, 1H), 6.58 (d, *J* = 8.9 Hz, 1H), 6.22 (d, *J* = 2.1 Hz, 1H), 5.13 (t, *J* = 5.3 Hz, 1H), 4.66 (d, *J* = 5.2 Hz, 2H), 3.88 (s, 3H).
¹³C NMR (126 MHz, Chloroform-*d*) *δ* (ppm) 156.70, 151.63, 150.44, 149.80, 142.24, 132.57, 131.56, 130.91, 128.08, 124.00, 122.32, 107.42, 104.41, 40.11, 38.76.
HRMS (APCI): calcd. for C₁₇H₁₆N₅S [M+H]⁺ = 322.1121, found [M+H]⁺ = 322.1122.

### Preparative Example 109: 5-((1-methyl-1H-pyrazol-3-yl)oxy)-3-(pyridin-4-yl)thieno[3,2-b]pyridine

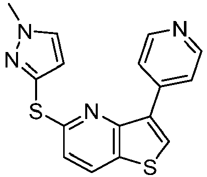

To a solution of 5-chloro-3-(pyridin-4-yl)thieno[3,2-*b*]pyridine **(Prep. Example 12)** (60 mg, 0.244 mmol) in toluene (1.5 mL) were added sodium *tert*-butoxide (26 mg, 0.268 mmol), 1-methyl-1*H*-pyrazole-3-thiol (28 mg, 0.244 mmol) and Josiphos SL-Pd-G3 (9 mg, 0.010 mmol) and the reaction mixture was stirred at room temperature for 6 hours. The solvents were evaporated and the residue was purified by flash chromatography (cyclohexane/ EtOAc, gradient from 50:50 to 0:100). The product was obtained as a pale yellow solid (17 mg, 21 %).
¹HNMR (500 MHz, Chloroform-*d*) *δ* (ppm) 8.59 (d, *J=* 5.2 Hz, 2H), 8.02 (s, 1H), 8.00 (d, *J=* 8.5 Hz, 1H), 7.91 (d, *J* = 6.3 Hz, 2H), 7.55 (d, *J* = 2.2 Hz, 1H), 7.18 (d, *J* = 8.6 Hz, 1H), 6.59 (d, J = 2.2 Hz, 1H), 4.01 (s, 3H).
¹³C NMR (126 MHz, Chloroform-*d*) *δ* (ppm) 158.33, 152.68, 149.36, 141.65, 139.54, 132.74, 131.84, 131.01, 130.91, 129.58, 122.38, 117.29, 113.43, 39.59.
HRMS (APCI): calcd. for C₁₆H₁₃N₄S₂ [M+H]⁺ = 325.0576, found [M+H]⁺ = 325.0574.
FTIR (neat), cm⁻¹: 3438, 3094, 2931, 1633, 1598, 1560, 1384, 1362, 1150, 1127, 856, 829, 794, 644, 553.

### Preparative Example 110: 5-((1-methyl-1H-pyrazol-3-yl)oxy)-3-(pyridin-4-yl)thieno[3,2-b]pyridine

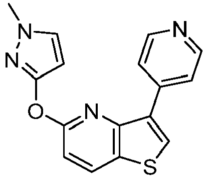

To a solution of 5-chloro-3-(pyridin-4-yl)thieno[3,2-*b*]pyridine **(Prep. Example 12)** (50 mg, 0.203 mmol) in DMF (0.8 mL) were added caesium carbonate (93 mg, 0.284 mmol), 1-methyl-1*H*-pyrazol-3-ol (20 mg, 0.203 mmol), copper (I) cyanide (1 mg, 0.010 mmol) and ethylendiamine (5 µL,0.075 mmol) and the reaction mixture was refluxed for 16 hours. The reaction mixture was quenched with water (20 mL) and extracted with EtOAc (2 × 15 mL). The combined organic extracts were washed with water (2 x 20 mL), dried over MgSO4, filtered, and the solvent was evaporated *in vacuo.* The solvents were evaporated and the residue was purified by flash chromatography chromatography (cyclohexane/EtOAc, gradient from 50:50 to 0:100) and preparative TLC (toluene/methanol, 97:3). The product was obtained as a pale yellow wax (14 mg, 22 %).
¹H NMR (500 MHz, Chloroform-*d*) *δ* (ppm) 8.60 (d, *J* = 5.1 Hz, 2H), 8.18 (d, *J* = 8.7 Hz, 1H), 8.04 (d, *J* = 0.5 Hz, 1H), 7.94 (dd, *J* = 4.6, 1.5 Hz, 2H), 7.36 (d, *J* = 2.3 Hz, 1H), 7.11 (d, *J* = 8.4 Hz, 1H), 6.20 (d, *J* = 2.3 Hz, 1H), 3.88 (s, 3H).
¹³C NMR (126 MHz, Chloroform-*d*) *δ* (ppm) 161.02, 157.54, 150.31, 149.78, 141.39, 133.85, 132.92, 130.93, 129.48, 129.23, 122.29, 108.97, 96.47, 39.24.
HRMS (APCI): calcd. for C₁₆H₁₃N₄OS [M+H]⁺ = 309.0805, found [M+H]⁺ = 309.0807.
FTIR (neat), cm⁻¹: 1630, 1600, 1579, 1561, 1385, 1337, 1263, 1219, 1168, 995, 795, 754, 626, 561, 486, 461.

### Preparative Example 111: 5-(pyridin-2-yl)-3-(pyridin-4-yl)thieno[3,2-b]pyridine

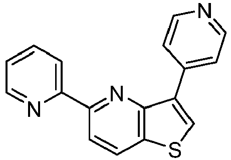

To a solution of 5-chloro-3-(pyridin-4-yl)thieno[3,2-*b*]pyridine **(Prep. Example 12)** (50 mg, 0.203 mmol) in dioxane (3 mL) were added 2-(tributylstannyl)pyridine (85 µL, 0.263 mmol) and bis(tri-tert-butylphosphine)palladium (0) (8 mg, 0.016 mmol) and the reaction mixture was refluxed for 16 hours. The solvents were evaporated and the residue was purified by flash chromatography (cyclohexane/ EtOAc, gradient from 100: 0 to 50:50). The product was obtained as a white solid (17 mg, 29 %).
¹H NMR (500 MHz, Chloroform-*d*) *δ* (ppm) 8.77 (dd, *J* = 4.9, 1.5 Hz, 2H), 8.72 (d, *J* = 5.8 Hz, 1H), 8.57 (dd, *J* = 8.2, 3.8 Hz, 2H), 8.36 (d, *J* = 8.5 Hz, 1H), 8.12 (dd, *J* = 4.6, 1.6 Hz, 2H), 8.08 (s, 1H), 7.88 (td, *J* = 7.7, 1.8 Hz, 1H), 7.35 (ddd, *J* = 7.6, 4.8, 1.3 Hz, 1H).
¹³C NMR (126 MHz, Chloroform-*d*) *δ* (ppm) 156.22, 154.23, 152.45, 150.10, 149.16, 141.79, 137.06, 134.56, 134.10, 131.63, 129.74, 123.91, 122.76, 121.26, 117.02.
HRMS (APCI): calcd. for C₁₇H₁₂N₃S [M+H]⁺ = 290.0746, found [M+H]⁺ = 290.0746.
FTIR (neat), cm⁻¹: 3057, 3075, 1632, 1598, 1542, 1470, 1435, 1389, 1176, 1144, 992, 806, 783, 768, 639, 495.

### Preparative Example 112: 3-(pyridin-4-yl)-5-(pyrimidin-2-yl)thieno[3,2-b]pyridine

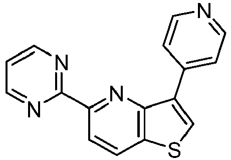

To a solution of 5-chloro-3-(pyridin-4-yl)thieno[3,2-*b*]pyridine **(Prep. Example 12)** (60 mg, 0.244 mmol) in dioxane (3.5 mL) were added 2-(tributylstannyl)pyrimidine (100 µL, 0.317 mmol) and bis(tri-tert-butylphosphine)palladium (0) (8 mg, 0.016 mmol) and the reaction mixture was refluxed for 16 hours. The solvents were evaporated and the residue was purified by flash chromatography (cyclohexane/EtOAc/methanol, gradient from 50:50:0 to 0:90:10) and preparative TLC (toluene:methanol, 95:5 - 2 elutions). The product was obtained as a white solid (30 mg, 43 %).
¹H NMR (500 MHz, Chloroform-*d*) *δ* (ppm) 8.97 (d, *J* = 4.9 Hz, 2H), 8.75 (dd, *J =* 4.6, 1.8 Hz, 2H), 8.56 (d, *J=* 8.5 Hz, 1H), 8.41 (d, *J=* 8.5 Hz, 1H), 8.20 (dd, *J* = 4.7, 1.7 Hz, 2H), 8.13 (s, 1H), 7.35 (t, *J* = 4.8 Hz, 1H).
¹³C NMR (126 MHz, Chloroform-*d*) *δ* (ppm) 164.12, 157.73, 153.17, 153.06, 150.28, 141.53, 135.64, 134.43, 131.61, 130.09, 122.56, 120.34, 119.37.
HRMS (APCI): calcd. for C₁₆H₁₁N₄S [M+H]⁺ = 291.0699, found [M+H]⁺ = 291.0702.
FTIR (neat), cm⁻¹: 1592, 1544, 1416, 1383, 1274, 827, 813, 789, 711, 634.

### Preparative Example 113: N-(pyridazin-3-yl)-3-(pyridin-4-yl)thieno[3,2-b]pyridin-5-amine

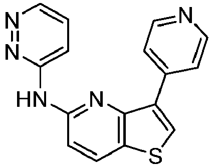

The compound was prepared according to General procedure C using 5-chloro-3-(pyridin-4-yl)thieno[3,2-*b*]pyridine **(Prep. Example 12)** (50 mg, 0.203 mmol) and pyridazin-3-amine (23 mg, 0.244 mmol). Reaction time: 16 hours. The solvents were evaporated and the residue was purified by flash chromatography (dichloromethane/methanol, gradient from 100:0 to 90:10). The product was obtained as a pale yellow solid (46 mg, 74 % yield).
¹H NMR (500 MHz, DMSO-*d₆*) *δ* (ppm) 10.42 (s, 1H), 8.82 (dd, *J* = 4.6, 1.5 Hz, 1H), 8.71 (d, *J* = 6.0 Hz, 2H), 8.58 (s, 1H), 8.44 (d, *J* = 8.9 Hz, 1H), 8.42 (dd, *J* = 9.1, 1.4 Hz, 1H), 8.10 (dd, *J* = 4.7, 1.6 Hz, 2H), 7.65 - 7.62 (m, 1H), 7.60 (d, *J* = 8.9 Hz, 1H).
¹³C NMR (126 MHz, DMSO-*d₆*) *δ* (ppm) 156.85, 152.41, 150.23, 149.80, 146.09, 141.37, 133.17, 131.95, 131.14, 127.64, 126.56, 122.30, 116.87, 110.41.
HRMS (APCI): calcd. for C₁₆H₁₂N₅S [M+H]⁺ = 306.0808, found [M+H]⁺ = 306.0807.
FTIR (neat), cm⁻¹: 3593 - 2799, 1599, 1572, 1440, 1393, 813, 700.

### Preparative Example 114: 3-(pyridin-4-yl)-N-(pyrimidin-4-yl)thieno[3,2-b]pyridin-5-amine

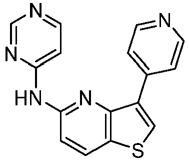

The compound was prepared according to General procedure C using 5-chloro-3-(pyridin-4-yl)thieno[3,2-*b*]pyridine **(Prep. Example 12)** (50 mg, 0.203 mmol) and pyrimidin-4-amine (23 mg, 0.244 mmol) in DMF (2 mL) instead of dioxane. Reaction time: 16 hours. The solvents were evaporated and the residue was purified by flash chromatography (dichloromethane/methanol, gradient from 100:0 to 90:10). The product was obtained as a pale yellow solid (46 mg, 74 % yield).
¹H NMR (500 MHz, DMSO-*d₆*) *δ* (ppm) 10.44 (s, 1H), 8.77 (s, 1H), 8.73 (d, *J* = 3.0 Hz, 2H), 8.62 (s, 1H), 8.50 (d, *J* = 5.5 Hz, 1H), 8.49 (d, *J* = 8.9 Hz, 1H), 8.13 (dd, *J* = 4.8, 1.6 Hz, 2H), 8.07 (d, *J* = 5.9 Hz, 1H), 7.68 (d, *J* = 8.9 Hz, 1H).
¹³C NMR (126 MHz, DMSO-*d₆*) *δ* (ppm) 158.61, 158.00, 156.59, 151.66, 150.34, 149.86, 141.29, 133.25, 132.03, 131.57, 127.42, 122.31, 110.91, 108.13.
HRMS (APCI): calcd. for C₁₆H₁₂N₅S [M+H]⁺ = 306.0808, found [M+H]⁺ = 306.0805.
FTIR (neat), cm⁻¹: 2923, 2852, 1742, 1622, 1599, 1571, 1514, 1401, 983, 827, 802, 626, 420.

### Preparative Example 115: 5-(pyrazolo[1,5-a]pyrimidin-3-yl)-3-(pyridin-4-yl)thieno[3,2-b]pyridine

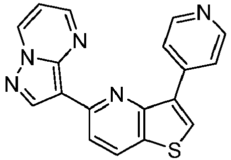

The compound was prepared according to General procedure B using 5-chloro-3-(pyridin-4-yl)thieno[3,2-*b*]pyridine **(Prep. Example 12)** (55 mg, 0.223 mmol) and 3-(4,4,5,5-tetramethyl-1,3,2-dioxaborolan-2-yl)pyrazolo[1,5-*a*]pyrimidine (65 mg, 0.268 mmol). Reaction time: 1.5 hours. The solvents were evaporated and the residue was purified by flash chromatography (EtOAc/methanol, gradient from 100:0 to 90:10). The product was obtained as a pale yellow solid (67 mg, 92 % yield).
¹H NMR (500 MHz, DMSO-*d₆*) *δ* (ppm) 9.28 (dd, *J* = 7.0, 1.7 Hz, 1H), 9.04 (s, 1H), 8.91 (s, 1H), 8.84 - 8.77 (m, 3H), 8.66 (d, *J* = 8.5 Hz, 1H), 8.63 - 8.58 (m, 3H), 7.22 (dd, *J* = 7.0, 4.0 Hz, 1H).
¹³C NMR (126 MHz, DMSO-*d₆*) *δ* (ppm) 152.14, 151.47, 149.11, 148.01, 145.09, 144.24, 143.01, 137.01, 133.46, 132.29, 131.11, 130.96, 122.50, 116.81, 109.65, 109.25.
HRMS (APCI): calcd. for C₁₈H₁₂N₅S [M+H]⁺ = 330.0808, found [M+H]⁺ = 330.0810.
FTIR (neat), cm⁻¹: 3400, 3067, 1620, 1574, 1524, 1397, 1360, 1254, 1184, 1063, 825, 796, 777, 639, 577.

### Preparative Example 116: tert-butyl 2-(((3-(pyridin-4-yl)thieno[3,2-b]pyridin-5-yl)oxy)-methyl)morpholine-4-carboxylate

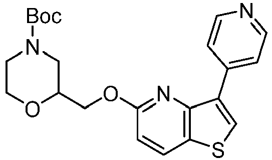

To a solution of 5-chloro-3-(pyridin-4-yl)thieno[3,2-*b*]pyridine **(Prep. Example 12**) (150 mg, 0.609 mmol) in toluene (9 mL) were added potassium hydroxide (82 mg, 1.46 mmol), *tert*-butyl *2-*(hydroxymethyl)morpholine-4-carboxylate (53 mg, 0.244 mmol) and 18-crown-6 (10 mg, 0.06 mmol) and the reaction mixture was refluxed for 16 hours. The reaction mixture was quenched with water (20 mL) and extracted with EtOAc (2 × 25 mL). The combined organic extracts were dried over MgSO4, filtered, and the solvents were evaporated *in vacuo.* The residue was purified by flash chromatography (cyclohexane/EtOAc, gradient from 60:40 to 40:60). The product was obtained as a pale yellow solid (156 mg, 60 % yield).
¹H NMR (500 MHz, Chloroform-*d*) *δ* (ppm) 8.70 (d, *J* = 6.3 Hz, 2H), 8.07 (d, *J* = 8.9 Hz, 1H), 8.02 - 7.97 (m, 3H), 6.91 (d, *J* = 8.7 Hz, 1H), 4.56 - 4.42 (m, 2H), 4.10 (br s, 1H), 3.97 (d, *J=* 11.5 Hz, 1H), 3.92 - 3.79 (m, 2H), 3.61 (td, *J* = 11.6, 2.8 Hz, 1H), 3.01 (s, 1H), 2.89 (s, 1H), 1.47 (s, 9H).
¹³C NMR (126 MHz, Chloroform-*d*) *δ* (ppm) 161.88, 154.77, 150.02, 149.94, 141.84, 133.24, 133.04, 129.00, 127.82, 122.28, 109.35, 80.20, 77.20, 73.73, 66.67, 66.41, 28.38.
HRMS (APCI): calcd. for C₂₂H₂₆N₃O₄S [M+H]⁺ = 428.1639, found [M+H]⁺ = 428.1638.
FTIR (neat), cm⁻¹: 2974, 2860, 1692, 1598, 1580, 1561, 1415, 1390, 1271, 1168, 1132, 1132, 1057, 730.

### Preparative Example 117: tert-butyl (S)-2-(((3-(pyridin-4-yl)thieno[3,2-b]pyridin-5-yl)oxy)-methyl)morpholine-4-carboxylate

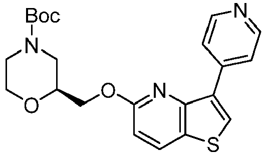

To a solution of 5-chloro-3-(pyridin-4-yl)thieno[3,2-*b*]pyridine **(Prep. Example 12**) (50 mg, 0.203 mmol) in toluene (3 mL) were added potassium hydroxide (23 mg, 0.406 mmol), tert-butyl (S)-2-(hydroxymethyl)-morpholine-4-carboxylate (53 mg, 0.244 mmol) and 18-crown-6 (5 µL, 0.006 mmol) and the reaction mixture was refluxed for 5 hours. The reaction mixture was quenched 1 M HCl until pH 7 and extracted with EtOAc (2 × 20 mL). The combined organic extracts were dried over MgSO4, filtered, and the solvents were evaporated *in vacuo.* The residue was purified by flash chromatography (cyclohexane/EtOAc, gradient from 80:20 to 0:100) and preparative TLC (cyclohexane/EtOAc, 80:20). The product was obtained as a pale yellow wax (22 mg, 33 %)
¹H NMR (500 MHz, Chloroform-*d*) *δ* (ppm) 8.72 (s, 2H), 8.07 (d, *J* = 8.9 Hz, 1H), 8.00 (d, *J* = 6.4 Hz, 3H), 6.91 (d, *J* = 8.9 Hz, 1H), 4.53 - 4.40 (m, 2H), 4.12 (br s, 1H), 3.98 (d, *J* = 10.8 Hz, 1H), 3.89 (dd, *J* = 5.3, 2.6 Hz, 2H), 3.61 (td, *J* = 11.7, 2.8 Hz, 1H), 3.02 (s, 1H), 2.89 (s, 1H), 1.47 (s, 9H).
¹³C NMR (126 MHz, Chloroform-*d*) *δ* (ppm) 153.89, 149.37, 149.12, 145.69, 144.20, 137.78, 135.30, 131.05, 130.78, 129.05, 126.92, 125.44, 124.28, 115.34, 108.10, 105.54, 39.24.
HRMS (APCI): calcd. for C₂₂H₂₆N₃O₄S [M+H]⁺ = 428.1639, found [M+H]⁺ = 428.1640.
FTIR (neat), cm⁻¹: 3417, 2926, 1692, 1599, 1416, 1391, 1271, 1271, 1168, 1132, 813.

### Preparative Example 118: 2-(((3-(pyridin-4-yl)thieno[3,2-b]pyridin-5-yl)oxy)methyl)-morpholine

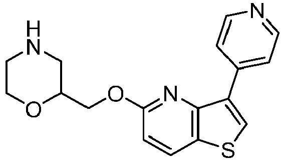

To a solution of *tert*-butyl 2-(((3-(pyridin-4-yl)thieno[3,2-*b*]pyridin-5-yl)oxy)-methyl)-morpholine -4-carboxylate **(Prep. Example 116)** (125 mg, 0.292 mmol) in dichloromethane (2.5 mL) was added dropwise trifluoroacetic acid (1 mL) and the reaction mixture was stirred at 25 °C for 45 min. The residue was purified by flash chromatography (dichloromethane/methanol, gradient from 100:0 to 85:5) and the resulting mixture was extracted in EtOAc (10 mL) and washed with a saturated aqueous solution of NaHCO₃ (2 × 15 mL). The combined organic extracts were dried over MgSO₄, filtered and the solvent was evaporated *in vacuo.* The product was obtained as a pale yellow solid (84 mg, 88 %).
¹H NMR (500 MHz, Methanol-*d₄*) *δ* (ppm) 8.61 (dd, *J* = 4.7, 1.8 Hz, 2H), 8.43 (s, 1H), 8.26 (d, *J* = 8.9 Hz, 1H), 8.24 (dd, *J* = 4.7, 1.7 Hz, 2H), 6.92 (d, *J* = 8.9 Hz, 1H), 4.48 (t, *J* = 5.0 Hz, 2H), 4.07 - 4.03 (m, 1H), 4.02 - 3.96 (m, 1H), 3.77 - 3.64 (m, 1H), 3.17 (dd, *J* = 12.7, 2.4 Hz, 1H), 3.01 - 2.94 (m, 2H), 2.89 (dd, *J* = 12.6, 10.8 Hz, 1H).
¹³CNMR (126 MHz, Methanol-*d₄*) *δ* (ppm) 163.32, 151.27, 150.20, 144.36, 135.04, 133.22, 132.08, 129.45, 123.87, 110.12, 74.98, 67.72, 67.32, 47.73, 45.52.
HRMS (APCI): calcd. for C₁₇H₁₈N₃O₂S [M+H]⁺ = 328.1114, found [M+H]⁺ = 328.1111.
FTIR (neat), cm⁻¹: 3600 - 3142, 2924, 2854, 1633 - 2364, 1674, 1602, 1448, 1270, 1201, 1129, 830, 784, 642.

### Preparative Example 119: 3-(isothiazol-5-yl)-5-(1-methyl-1H-pyrazol-3-yl)thieno[3,2-b]pyridine

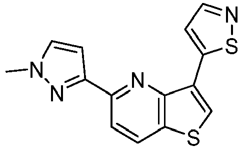

The compound was prepared according to General procedure B using 5-chloro-3-(isothiazol-5-yl)thieno[3,2-*b*]pyridine **(Prep. Example 66)** (45 mg, 0.178 mmol) and 1-methyl-3-(4,4,5,5-tetramethyl-1,3,2-dioxaborolan-2-yl)-1*H*-pyrazole (44 mg, 0.214 mmol). Reaction time: 45 min. The solvents were evaporated and the residue was purified by flash chromatography (hexane/EtOAc, gradient from 80:20 to 50:50). The product was obtained as a white solid (26 mg, 49 % yield).
¹H NMR (500 MHz, Chloroform-*d*) *δ* (ppm) 8.53 (d, *J* = 2.0 Hz, 1H), 8.22 (d, *J* = 8.4 Hz, 1H), 8.12 (d, *J* = 8.5 Hz, 1H), 8.09 (s, 1H), 7.77 (d, *J* = 1.8 Hz, 1H), 7.49 (d, *J* = 2.3 Hz, 1H), 7.27 (d, *J* = 2.3 Hz, 1H), 4.02 (s, 3H).
¹³C NMR (126 MHz, Chloroform-*d*) *δ* (ppm) 157.60, 155.81, 151.88, 151.71, 150.58, 131.81, 131.67, 131.20, 128.25, 126.34, 119.42, 116.27, 105.67, 39.28.
HRMS (APCI): calcd. for C₁₄H₁₁N₄S₂ [M+H]⁺ = 299.0420, found [M+H]⁺ = 299.0423.
FTIR (neat), cm⁻¹: 3099, 1570, 1558, 1422, 1282, 1225, 829, 814, 778, 768, 548, 438, 434.

### Preparative Example 120: 3-((3-(isothiazol-5-yl)thieno[3,2-b]pyridin-5-yl)amino)-1-metyl-1H-pyrazole-4-carbonitrile

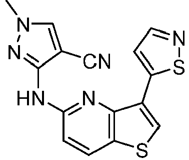

The compound was prepared according to General procedure C using 5-chloro-3-(isothiazol-5-yl)thieno[3,2-b]pyridine **(Prep. Example 66)** (45 mg, 0.178 mmol) and 1,4-dimethyl-1*H*-pyrazol-3-amine (26 mg, 0.214 mmol). After 30 hours, BrettPhosPdG3 (10 mg, 0.011 mmol) and methanol (1 mL) were added and the mixture was refluxed for additional 16 hours. The solvents were evaporated and the residue was purified by flash chromatography (cyclohexane/EtOAc, gradient from 70:30 to 0:100). The product was obtained as a white solid (5 mg, 10 % yield).
¹H NMR (500 MHz, Methanol-*d₄*) *δ* (ppm) 8.44 (d, *J* = 1.9 Hz, 1H), 8.39 (s, 1H), 8.22 (s, 1H), 8.17 (d*, J* = 8.9 Hz, 1H), 7.90 (d, *J* = 2.0 Hz, 1H), 7.20 (d, *J=* 8.8 Hz, 1H), 3.95 (s, 3H).
¹³CNMR (126 MHz, Methanol-*d₄*) *δ* (ppm) 159.51, 156.95, 155.58, 152.53, 151.60, 138.06, 133.74, 128.65, 128.10, 126.73, 120.66, 114.42, 109.96, 87.05, 39.78.
HRMS (APCI): calcd. for C₁₅H₁₁N₆S₂ [M+H]⁺ = 339.0481, found [M+H]⁺ = 339.0478.
FTIR (neat), cm⁻¹: 3089, 2224, 1581, 1552, 1533, 1400, 1349, 1163, 799.

### Preparative Example 121: 3-phenyl-5-(1H-pyrazol-4-yl)thieno[3,2-b]pyridine

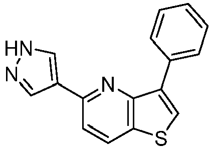

The compound was prepared according to General procedure B using 5-chloro-3-phenylthieno[3,2-*b*]pyridine **(Prep. Example 1)** (63 mg, 0.259 mmol) and *tert*-butyl 4-(4,4,5,5-tetramethyl-1,3,2-dioxaborolan-2-yl)-1*H*-pyrazole-1-carboxylate (96 mg, 0.324 mmol). After 2 hours at 110 °C, the reaction was stirred at 25 °C for 16 hours. The solvents were evaporated and the residue was purified by flash chromatography (hexane/EtOAc, gradient from 100:0 to 50:50) and preparative TLC (dichloromethane/methanol, 98:2). The product was obtained as a white solid (27 mg, 37 % yield).
¹H NMR (500 MHz, Methanol-*d₄*) *δ* (ppm) 8.32 (d, *J=* 8.4 Hz, 1H), 8.23 (s, 2H), 8.12 (d, *J=* 7.7 Hz, 2H), 8.06 (s, 1H), 7.69 (d, *J* = 8.4 Hz, 1H), 7.49 (t, *J* = 7.6 Hz, 2H), 7.38 (t, *J* = 7.4 Hz, 1H).
¹³CNMR(126 MHz, Methanol-*d₄*) *δ* (ppm) 154.22, 150.96, 137.64, 136.13, 133.35, 132.80, 129.56, 129.38, 128.83, 128.58, 116.95.
HRMS calculated for C₁₆H₁₁N₃S [M+H]⁺= 278.0746, found [M+H]⁺ = 278.0744.
FTIR (neat), cm⁻¹: 3136, 1583, 1566, 1397, 1381, 1254, 1214, 1125, 1027, 987, 864, 811, 743, 717, 687, 614, 544, 489.

### Preparative Example 122: 3-phenyl-5-(3,4,5-trifluorophenyl)thieno[3,2-b]pyridine

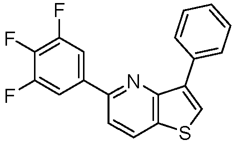

The compound was prepared according to General procedure B using 5-chloro-3-phenylthieno[3,2-*b*]pyridine **(Prep. Example 1)** (81 mg, 0.330 mmol) and (3,4,5-trifluorophenyl)boronic acid (70 mg, 0.396 mmol). Reaction temperature: 80 °C. Reaction time: 19 hours. The solvents were evaporated and the residue was purified by flash chromatography (hexane/EtOAc, gradient from 100:0 to 95:5). The product was obtained as a white solid (60 mg, 54 % yield).
¹H NMR (500 MHz, Chloroform-*d*) *δ* (ppm) 8.29 (d, *J* = 8.5 Hz, 1H), 8.06 (d, *J* = 6.8 Hz, 1H), 7.90 (s, 1H), 7.79 (dd, *J* = 8.8, 6.6 Hz, 2H), 7.68 (d, *J* = 8.5 Hz, 1H), 7.55 (t, *J* = 7.5 Hz, 2H), 7.45 (d, *J* = 7.5 Hz, 1H). ¹³C NMR (126 MHz, Chloroform-*d*) *δ* (ppm) 153.34, 152.51 (dd, *J* = 10.1, 4.17 Hz), 151.22 (q, *J=* 2.4 Hz), 150.54 (dd, *J* = 10.1, 4.0 Hz), 141.32 (t, *J* = 15.6 Hz), 139.30 (t, *J* = 15.6 Hz), 137.15, 135.67 - 135.52 (m), 134.29, 133.73, 131.68, 128.56, 128.54, 127.95, 127.89, 115.26, 110.99 (dd, *J* = 17.5, 5.4 Hz).
¹⁹F NMR (471 MHz, Chloroform-*d*) *δ* -133.95 (d, *J* = 20.5 Hz), -160.18 (t, *J* = 20.4 Hz).
HRMS calcd. for C₁₉H₁₁F₃NS [M+H]⁺= 342.0559, found [M+H]⁺ = 342.0557.
FTIR (neat), cm⁻¹: 1619, 1551, 1520, 1452, 1444, 1412, 1390, 1353, 1240, 1207, 1172, 1037, 973, 865, 778, 750, 733, 692, 644, 585, 441.

### Preparative Example 123: 5-(furan-3-yl)-3-phenylthieno[3,2-b]pyridine

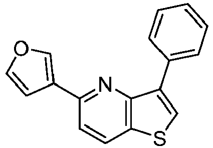

The compound was prepared according to General procedure B using 5-chloro-3-phenylthieno[3,2-*b*]pyridine **(Prep. Example 1)** (75 mg, 0.305 mmol) and 2-(furan-3-yl)-4,4,5,5-tetramethyl-1,3,2-dioxaborolane (71 mg, 0.366 mmol). Reaction temperature: 80 °C. Reaction time: 20 hours. The solvents were evaporated and the residue was purified by flash chromatography (hexane/EtOAc, gradient from 100:0 to 98:2). The product was obtained as a brown solid (77 mg, 92 % yield).
¹H NMR (500 MHz, Chloroform-*d*) *δ* (ppm) 8.18 (d, *J* = 8.4 Hz, 1H), 8.15 - 8.07 (m, 3H), 7.85 (s, 1H), 7.53 - 7.50 (m, 3H), 7.49 (d, *J* = 4.0 Hz, 1H), 7.40 (t, *J* = 7.4 Hz, 1H), 7.03 (d, *J=* 1.1 Hz, 1H).
¹³C NMR (126 MHz, Chloroform-*d*) *δ* (ppm) 153.23, 149.20, 143.74, 141.35, 136.79, 134.60, 132.23, 131.07, 128.54, 128.37, 127.62, 127.00, 124.30, 115.83, 109.09.
HRMS calcd. for C₁₇H₁₂NOS [M+H]⁺= 278.0634, found [M+H]⁺ = 278.0637.
FTIR (neat), cm⁻¹: 1596, 1564, 1550, 1504, 1390, 1157, 1063, 872, 791, 777, 750, 719, 692, 555.

### Preparative Example 124: 4-(3-phenylthieno[3,2-b]pyridin-5-yl)isoxazole

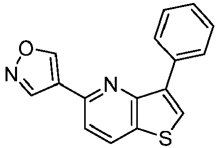

The compound was prepared according to General procedure B using 5-chloro-3-phenylthieno[3,2-*b*]pyridine **(Prep. Example 1)** (80 mg, 0.330 mmol) and 4-isoxazoleboronic acid pinacol ester (77 mg, 0.396 mmol). Reaction temperature: 80 °C. After 3 hours, triethylamine (0.15 mL, 0.990 mmol) was added and the mixture was refluxed for additional 18 hours. The solvents were evaporated and the residue was purified by flash chromatography (hexane/EtOAc, gradient from 100:0 to 98:2). The product was obtained as a yellow solid (18 mg, 30 % yield).
¹H NMR (500 MHz, Chloroform-*d*) *δ* (ppm) 8.94 (d, *J* = 2.3 Hz, 1H), 8.27 (d, *J* = 9.0 Hz, 1H), 7.84 (s, 1H), 7.68 - 7.57 (m, 5H), 7.52 (t, *J* = 7.2 Hz, 1H), 7.39 (d, *J* = 9.0 Hz, 1H).
¹³C NMR (126 MHz, Chloroform-*d*) *δ* (ppm) 181.22, 153.03, 141.21, 135.36, 133.23, 131.86, 130.09, 129.61, 129.31, 129.19, 127.99, 119.64, 115.77, 80.93.
HRMS calculated for C₁₆H₁₁N₂O_{S} [M+H]⁺= 279.0587, found [M+H]⁺ = 279.0588.
FTIR (neat), cm⁻¹: 3127, 2957, 2921, 2192, 1731, 1598, 1511, 1496, 1424, 1378, 1319, 1249, 1233, 1155, 960, 900, 860, 814, 795, 757, 738, 695, 652, 640, 594, 558, 486, 448, 421.

### Preparative Example 125: 4-(5-chlorothieno[3,2-b]pyridin-3-yl)-N,N-dimethylpyridin-2-amine

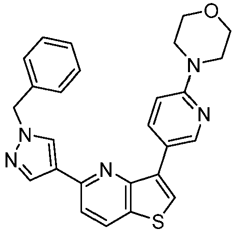

The compound was prepared according to General procedure B using 4-(5-(5-chlorothieno[3,2-b]pyridin-3-yl)pyridin-2-yl)morpholine **(Prep. Example 41)** (45 mg, 0.136 mmol) and 1-benzyl-4-(4,4,5,5-tetramethyl-1,3,2-dioxaborolan-2-yl)-1*H*-pyrazole (48 mg, 0.170 mmol). After 4 hours, triethylamine was added and the mixture was refluxed for additional 16 hours. The solvents were evaporated and the residue was purified by two flash chromatography (cyclohexane/EtOAc, gradient from 80:20 to 60:40). The product was obtained as a white solid (41 mg, 67 % yield).
¹HNMR(500 MHz, Chloroform-*d*) *δ* (ppm) 8.90 (s, 1H), 8.30 (dd, *J* = 8.8, 2.4 Hz, 1H), 8.14 *(d, J=* 8.4 Hz, 1H), 8.11 (s, 1H), 8.04 (br s, 1H), 7.73 (s, 1H), 7.47 (s, 1H), 7.41 - 7.31 (m, 3H), 7.29 - 7.27 (m, 2H), 6.77 (d, *J* = 8.8 Hz, 1H), 5.39 (s, 2H), 3.88 (s, 4H), 3.60 (s, 4H).
¹³C NMR (126 MHz, Chloroform-*d*) *δ* (ppm) 153.21, 149.49, 147.11, 138.22, 137.75, 136.39, 131.54, 131.14, 128.85, 128.54, 128.10, 127.69, 125.00, 124.27, 121.10, 115.58, 106.55, 66.76, 56.28, 45.85.
HRMS (APCI): calcd. for C₂₆H₂₄N₅OS [M+H]⁺ = 454.1696, found [M+H]⁺ = 454.1693.
FTIR (neat), cm⁻¹: 3060, 2960, 2857, 1602, 1577, 1556, 1481, 1450, 1402, 1365, 1317, 1243, 1215, 1182, 1173, 1114, 991, 942, 796, 702, 628, 578, 470.

### Preparative Example 126: Ethyl 3-((3-(2-((tert-butoxycarbonyl)amino)pyridin-4-yl)thieno[3,2-b]pyridin-5-yl)amino)-1-methyl-1H-pyrazole-4-carboxylate

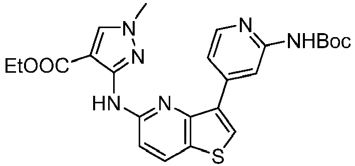

The compound was prepared according to General procedure C using tert-butyl (4-(5-chlorothieno[3,2-*b*]pyridin-3-yl)pyridin-2-yl)carbamate **(Prep. Example 23)** (100 mg, 0.276 mmol) and ethyl 3-amino-1-methyl-1*H*-pyrazole-4-carboxylate (56 mg, 0.331 mmol). Reaction time: 3 hours. The solvents were evaporated and the residue was purified by flash chromatography (cyclohexane/EtOAc/methanol, gradient from 70:30:0 to 0:95:5). The product was obtained as a white solid (77 mg, 73 % yield).
¹H NMR (500 MHz, Chloroform-*d*) *δ* (ppm) 8.85 (s, 1H), 8.46 (m, 1H), 8.35 (dd, *J* = 5.2, 0.8 Hz, 1H), 8.18 (d, *J* = 8.9 Hz, 1H), 8.13 (d, *J* = 3.1 Hz, 1H), 8.12 (d, *J* = 4.3 Hz, 1H), 8.08 (s, 1H), 7.72 (s, 1H), 7.46 (s, 1H), 4.33 (q, *J* = 7.1 Hz, 2H), 3.88 (s, 3H), 1.56 (s, 9H), 1.39 (t, *J* = 7.1 Hz, 3H).
¹³C NMR (126 MHz, Chloroform-*d*) *δ* (ppm) 163.81, 152.42, 152.15, 151.99, 151.60, 151.54, 148.00, 144.12, 133.11, 132.86, 132.39, 129.64, 126.77, 118.13, 110.42, 108.60, 100.08, 80.81, 60.08, 39.42, 28.34, 14.38.
HRMS (APCI): calcd. for C₂₄H₂₇N₆O₄S [M+H]⁺ = 495.1809, found [M+H]⁺ = 495.1805.
FTIR (neat), cm⁻¹: 2977, 1717, 1689, 1566, 1391, 1287, 1156, 1057, 773.

### Preparative Example 127: Methyl 3-((3-(2-(((tert-butoxycarbonyl)amino)methyl)pyridin-4-yl)thieno[3,2-b]pyridin-5-yl)amino)-1-methyl-1H-pyrazole-4-carboxylate (intermediate)

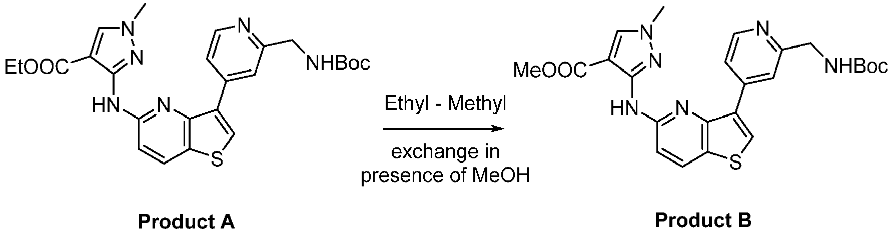

The compound was prepared according to General procedure C using tert-butyl ((4-(5-chlorothieno[3,2-b]pyridin-3-yl)pyridin-2-yl)methyl)carbamate **(Prep. Example 26)** (427 mg, 1.13 mmol) and ethyl 3-amino-1-methyl-1*H*-pyrazole-4-carboxylate (229 mg, 1.36 mmol). Reaction time: 16 hours. The solvents were evaporated and the residue was purified by flash chromatography (cyclohexane/EtOAc, gradient from 50:50 to 0:100). The product was obtained as an impure pale yellow solid (64 mg, < 55 % yield), which was used without further purification in the next step.
¹H NMR (500 MHz, Chloroform-*d*) *δ* (ppm) 8.80 (s, 1H), 8.64 (d, *J* = 5.2 Hz, 1H), 8.16 (d, *J* = 8.9 Hz, 1H), 8.13 (s, 1H), 7.97 (s, 1H), 7.92 - 7.90 (m, 1H), 7.70 (s, 1H), 7.54 (s, 1H), 5.69 (s, 1H), 4.54 (d, *J* = 5.0 Hz, 2H), 4.26 (q, *J* = 7.1 Hz, 2H), 3.69 (s, 3H), 1.46 (s, 9H), 1.32 (t, *J* = 7.1 Hz, 3H).
¹³C NMR (126 MHz, Chloroform-*d*) *δ* (ppm) 164.27, 156.46, 151.99, 151.56, 151.40, 149.39, 142.61, 133.13, 132.84, 132.77, 132.43, 129.21, 126.79, 121.30, 120.26, 108.82, 99.74, 79.29, 59.65, 51.28, 39.45, 28.42, 14.45.
HRMS (APCI): calcd. for C₂₅H₂₉N₆O₄S [M+H]⁺ = 509.1966 **(Product A),** found [M+H]⁺ = 495.1809 (measure in MeOH, **Product B** found as unique product).
FTIR (neat), cm⁻¹: 3360, 2978, 1688, 1568, 1553, 1511, 1391, 1246, 1165, 1109, 775, 730, 647.

### Preparative Example 128: Methyl 3-((3-(2-(aminomethyl)pyridin-4-yl)thieno[3,2-b]pyridin-5-yl)amino)-1-methyl-1H-pyrazole-4-carboxylate

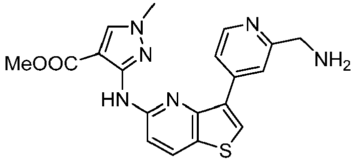

The compound was prepared according to General procedure D using ethyl 3-((3-(2-((*tert-*butoxycarbonyl)amino)pyridin-4-yl)thieno[3,2-*b*]pyridin-5-yl)amino)-1-methyl-1*H*-pyrazole-4-carboxylate **(Prep. Example 128)** (251 mg, 0.495 mmol). Reaction time: 16 hours. The solvents were evaporated and the residue was purified by preparative TLC (dichloromethane/methanol/7 M NH₃ in methanol, 92:5:3 - 1 elution; then EtOAc - 3 elutions). The product was obtained as a pale yellow solid (95 mg, 47 % yield).
¹H NMR (500 MHz, Methanol-*d₄*) *δ* (ppm) 8.71 (d, *J* = 5.1 Hz, 1H), 8.42 (s, 1H), 8.34 (s, 1H), 8.30 (d, *J* = 9.0 Hz, 1H), 8.25 - 8.21 (m, 2H), 8.02 (s, 1H), 4.39 (s, 2H), 3.89 (s, 3H), 3.86 (s, 3H).
¹³CNMR (126 MHz, Methanol-*d₄*) *δ* (ppm) 166.31, 153.76, 153.57, 152.69, 152.48, 150.66, 144.86, 134.88, 134.04, 132.65, 132.37, 128.42, 123.36, 121.90, 109.93, 100.50, 51.74, 44.46, 39.56.
HRMS (APCI): calcd. for C₁₉H₁₉N₆O₂S₂ [M+H]⁺ = 395.1285, found [M+H]⁺ = 395.1282.
FTIR (neat), cm⁻¹: 3404, 3368, 1688, 1604, 1580, 1391, 1299, 1124, 774.

### Preparative Example 129: 3-([1,2,4]triazolo[1,5-α]pyridin-6-yl)-5-(1-methyl-1H-pyrazol-3-yl)thieno[3,2-b]pyridine

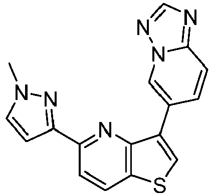

The compound was prepared according to General procedure B using 3-([1,2,4]triazolo[1,5-*a*]pyridin-6-yl)-5-chlorothieno[3,2-b]pyridine **(Prep. Example 52)** (50 mg, 0.174 mmol) and 1-methyl-3-(4,4,5,5-tetramethyl-1,3,2-dioxaborolan-2-yl)-1*H*-pyrazole (44 mg, 0.214 mmol). After 6 hours, additional potassium phosphate (55 mg, 0.261 mmol), [1,1'-bis(diphenylphosphino)ferrocene]dichloropalladium(II) (13 mg, 0.018 mmol) and H₂O (0.2 mL) were added and the mixture was refluxed for additional 16 hours.

The solvents were evaporated and the residue was purified by flash chromatography (hexane/EtOAc, gradient from 80:20 to 0:100) and preparative TLC (dichloromethane/methanol, 97:3). The product was obtained as a brown solid (10 mg, 17 % yield).
¹H NMR (500 MHz, Chloroform-*d*) *δ* (ppm) 10.19 (s, 1H), 8.53 (s, 1H), 8.31 (d, *J=* 9.1 Hz, 1H), 8.29 (d, *J* = 8.5 Hz, 1H), 8.15 (d, *J* = 8.6 Hz, 1H), 8.07 (d, *J* = 8.0 Hz, 2H), 7.47 (d, *J* = 2.2 Hz, 1H), 7.06 (d, *J* = 2.3 Hz, 1H), 4.03 (s, 3H).
¹³C NMR (126 MHz, Chloroform-*d*) *δ* (ppm) 157.74, 153.16, 152.10, 151.85, 150.47, 132.89, 131.86, 131.41, 131.05, 130.74, 127.99, 127.72, 122.41, 116.29, 115.79, 105.07, 39.29.
HRMS (APCI): calcd. for C₁₇H₁₃N₆S [M+H]⁺ = 333.0917, found [M+H]⁺ = 333.0921.

### Preparative Example 130: 3-([1,2,4]triazolo[1,5-a]pyridin-6-yl)-5-(pyrimidin-2-yl)thieno[3,2-b]pyridine

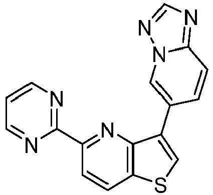

To a solution of 3-([1,2,4]triazolo[1,5-*a*]pyridin-6-yl)-5-chlorothieno[3,2-*b*] **(Prep. Example 52)** (50 mg, 0.174 mmol) in dioxane (3 mL) were added 2-(tributylstannyl)pyrimidine (73 µL, 0.226 mmol) and bis(tri-tert-butylphosphine)palladium (0) (7 mg, 0.014 mmol) and the reaction mixture was refluxed for 16 hours. The solvents were evaporated and the residue was purified by flash chromatography (cyclohexane/EtOAc/methanol, gradient from 50:50:0 to 0:95:5). The product was obtained as a white solid (18 mg, 32 %).
¹H NMR (500 MHz, DMSO-*d₆*) *δ* (ppm) 10.50 (s, 1H), 9.08 (d, *J* = 4.9 Hz, 2H), 8.86 (s, 1H), 8.80 (d, *J* = 8.5 Hz, 1H), 8.57 (s, 1H), 8.53 (d, *J* = 8.5 Hz, 1H), 8.46 (dd, *J* = 9.3, 1.7 Hz, 1H), 8.01 (d, *J* = 9.3 Hz, 1H), 7.63 (d, *J* = 4.8 Hz, 1H).
¹³C NMR (126 MHz, DMSO-*d₆*) *δ* (ppm) 163.12, 158.05, 154.37, 152.42, 152.40, 149.06, 135.04, 132.70, 130.98, 130.21, 129.72, 127.29, 121.18, 121.09, 118.96, 115.92.
HRMS (APCI): calcd. for C₁₇H₁₁N₆S [M+H]⁺ = 330.0808, found [M+H]⁺ = 331.0708.
FTIR (neat), cm⁻¹: 1638, 1555, 1439, 1421, 1407, 1256, 907, 793, 715, 656, 634, 586, 447.

### Preparative Example 131: 3-([1,2,4]triazolo[1,5-a]pyridin-6-yl)-5-(pyridin-3-yl)thieno[3,2-b]pyridine

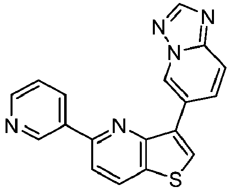

The compound was prepared according to General procedure B using 3-([1,2,4]triazolo[1,5-*a*]pyridin-6-yl)-5-chlorothieno[3,2-*b*]pyridine **(Prep. Example 52)** (50 mg, 0.174 mmol) and pyridin-3-ylboronic acid (26 mg, 0.209 mmol). Reaction time: 1 hour. The solvents were evaporated and the residue was purified by flash chromatography (EtOAc/methanol, gradient from 100:0 to 95:5). The product was obtained as a white solid (24 mg, 42 %).
¹H NMR (500 MHz, Methanol-*d₄*) *δ* (ppm) 10.09 (s, 1H), 9.37 (d, *J=* 2.3 Hz, 1H), 8.67 (dt, *J* = 7.9, 2.0 Hz, 1H), 8.64 (dd, *J* = 4.8, 1.6 Hz, 1H), 8.58 (d, *J* = 8.5 Hz, 1H), 8.53 (s, 1H), 8.48 (s, 1H), 8.41 (dd, *J* = 9.3, 1.7 Hz, 1H), 8.07 (d, *J* = 8.5 Hz, 1H), 7.91 (dd, *J* = 9.2, 0.9 Hz, 1H), 7.63 (dd, *J* = 8.0, 4.8 Hz, 1H).
¹³CNMR (126 MHz, Methanol-*d₄*) *δ* (ppm) 154.83, 154.12, 153.41, 150.64, 150.38, 148.91, 136.80, 136.66, 135.49, 133.78, 132.72, 131.74, 131.61, 128.78, 125.58, 123.82, 117.82, 116.57.
HRMS (APCI): calcd. for C₁₈H₁₂N₅S [M+H]⁺ = 330.0808, found [M+H]⁺ = 330.0811.
FTIR (neat), cm⁻¹: 3072, 1637, 1579, 1391, 1261, 1190, 811, 789, 699, 657, 574, 423.

### Preparative Example 132: 3-([1,2,4]triazolo[1,5-a]pyridin-6-yl)-5-(pyridin-4-yl)thieno[3,2-b]pyridine

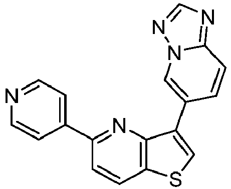

The compound was prepared according to General procedure B using 3-([1,2,4]triazolo[1,5-*a*]pyridin-6-yl)-5-chlorothieno[3,2-b]pyridine **(Prep. Example 52)** (41 mg, 0.139 mmol) and pyridin-4-ylboronic acid (21 mg, 0.167 mmol). After 2 hours, additional potassium phosphate (88 mg, 0.417 mmol) and [1,1'-bis(diphenylphosphino)ferrocene]dichloropalladium(II) (5 mg, 0.007 mmol) were added and the mixture was refluxed for additional 30 min. The solvents were evaporated and the residue was purified by flash chromatography (cyclohexane/EtOAc/methanol, gradient from 50:50:0 to 0:97:3). The product was obtained as a white solid (25 mg, 54 %).
¹H NMR (500 MHz, DMSO-*d₆*) *δ* (ppm) 10.08 (s, 1H), 8.80 (t, *J* = 4.3 Hz, 2H), 8.79 (d, *J* = 6.0 Hz, 2H), 8.59 (s, 1H), 8.44 (dd, *J* = 9.2, 1.8 Hz, 1H), 8.25 (s, 1H), 8.19 (dd, *J* = 4.5, 1.6 Hz, 2H), 8.03 (dd, *J =* 9.3, 0.9 Hz, 1H).
¹³C NMR (126 MHz, DMSO-*d₆*) *δ* (ppm) 154.42, 152.40, 151.55, 150.47, 149.10, 145.63, 134.30, 133.24, 131.38, 130.60, 129.98, 127.17, 121.21, 121.02, 116.96, 115.91.
HRMS (APCI): calcd. for C₁₈H₁₂N₅S [M+H]⁺ = 330.0808, found [M+H]⁺ = 330.0811.
FTIR (neat), cm⁻¹: 3094, 3060, 2955, 2923, 2853, 1719, 1598, 1549, 1437, 1393, 1315, 1264, 1239, 1183, 1167, 1124, 809, 726, 693, 658, 538.

### Preparative Example 133: tert-butyl 4-((3-([1,2,4]triazolo[1,5-a]pyridin-6-yl)thieno[3,2-b]pyridin-5-yl)amino)-1H-pyrazole-1-carboxylate

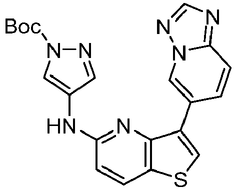

To a solution of 3-([1,2,4]triazolo[1,5-*a*]pyridin-6-yl)-5-chlorothieno[3,2-*b*]pyridine **(Prep. Example 52)** (60 mg, 0.209 mmol) in dioxane (2 mL) were added triethylamine (87 µL, 0.627 mmol), *tert*-butyl 4-amino-1*H*-pyrazole-1-carboxylate (46 mg, 0.251 mmol) and BrettPhosPdG3 (9 mg, 0.016 mmol) and the reaction mixture was refluxed for 16 hours. The solvents were evaporated and the residue was purified by flash chromatography (cyclohexane/EtOAc, gradient from 30:70 to 0:100). The product was obtained as a brown solid (30 mg, 33 %).
¹H NMR (500 MHz, Chloroform-*d*) *δ* (ppm) 9.47 (s, 1H), 8.44 (s, 1H), 8.37 (s, 1H), 8.16 (d, *J* = 9.2 Hz, 1H), 8.02 (d, *J* = 8.7 Hz, 1H), 7.91 (d, *J* = 9.3 Hz, 1H), 7.85 (s, 1H), 7.76 (s, 1H), 6.78 (d, *J* = 8.9 Hz, 1H), 6.57 (s, 1H), 1.58 (s, 9H).
¹³C NMR (126 MHz, Chloroform-*d*) *δ* (ppm) 154.17, 153.60, 151.33, 147.64, 137.93, 132.43, 130.81, 130.57, 127.54, 127.07, 125.66, 125.46, 122.37, 120.49, 119.86, 116.38, 107.53, 85.26, 27.88.
HRMS (APCI): calcd. for C₂₁H₂₀N₇O₂S [M+H]⁺ = 434.1394, found [M+H]⁺ = 434.1392.
FTIR (neat), cm⁻¹: 3295, 3095, 2981, 1746, 1580, 1419, 1381, 1371, 1249, 1149, 955, 843, 731, 660.

### Preparative Example 134: 3-([1,2,4]triazolo[1,5-a]pyridin-6-yl)-N-(1H-pyrazol-4-yl)thieno[3,2-b]pyridin-5-amine

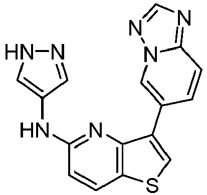

To a solution of *tert*-butyl 4-((3-([1,2,4]triazolo[1,5-*a*]pyridin-6-yl)thieno[3,2-*a*]pyridin-5-yl)amino)-1*H-*pyrazole-1-carboxylate **(Prep. Example 133)** (19 mg, 0.044 mmol) in dichloromethane (1 mL) was added trifluoroacetic acid (0.3 mL) and the reaction mixture was stirred at 25 °C for 2 hours. The solvents were evaporated and the residue was purified by TLC (cyclohexane/EtOAc/7 M NH₃ in methanol, 10:88:2). The product was obtained as a white solid (4 mg, 27 %).
¹H NMR (500 MHz, Chloroform-*d*) *δ* (ppm) 9.78 (s, 1H), 8.46 (s, 1H), 8.33 (d, *J* = 9.0 Hz, 1H), 8.15 (s, 1H), 8.03 (d, *J* = 9.0 Hz, 1H), 7.87 (d, *J* = 9.3 Hz, 2H), 7.73 (s, 1H), 6.81 (d, *J* = 8.9 Hz, 1H).
¹³C NMR (126 MHz, Chloroform-*d*) *δ* (ppm) 156.00, 154.73, 152.34, 133.15, 132.89, 131.20, 128.76, 128.51, 125.47, 124.75, 116.23, 109.82.
HRMS (APCI): calcd. for C₁₆H₁₂N₇S [M+H]⁺ = 334.0869, found [M+H]⁺ = 334.0865.
FTIR (neat), cm⁻¹: 2923, 2853, 2363, 2237, 2182, 2168, 2114, 1990, 1579, 1396, 426.

### Preparative Example 135: 3-([1,2,4]triazolo[4,3-a]pyridin-6-yl)-N-(1-methyl-1H-pyrazol-3-yl)thieno[3,2-b]pyridin-5-amine

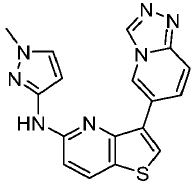

The compound was prepared according to General procedure B using 3-([1,2,4]triazolo[4,3-*a*]pyridin-6-yl)-5-chlorothieno[3,2-*b*]pyridine **(Prep. Example 58)** (55 mg, 0.192 mmol) and 1-methyl-1*H*-pyrazol-3-amine (20 µL, 0.230 mmol). Reaction time: 16 hours. The solvents were evaporated and the residue was purified by flash chromatography (cyclohexane/EtOAc/methanol, gradient from 50:50:0 to 0:95:5). The product was obtained as a white solid (27 mg, 40 %).
¹H NMR (500 MHz, Methanol-*d₄*) *δ* (ppm) 9.82 (s, 1H), 8.23 (s, 1H), 8.11 (dd, *J* = 9.2, 1.8 Hz, 2H), 7.82 (d, *J=* 9.6 Hz, 1H), 7.53 (d, *J* = 2.2 Hz, 1H), 7.16 (d, *J* = 8.9 Hz, 1H), 6.51 (d, *J=* 2.3 Hz, 1H), 3.84 (s, 3H).
¹³CNMR(126 MHz, Methanol-*d₄*) *δ* (ppm) 155.49, 151.02, 149.75, 144.57, 138.05, 133.36, 132.57, 131.83, 130.59, 129.19, 126.45, 124.00, 123.87, 115.19, 109.50, 97.87, 38.73.
HRMS (APCI): calcd. for C₁₇H₁₄N₇S [M+H]⁺ = 348.1026, found [M+H]⁺ = 348.1024.
FTIR (neat), cm⁻¹: 2923, 2853, 1709, 1583, 1545, 1437, 1411, 1165, 724, 699, 567, 533.

### Preparative Example 136: 5-chloro-3-(1-phenylvinyl)thieno[3,2-b]pyridine (intermediate)

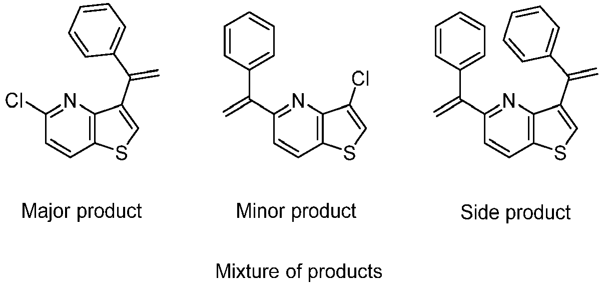

The compound was prepared according to General procedure A using 3-bromo-5-chlorothieno[3,2-*b*]pyridine (200 mg, 0.804 mmol) and (1-phenylvinyl)boronic acid (143 mg, 0.965 mmol). Reaction time: 2 hours. The solvents were evaporated and the residue was purified by flash chromatography (cyclohexane/EtOAc, gradient from 70:30 to 40:60) and preparative TLC (cyclohexane/EtOAc, 90:10 - 1 elution; 98:2 - 1 elution, 100:0 - 1 elution). The product was obtained as an impure yellow wax (150 mg, <69 % yield), which was used without further purification in the next step.
¹H NMR (500 MHz, Chloroform-*d*) (Major product) *δ* (ppm) 8.09 (d, *J* = 8.5 Hz, 1H), 7.48 (s, 1H), 7.42 (d, *J* = 1.4 Hz, 1H), 7.39 (d, *J* = 1.5 Hz, 1H), 7.36 - 7.34 (m, 3H), 7.29 (d, *J* = 8.5 Hz, 1H), 6.50 (d, *J* = 1.5 Hz, 1H), 5.74 *(d, J=* 1.5 Hz, 1H).
HRMS (APCI): calcd. for C₁₅H₁₁ClNS (Major product) [M+H]⁺ = 272.0295, found [M+H]⁺ = 272.0298.

### Preparative Example 137: 5-(1-methyl-1H-pyrazol-4-yl)-3-(1-phenylvinyl)thieno[3,2-b]pyridine and 3-(1-methyl-1H-pyrazol-4-yl)-5-(1-phenylvinyl)thieno[3,2-b]pyridine

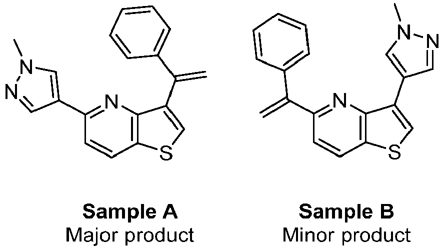

The compound was prepared according to General procedure B using of (5-chlorothieno[3,2-b]pyridin-3-yl)(phenyl)methanone (Prep. Example 136) (144 mg, 0.371 mmol) and 1-methyl-4-(4,4,5,5-tetramethyl-1,3,2-dioxaborolan-2-yl)-1*H*-pyrazole (93 mg, 0.445 mmol). Reaction time: 1 hour. The solvents were evaporated and the residue was purified by flash chromatography (cyclohexane/EtOAc, gradient from 98:2 to 97:3). The products were obtained as a yellow solid **(Sample A)** (29 mg, 25 %) and yellow wax **(Sample B)** (19 mg).
¹H NMR (500 MHz, Chloroform-*d*) **(Sample A)** *δ* (ppm) 8.17 (s, 1H), 8.13 (d, *J=* 8.5 Hz, 1H), 7.96 (s, 1H), 7.69 (s, 1H), 7.48 - 7.44 (m, 3H), 7.44 - 7.38 (m, 3H), 6.14 (d, *J* = 1.2 Hz, 1H), 5.69 (d, *J* = 1.2 Hz, 1H), 3.84 (s, 3H).
¹H NMR (500 MHz, Chloroform-*d*) **(Sample B)** *δ* (ppm) 8.11 (d, *J* = 8.4 Hz, 1H), 7.92 (s, 1H), 7.81 (s, 1H), 7.49 (s, 1H), 7.47 - 7.41 (m, 3H), 7.40 - 7.31 (m, 3H), 6.59 (d, *J* = 1.9 Hz, 1H), 5.70 (d, *J* = 1.9 Hz, 1H), 3.94 (s, 3H).
¹³CNMR (126 MHz, Chloroform-*d*) **(Sample A)** *δ* (ppm) 155.66, 152.72, 149.69, 141.18, 137.23, 132.47, 130.64, 129.03, 128.04, 127.48, 122.37, 117.60, 117.47, 115.77, 38.86.
¹³C NMR (126 MHz, Chloroform-*d*) **(Sample B)** *δ* (ppm) 153.74, 149.30, 142.97, 142.43, 137.71, 136.39, 131.33, 130.98, 129.72, 129.04, 128.22, 128.09, 127.41, 124.14, 117.07, 115.25, 39.11.
HRMS (APCI) **(Samples A):** calcd. for C₁₉H₁₆N₃S [M+H]⁺ = 318.1059, found [M+H]⁺ = 318.1061.
HRMS (APCI) **(Samples B):** calcd. for C₁₉H₁₆N₃S [M+H]⁺ = 318.1059, found [M+H]⁺ = 318.1059.
FTIR (neat), cm⁻¹**(Sample A):** 3094, 2933, 1563, 1539, 1402, 1375, 1174, 982, 777, 702.
FTIR (neat), cm⁻¹**(Sample B):** 3097, 2937, 1578, 1539, 1490, 1399, 1214, 979, 830, 805, 777, 701, 656.

### Preparative Example 138: 3-bromo-5-phenoxythieno[3,2-b]pyridine (intermediate)

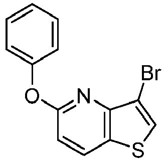

To a solution of 3-bromo-5-chlorothieno[3,2-*b*]pyridine (200 mg, 0.805 mmol) in DMF (8 mL) were added phenol (76 mg, 0.805 mmol), copper(I) cyanide (4 mg, 0.040 mmol), cesium carbonate (367 mg, 1.13 mmol) and *N,N*-dimethylethylenediamine (10 µL, 0.089 mmol) and the reaction mixture was refluxed for 16 hours. The solvents were evaporated and the residue was purified twice by flash chromatography (cyclohexane/EtOAc, gradient from 97:3 to 99:1) and preparative TLC (cyclohexane/EtOAc, 98:2 - 1 elution; cyclohexane, 100 % - 2 elutions; toluene, 100 % - 2 elutions). The product was obtained as a pale yellow solid (68 mg, 22 %), which was used without further purification in the next step.
¹H NMR (500 MHz, Chloroform-*d*) *δ* (ppm) 7.99 (d, *J =* 8.7 Hz, 1H), 7.61 (s, 1H), 7.34 - 7.27 (m, 2H), 7.17 - 7.14 (m, 2H), 7.13 - 7.08 (m, 1H), 6.81 (d, *J* = 8.7 Hz, 1H).
¹³C NMR (126 MHz, Chloroform-*d*) *δ* (ppm) 162.59, 154.23, 150.24, 133.88, 129.57, 128.84, 127.70, 126.83, 124.64, 120.92, 109.31.
HRMS (APCI): calcd. for C₁₃H₉ClNOS [M+H]⁺ = 262.0088, found [M+H]⁺ = not found.
FTIR (neat), cm⁻¹: 3102, 1572, 1546, 1483, 1390, 1281, 1235, 894, 811, 767, 693.

### Preparative Example 139: 3-(1-methyl-1H-pyrazol-4-yl)-5-phenoxythieno[3,2-b]pyridine

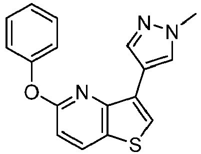

To a solution of 3-bromo-5-phenoxythieno[3,2-*b*]pyridine (45 mg, 0.206 mmol) **(Prep. Example 138)** in dioxane (3 mL) were added tripotassium phosphate (131 mg, 0.618 mmol), 1-methyl-4-(4,4,5,5-tetramethyl-1,3,2-dioxaborolan-2-yl)-1*H*-pyrazole (52 mg, 0.248 mmol), [1,1'-Bis(diphenylphosphino)ferrocene]dichloropalladium(II) (12 mg, 0.016 mmol) and H₂O (0.5 mL) and the reaction mixture was refluxed for 4 hours. The solvents were evaporated and the residue was purified twice by flash chromatography (cyclohexane/EtOAc, gradient from 60:40 to 40:60). The product was obtained as a pale yellow wax (22 mg, 35 %).
¹H NMR (500 MHz, Chloroform-*d*) *δ* (ppm) 8.13 (d, *J* = 8.7 Hz, 1H), 7.82 (d, *J* = 7.7 Hz, 2H), 7.64 (s, 1H), 7.52 - 7.46 (m, 2H), 7.33 - 7.29 (m, 1H), 7.28 - 7.26 (m, 2H), 7.03 (d, *J=* 8.7 Hz, 1H), 3.76 (s, 3H).
¹³C NMR (126 MHz, Chloroform-*d*) *δ* (ppm) 162.31, 154.29, 150.45, 136.83, 133.67, 129.52, 128.81, 128.04, 127.98, 124.68, 122.50, 122.33, 115.60, 108.21, 38.71.
HRMS (APCI): calcd. for C₁₇H₁₄N₃OS [M+H]⁺ 308.0852, found [M+H]⁺ = 308.0854.
FTIR (neat), cm⁻¹: 1574, 1549, 1488, 1398, 1375, 1277, 1237, 1199, 1158, 982, 811, 772, 692.

### Preparative Example 140: 5-chloro-2-iodo-3-(pyridin-4-yl)thieno[3,2-b]pyridine (intermediate)

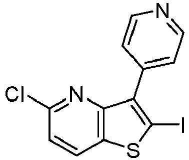

To a degassed solution of 5-chloro-3-(pyridin-4-yl)thieno[3,2-*b*]pyridine **(Prep. Example 12)** (200 mg, 0.812 mmol) in carbon tetrachloride (96 µL) were added iodine (62 mg, 0.243 mmol), NaIO₄ (86 mg, 0.406 mmol), acetic acid (1.2 mL), sulfuric acid (0.4 mL) and H₂O (0.1 mL) and the mixture was stirred at 65 °C for 8 hours. The reaction mixture was quenched with Na₂S₂O₃ (sat) (20 mL), extracted with EtOAc (2 × 20 mL) and washed with H₂O (20 mL). The combined organic extracts were dried over MgSO₄, filtered, and the solvent was evaporated *in vacuo.* The residue obtained after the workup was purified by flash chromatography (cyclohexane/EtOAc, gradient from 90:10 to 0:100). The product was obtained as a yellow solid (71 mg, 24 %).
¹H NMR (500 MHz, Chloroform-*d*) *δ* (ppm) 8.79 (d, *J* = 3.0 Hz, 2H), 8.07 (d, *J* = 8.5 Hz, 1H), 7.61 (d*, J* = 5.7 Hz, 2H), 7.30 (d, *J* = 8.5 Hz, 1H).
¹³C NMR (126 MHz, Chloroform-*d*) *δ* (ppm) 151.95, 149.85, 149.45, 149.11, 142.48, 140.96, 136.61, 131.46, 125.33, 120.29, 88.27.
HRMS (APCI): calcd. for C₁₂H₇ClHN₂S [M+H]⁺ = 372.9058, found [M+H]⁺ = 372.9054.
FTIR (neat), cm⁻¹: 1600, 1559, 1377, 1264, 1152, 1126, 993, 811, 733. 702, 616.

### Preparative Example 141: 5-chloro-2-methyl-3-(pyridin-4-yl)thieno[3,2-b]pyridine (intermediate)

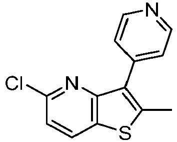

To a solution of 5-chloro-2-iodo-3-(pyridin-4-yl)thieno[3,2-*b*]pyridine **(Prep. Example 140)** (130 mg, 0.349 mmol) in dioxane (7 mL) were added potassium phosphate (222 mg, 1.05 mmol), methylboronic acid (25 mg, 0.419 mmol), [1,1'-bis(diphenylphosphino)-ferrocene]dichloropalladium(II) (13 mg, 0.017 mmol) and H₂O (1.7 mL) and the mixture was refluxed for 4 hours. Additional potassium phosphate (222 mg, 1.05 mmol) and [1,1'-bis(diphenyl-phosphino)ferrocene]dichloropalladium(II) (13 mg, 0.017 mmol) were added, and the reaction mixture was stirred for additional 4 hours. The solvents were evaporated and the residue was purified by flash chromatography (cyclohexane/EtOAc, gradient from 100:0 to 40:60). The product was obtained as a white solid (35 mg, 39 %).
¹H NMR (500 MHz, Chloroform-*d*) *δ* (ppm) 8.75 (d, *J* = 5.1 Hz, 2H), 8.04 (d, *J* = 8.4 Hz, 1H), 7.51 (dd, *J* = 4.8, 1.6 Hz, 2H), 7.28 (s, 1H), 2.67 (s, 3H).
¹³C NMR (126 MHz, Chloroform-*d*) *δ* (ppm) 154.15, 149.62, 149.17, 144.64, 141.49, 132.01, 130.67, 125.08, 119.46, 15.81.
HRMS (APCI): calcd. for C₁₃H₁₀ClN₂S [M+H]⁺ = 261.0248, found [M+H]⁺ = 261.0247.
FTIR (neat), cm⁻¹: 3040, 2917, 1604, 1555, 1525, 1493, 1393, 1355, 1153, 1121, 989, 966, 836, 810, 765, 631, 532, 460.

### Preparative Example 142: 2-methyl-5-(1-methyl-1H-pyrazol-4-yl)-3-(pyridin-4-yl)thieno[3,2-b]pyridine

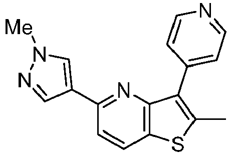

To a solution of 5-chloro-2-methyl-3-(pyridin-4-yl)thieno[3,2-*b*]pyridine **(Prep. Example 141)** (25 mg, 0.095 mmol) in dioxane (1.5 mL) were added potassium phosphate (61 mg, 0.288 mmol), 1-methyl-4-(4,4,5,5-tetramethyl-1,3,2-dioxaborolan-2-yl)-1*H*-pyrazole (24 mg, 0.115 mmol), [1,1'-bis(diphenylphosphino)ferrocene]dichloropalladium(II) (4 mg, 0.005 mmol) and H₂O (0.3 mL) and the mixture was refluxed for 4 hours. Additional potassium phosphate (30 mg, 0.144 mmol) and [1,1'-bis(diphenyl-phosphino)ferrocene]dichloropalladium(II) (4 mg, 0.005 mmol) were added and the reaction mixture was stirred for additional 6 hours. The solvents were evaporated and the residue was purified by flash chromatography (cyclohexane/EtOAc, gradient from 50:50 to 100:0). The product was obtained as a yellow solid (25 mg, 86 %).
¹H NMR (500 MHz, Chloroform-*d*) *δ* (ppm) 8.76 (s, 2H), 8.06 (d, *J* = 8.4 Hz, 1H), 7.94 (s, 1H), 7.88 (s, 1H), 7.62 (d, *J* = 5.3 Hz, 2H), 7.43 (d, *J* = 8.4 Hz, 1H), 3.95 (s, 3H), 2.68 (s, 3H).
¹³C NMR (126 MHz, Chloroform-*d*) *δ* (ppm) 154.28, 149.63, 148.95, 143.21, 142.80, 137.59, 130.73, 130.17, 129.47, 129.00, 125.49, 123.93, 115.38, 39.18, 15.75.
HRMS (APCI): calcd. for C₁₇H₁₅N₄S [M+H]⁺ = 307.1012, found [M+H]⁺ = 307.1010.
FTIR (neat), cm⁻¹: 3089, 2916, 2848, 1716, 1595, 1573, 1535, 1433, 1227, 1187, 1142, 995, 983, 937, 829, 811, 705, 629, 577, 564, 519, 475.

### Preparative Example 143: 2-methyl-5-(1-methyl-1H-pyrazol-3-yl)-3-(pyridin-4-yl)thieno[3,2-b]pyridine

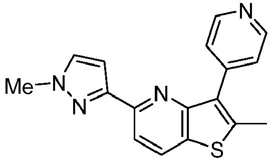

To a solution of 5-chloro-2-methyl-3-(pyridin-4-yl)thieno[3,2-*b*]pyridine **(Prep. Example 141)** (15 mg, 0.057 mmol) in dioxane (1 mL) were added potassium phosphate (37 mg, 0.172 mmol), 1-methyl-3-(4,4,5,5-tetramethyl-1,3,2-dioxaborolan-2-yl)-1*H*-pyrazole (14 mg, 0.069 mmol), [1,1'-bis(diphenylphosphino)ferrocene]dichloropalladium(II) (2 mg, 0.003 mmol) and H₂O (0.2 mL) and the mixture was refluxed for 2.5 hours. The solvents were evaporated and the residue was purified by flash chromatography (EtOAc/cyclohexane, gradient from 0:100 to 100:0) and preparative TLC (EtOAc/cyclohexane, gradient from 50:50 - 4 elutions). The product was obtained as a white solid (5 mg, 30%).
¹H NMR (500 MHz, Chloroform-*d*) *δ* (ppm) 8.77 (s, 2H), 8.12 (d, *J=* 8.4 Hz, 1H), 8.00 (d, *J=* 8.4 Hz, 1H), 7.77 (d, *J* = 5.1 Hz, 2H), 7.39 (d, *J* = 2.1 Hz, 1H), 6.86 (d, *J* = 2.2 Hz, 1H), 3.98 (s, 3H), 2.72 (s, 3H).
¹³C NMR (126 MHz, Chloroform-*d*) *δ* (ppm) 153.80, 152.17, 150.15, 147.53, 144.12, 143.72, 131.53, 130.73, 130.32, 130.16, 125.85, 115.71, 104.83, 39.21, 15.89.
HRMS (APCI): calcd. for C₁₇H₁₅N₄S [M+H]⁺ = 307.1012, found [M+H]⁺ = 307.1009.
FTIR (neat), cm⁻¹: 2953, 2921, 2852, 1632, 1600, 1557, 1435, 1397, 1335, 1155, 1112, 829, 632.

### Preparative Example 144: tert-butyl (2-bromothiophen-3-yl)carbamate (intermediate)

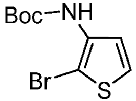

To a solution of tert-butyl thiophen-3-ylcarbamate (1.00 g, 5.02 mmol) in chloroform (12 mL) was added *N*-bromosuccinimide (893 mg, 5.02 mmol) and the reaction mixture was refluxed for 3 hours. The mixture was quenched with saturated aqueous solution of Na₂S₂O₃ (15 mL) and extracted with dichloromethane (20 mL). The organic extract was dried over MgSO₄, filtered, and the solvent was evaporated *in vacuo.* The solvents were evaporated and the residue was purified by flash chromatography (cyclohexane/EtOAc, gradient from 100:0 to 90:10). The product was obtained as a pale yellow solid (1.36 g, 97 %).
¹H NMR (500 MHz, Chloroform-*d*) *δ* (ppm) 7.56 (br s, 1 H), 7.23 (d, *J=* 5.9 Hz, 1H), 6.55 (br s, 1H), 1.52 (s, 9H).
¹³C NMR (126 MHz, Chloroform-*d*) *δ* (ppm) 152.14, 135.74, 124.78, 121.67, 81.15, 28.27.
FTIR (neat), cm⁻¹: 2978, 1727, 1579, 1478, 1381, 1367, 1230, 1156, 1072, 1000, 943, 879, 709.

### Preparative Example 145: tert-butyl (2-bromothiophen-3-yl)(methacryloyl)carbamate (intermediate)

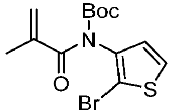

To a solution of *tert*-butyl (2-bromothiophen-3-yl)carbamate **(Prep. Example 144)** (100 mg, 0.359 mmol) in THF (1.5 mL) was added sodium hydride (60 % in oil, 17 mg, 0.431 mmol) at -10 °C. After 5 min stirring, methacryloyl chloride (42 µL, 0.431 mmol) was added to the reaction mixture. Reaction time: 40 min. The mixture was quenched with aqueous saturated aqueous solution of NaHCO₃ (2 mL) and extracted with EtOAc (10 mL ×2). The combined organic extracts were washed with H₂O (8 mL), brine (10 mL), dried over MgSO₄, filtered, and the solvent was evaporated *in vacuo.* The solvents were evaporated and the residue was purified by flash chromatography (cyclohexane/EtOAc, gradient from 100:0 to 85:15). The product was obtained as a pale yellow wax (109 mg, 88 %).
¹H NMR (500 MHz, Chloroform-*d*) *δ* (ppm) 7.28 (d, *J=* 5.8 Hz, 1H), 6.81 (d, *J=* 5.8 Hz, 1H), 5.62 (s, 1H), 5.38 (s, 1H), 2.07 (s, 3H), 1.45 (s, 9H).
¹³C NMR (126 MHz, Chloroform-*d*) *δ* (ppm) 172.56, 151.76, 142.14, 135.81, 126.70, 125.10, 119.04, 110.74, 83.93, 27.75, 26.92, 19.12.
FTIR (neat), cm⁻¹: 2979, 1739, 1692, 1382, 1370, 1279, 1247, 1151, 1112.

### Preparative Example 146: 6-methylthieno[3,2-b]pyridin-5(4H)-one (intermediate)

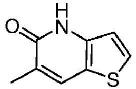

To a solution of *tert*-butyl (2-bromothiophen-3-yl)(methacryloyl) **(Prep. Example 145)** (400 mg, 1.15 mmol) in *N,N*-dimethylacetamide (5.5 mL) were added potassium acetate (451 mg, 4.60 mmol), XPhos (27 mg, 0.0575 mmol) and PdXPhosG3 (97 mg, 0.115 mmol), and the reaction mixture was stirred at 130 °C for 16 hours. The solvents were evaporated and the residue was purified by flash chromatography (EtOAc/methanol, gradient from 0:100 to 95:5). The product was obtained as an orange wax (92 mg, 48 %). ¹H NMR (500 MHz, Chloroform-*d*) *δ* (ppm) 7.55 *(d, J=* 5.3 Hz, 1H), 7.18 *(d, J=* 5.4 Hz, 1H), 6.42 (s, 1H), 2.44 (s, 3H).
¹³CNMR (126 MHz, Chloroform-*d*) *δ* (ppm) 165.27, 146.97, 141.66, 129.49, 121.21, 117.60, 115.49, 20.22. HRMS (APCI): calcd. for C₈H₈NOS [M+H]⁺ = 166.0321, found [M+H]⁺ = 166.0318.
FTIR (neat), cm⁻¹: 2922, 1633, 1540, 1459, 1428, 1375, 1205, 1120, 954, 923, 833, 768, 734, 711, 670, 590, 512, 413.

### Preparative Example 147: 5-chloro-6-methylthieno[3,2-b]pyridine (intermediate)

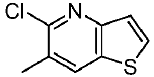

To a solution of 6-methylthieno[3,2-*b*]pyridin-5(4*H*)-one **(Prep. Example 146)** (103 mg, 0.623 mmol) in phosphoryl chloride (3.5 mL, 37.4 mmol) was added phosphorus pentachloride (194 mg, 0.934 mmol) and the reaction mixture was stirred at 70 °C for 16 hours. Additional phosphorus pentachloride (78 mg, 0.374 mmol) was added and the mixture was stirred for additional 4 hours. The reaction mixture was quenched with saturated aqueous solution of NaHCO₃ to adjust pH to 7 at -20 °C, H₂O (30 mL) was added and, the resulting mixture was extracted with EtOAc (50 mL). The combined organic extracts were dried over MgSO4, filtered, and the solvents were evaporated *in vacuo.* The solvents were evaporated and the residue was purified by flash chromatography (cyclohexane/EtOAc/methanol, gradient from 100:0:0 to 0:90:10). The product was obtained as yellow wax (57 mg, 50 %).
¹H NMR (500 MHz, Chloroform-*d*) *δ* (ppm) 7.76 (d, *J* = 5.4 Hz, 1H), 7.52 (d, *J* = 5.5 Hz, 1H), 7.12 (s, 1H), 2.58 (s, 3H).
¹³C NMR(126 MHz, Chloroform-*d*) *δ* (ppm) 155.19, 149.21, 144.27, 132.70, 131.10, 124.91, 119.42, 19.89. HRMS (APCI): calcd. for C₈H₇ClNS [M+H]⁺ = 183.9982, found [M+H]⁺ = 183.9980.
FTIR (neat), cm⁻¹: 1554, 1437, 1361, 1299, 1248, 1142, 1030, 887, 815, 774, 704, 624, 542, 498.

### Preparative Example 148: 3-bromo-5-chloro-6-methylthieno[3,2-b]pyridine (intermediate)

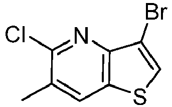

To a solution of 5-chloro-6-methylthieno[3,2-*b*]pyridine **(Prep. Example 147)** (77 mg, 0.419 mmol) in chloroform (4 mL) were added sodium bicarbonate (35 mg, 0.419 mmol), magnesium sulfate (76 mg, 0.628 mmol) and potassium hydrogenphosphate (109 mg, 0.628 mmol), and the reaction mixture was refluxed for 30 min. Additional bromine (160 µL, 3.10 mmol) was added portionwise during 48 hours. The reaction mixture was quenched with saturated aqueous solution of Na₂S₂O₃ (20 mL) and the resulting mixture was extracted with dichloromethane (2 × 20 mL). The combined organic extracts were dried over MgSO₄, filtered, and the solvent was evaporated *in vacuo.* The solvents were evaporated and the residue was purified by flash chromatography (cyclohexane/EtOAc, gradient from 100:0 to 97:3). The product was obtained as a pale yellow solid (74 mg, 67 %).
¹H NMR (500 MHz, Chloroform-*d*) *δ* (ppm) 7.77 (s, 1H), 7.19 (s, 1H), 2.58 (d, *J=* 0.7 Hz, 3H).
¹³CNMR (126 MHz, Chloroform-*d*) *δ* (ppm) 151.56, 150.57, 144.53, 131.69, 127.65, 120.71, 109.82, 19.17. HRMS (APCI): calcd. for C₈H₆BrClNS [M+H]⁺ = 261.9087, found [M+H]⁺ = 261.9090.
FTIR (neat), cm⁻¹:3069, 1554, 1481, 1426, 1376, 1357, 1297, 1145, 1077, 1045, 904, 835, 818, 777, 498.

### Preparative Example 149: 5-chloro-6-methyl-3-(pyridin-4-yl)thieno[3,2-b]pyridine (intermediate)

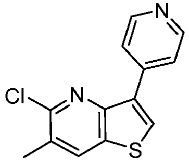

To a solution of 3-bromo-5-chloro-6-methylthieno[3,2-*b*]pyridine **(Prep. Example 148)** (50 mg, 0.190 mmol) in dioxane (3 mL) were added potassium phosphate (69 mg, 0.327 mmol), pyridin-4-ylboronic acid (28 mg, 0.228 mmol), [1,1'-bis(diphenylphosphino)ferrocene]-dichloropalladium(II) (4 mg, 0.005 mmol) and H₂O (0.6 mL) and the reaction mixture was refluxed for 2 hours. Additional potassium phosphate (35 mg, 0.164 mmol) and [1,1'-bis(diphenyl-phosphino)ferrocene]dichloropalladium(II) (0.05 equiv, 4 mg, 0.005 mmol) were added, and the reaction mixture was refluxed for additional 30 min. The solvents were evaporated and the residue was purified by flash chromatography (cyclohexane/EtOAc, gradient from 80:20 to 20:80). The product was obtained as a pale yellow solid (31 mg, 63 %).
¹H NMR (500 MHz, Chlorofonn-*d*) *δ* (ppm) 8.72 (d, *J* = 4.6 Hz, 2H), 8.05 (s, 1H), 7.98 (d, *J=* 5.5 Hz, 2H), 7.21 (s, 1H), 2.62 (s, 3H).
¹³C NMR (126 MHz, Chloroform-*d*) *δ* (ppm) 152.13, 150.02, 149.80, 144.43, 141.26, 133.96, 133.93, 129.53, 122.47, 120.24, 19.73.
HRMS (APCI): calcd. for C₁₃H₁₀ClN₂S [M+H]⁺ = 261.0248, found [M+H]⁺ = 261.0246.

### Preparative Example 150: 6-methyl-5-(1-methyl-1H-pyrazol-4-yl)-3-(pyridin-4-yl)thieno[3,2-b]pyridine

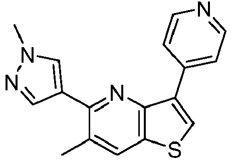

To a solution of 5-chloro-6-methyl-3-(pyridin-4-yl)thieno[3,2-*b*]pyridine (Prep. Example 149) (24 mg, 0.092 mmol) in dioxane (2 mL) were added potassium phosphate (58 mg, 0.276 mmol), 1-methyl-4-(4,4,5,5-tetramethyl-1,3,2-dioxaborolan-2-yl)-1*H*-pyrazole (23 mg, 0.110 mmol), [1,1'-bis(diphenylphosphino)ferrocene]dichloropalladium(II) (7 mg, 0.010 mmol) and H₂O (0.4 mL), and the reaction mixture was refluxed for 1 hour. The solvents were evaporated and the residue was purified by flash chromatography (cyclohexane/EtOAc/methanol, gradient from 50:50:0 to 0:98:2). The product was obtained as a pale yellow solid (24 mg, 86 %).
¹H NMR (500 MHz, Chlorofonn-*d*) *δ* (ppm) 8.73 (s, 2H), 8.12 (d, *J* = 4.2 Hz, 2H), 8.03 (d, *J* = 3.8 Hz, 2H), 8.00 (s, 1H), 7.36 (s, 1H), 4.00 (s, 3H), 2.64 (s, 3H).
¹³C NMR (126 MHz, Chloroform-*d*) *δ* (ppm) 152.76, 150.46, 149.54, 142.45, 142.03, 137.78, 134.00, 132.62, 129.08, 128.78, 123.92, 122.77, 116.46, 39.25, 19.98.
HRMS (APCI): calcd. for C₁₇H₁₅N₄ [M+H]⁺ = 307.1012, found [M+H]⁺ = 307.1012.
FTIR (neat), cm⁻¹: 3397, 3093, 2932, 1597, 1585, 1549, 1416, 1200, 998, 850, 829, 788, 703, 638, 530.

### Preparative Example 151: 6-methyl-5-(1-methyl-1H-pyrazol-3-yl)-3-(pyridin-4-yl)thieno[3,2-b]pyridine

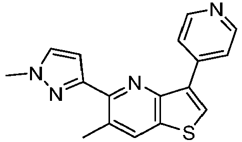

To a solution of 5-chloro-6-methyl-3-(pyridin-4-yl)thieno[3,2-*b*]pyridine **(Prep. Example 149)** (35 mg, 0.134 mmol) ) in dioxane (2 mL) were added potassium phosphate (85 mg, 0.402 mmol), 1-methyl-3-(4,4,5,5-tetramethyl-1,3,2-dioxaborolan-2-yl)-1*H*-pyrazole (33 mg, 0.161 mmol), [1,1'-bis(diphenylphosphino)ferrocene]dichloropalladium(II) (8 mg, 0.010 mmol) and H₂O (0.5 mL), and the reaction mixture was refluxed for 2 hours. The solvents were evaporated and the residue was purified twice by preparative TLC (EtOAc, 100% - 2 elutions; cyclohexane/EtOAc, 50:50 - 1 elutions; cyclohexane, 100% - 3 elutions; toluene, 100% - 2 elutions). The product was obtained as a pale yellow solid (11 mg, 27%).
¹H NMR (500 MHz, Chlorofonn-*d*) *δ* (ppm) 8.73 *(d, J=* 6.3 Hz, 2H), 8.15 (d*, J* = 6.4 Hz, 2H), 8.03 (s, 1H), 7.93 (s, 1H), 7.44 (d, *J* = 2.3 Hz, 1H), 7.03 (d, *J* = 2.3 Hz, 1H), 4.01 (s, 3H), 2.66 (s, 3H).
¹³C NMR (126 MHz, Chloroform-*d*) *δ* (ppm) 152.60, 152.38, 150.80, 149.98, 142.06, 142.04, 134.30, 133.87, 131.60, 128.34, 122.66, 116.39, 105.02, 39.24, 19.90.
HRMS (APCI): calcd. for C₁₇H₁₅N₄S [M+H]⁺ = 307.1012, found [M+H]⁺ = 307.1015.
FTIR (neat), cm⁻¹: 3671 - 2780, 1598, 1579, 1558, 1368, 1280, 1205, 829, 776, 765, 728, 638, 531.

### Preparative Example 152: N-(thiophen-3-yl)acetamide (intermediate)

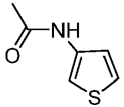

A solution of thiophen-3-amine (214 mg, 1.58 mmol) in acetic anhydride (2.9 mL) was stirred at 25 °C for 2.5 hours. The reaction mixture was quenched with H₂O (7 mL) and stirred for 15 min. Then, NaOH (6 M) was added to adjust pH to 9 and the resulting mixture was extracted with dichloromethane (3 × 20 mL). The combined organic extracts were washed with brine (10 mL), dried over MgSO₄, filtered, and the solvent was evaporated *in vacuo.* The product was obtained as a brown solid (203 mg, 91 %).
¹H NMR (500 MHz, Chloroform-*d*) *δ* (ppm) 7.54 (dd, *J* = 3.1, 1.2 Hz, 2H), 7.22 (dd, *J* = 5.1, 3.2 Hz, 1H), 6.98 (dd, *J* = 5.2, 1.3 Hz, 1H), 2.16 (s, 3H).
¹³C NMR (126 MHz, Chloroform-*d*) *δ* (ppm) 167.44, 135.53, 124.49, 120.91, 110.29, 23.88.
HRMS (APCI): calcd. for C₆H₈NOS [M+H]⁺ = 142.0321, found [M+H]⁺ = 142.0322.
FTIR (neat), cm⁻¹: 3267, 3221, 1654, 1572, 1544, 1375, 1362, 1294, 1151, 839, 772, 750, 725, 597.

### Preparative Example 153: 7-methylthieno[3,2-b]pyridin-5(4H)-one (intermediate)

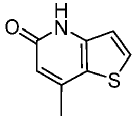

To a solution of *N-*(thiophen-3-yl)acetamide **(Prep. Example 152)** (450 mg, 3.19 mmol) in isopropanol (6 mL) were added ethyl but-2-ynoate (0.56 mL, 4.78 mmol), dichloro(*p*-cymene)ruthenium (II) dimer (97 mg, 0.160 mmol), silver hexafluoroantimoniate (V) (279 mg, 0.638 mmol) and pivalic acid (3.26 g, 31.9 mmol) in a sealed tube and the reaction mixture was stirred at 130 °C for 22 hours. Additional pivalic acid (2.15 g, 21.0 mmol) and the reaction mixture was stirred for additional 24 hours. Then, additional dichloro(p-cymene)ruthenium (II) dimer (97 mg, 0.160 mmol), silver hexafluoroantimoniate (V) (279 mg, 0.638 mmol) and pivalic acid (3.26 g, 31.9 mmol) were added and the reaction was stirred for additional 72 hours. The solvents were evaporated and the residue was purified twice by flash chromatography (dichloromethane/methanol, gradient from 100:0 to 80:20). The product was obtained as a brown solid (250 mg, 48 %).
¹H NMR (500 MHz, Methanol-*d₄*) *δ* (ppm) 7.80 (d, *J* = 5.4 Hz, 1H), 7.11 (d, *J* = 5.4 Hz, 1H), 6.49 - 6.21 (m, 1H), 2.45 (d, *J=* 1.0 Hz, 3H).
¹³C NMR (126 MHz, Methanol-*d₄*) *δ* (ppm) 165.85, 149.07, 142.89, 131.78, 122.51, 118.10, 115.88, 20.06.
HRMS (APCI): calcd. for C₈H₈NOS [M+H]⁺ = 166.0321, found [M+H]⁺ = 166.0319.
FTIR (neat), cm⁻¹: 2921, 1724, 1642, 1572, 1542, 1457, 1432, 1375, 1212, 838, 659.

### Preparative Example 154: 5-chloro-7-methylthieno[3,2-b]pyridine (intermediate)

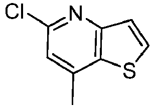

A solution of 7-methylthieno[3,2-*b*]pyridin-5(4*H*)-one **(Prep. Example 153)** (200 mg, 1.21 mmol) in phosphorus(V) oxychloride (7 mL, 72.3 mmol) was stirred at 70 °C for 3 hours. Then, triethylamine (0.26 mL) was added and the reaction mixture was stirred for 16 hours. The reaction mixture was quenched with NaOH (6 M) to adjust pH to 7 at -30 °C and the resulting mixture was extracted with EtOAc (2 × 30 mL) and washed with H₂O (30 mL). The combined organic extracts were washed with brine (2 × 15 mL), dried over MgSO₄, filtered, and the solvent was evaporated *in vacuo.* The solvents were evaporated and the residue was purified by flash chromatography (cyclohexane/EtOAc, gradient from 100:0 to 90:10). The product was obtained as a yellow wax (107 mg, 48 %).
¹H NMR (500 MHz, Chlorofonn-*d*) *δ* (ppm) 7.75 *(d, J=* 5.5 Hz, 1H), 7.51 *(d, J=* 5.5 Hz, 1H), 7.12 (s, 1H), 2.58 (d, *J* = 0.6 Hz, 3H).
¹³CNMR (126 MHz, Chloroform-*d*) *δ* (ppm) 155.38, 149.35, 144.11, 132.64, 130.95, 125.04, 119.41, 19.88.
HRMS (APCI): calcd. for C₈H₇ClNS [M+H]⁺ = 183.9982, found [M+H]⁺ = 183.9984.
FTIR (neat), cm⁻¹: 1554, 1437, 1361, 1299, 1248, 1142, 1030, 887, 815, 774, 704, 624, 542, 498.

### Preparative Example 155: 3-bromo-5-chloro-7-methylthieno[3,2-b]pyridine (intermediate)

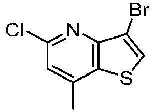

To a solution of 5-chloro-7-methylthieno[3,2-*b*]pyridine **(Prep. Example 154)** (76 mg, 0.413 mmol) in chloroform (3 mL) were added sodium bicarbonate (35 mg, 0.413 mmol), magnesium sulfate (75 mg, 0.620 mmol) and potassium hydrogenphosphate (108 mg, 0.620 mmol) and the mixture was refluxed for 30 min. Then, bromine (39 µL, 0.758 mmol) was added and the reaction mixture was stirred for 16 hours. Additional bromine (100 µL, 1.95 mmol) was added and the mixture was stirred for additional 53 hours. The reaction mixture was quenched with saturated aqueous solution of Na₂S₂O₃ (20 mL) and the resulting mixture was extracted with dichloromethane (4 × 15 mL). The combined organic extracts were dried over MgSO₄, filtered, and the solvent was evaporated *in vacuo.* The solvents were evaporated and the residue was purified by flash chromatography (cyclohexane/EtOAc, gradient from 100:0 to 95:5). The product was obtained as an impure pale yellow solid (61 mg, 56 %).
¹H NMR (500 MHz, Chlorofonn-*d*) *δ* (ppm) 7.77 (s, 1H), 7.20 (s, 1H), 2.58 (br s, 3H).
¹³CNMR (126 MHz, Chloroform-*d*) *δ* (ppm) 151.58, 150.59, 144.53, 131.70, 127.65, 120.72, 109.84, 19.18.
HRMS (APCI): calcd. for C₈H₆BrClNS= 261.9087, found [M+H]⁺ = 261.9089.
FTIR (neat), cm⁻¹: 3074, 2923, 1557, 1378, 1359, 1299, 1150, 906, 839, 818, 781.

### Preparative Example 156: 5-chloro-7-methyl-3-(pyridin-4-yl)thieno[3,2-b]pyridine (intermediate)

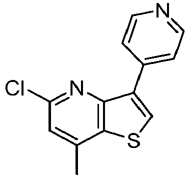

To a solution of 3-bromo-5-chloro-7-methylthieno[3,2-*b*]pyridine **(Prep. Example 155)** (58 mg, 0.221 mmol) in dioxane (6 mL) were added potassium phosphate (141 mg, 0.630 mmol), pyridin-4-ylboronic acid (33 mg, 0.265 mmol), [1,1'-bis(diphenylphosphino)ferrocene]-dichloropalladium(II) (8 mg, 0.011 mmol) and H₂O (0.5 mL) and the mixture was refluxed for 5 hours. Additional potassium phosphate (141 mg, 0.063 mmol) and [1,1'-bis(diphenylphosphino)ferrocene]dichloropalladium(II) (8 mg, 0.011 mmol) were added, and the reaction mixture was refluxed for additional 3 hours. The solvents were evaporated and the residue was purified by flash chromatography (cyclohexane/EtOAc, gradient from 60:40 to 0:100). The product was obtained as a pale yellow solid (22 mg, 39 %).
¹H NMR (500 MHz, Chloroform-*d*) *δ* (ppm) 8.72 (dd, *J* = 4.7, 1.3 Hz, 2H), 8.06 (s, 1H), 7.98 (d, *J* = 6.1 Hz, 2H), 7.21 (s, 1H), 2.62 (d, *J=* 0.7 Hz, 3H).
¹³C NMR (126 MHz, Chloroform-*d*) *δ* (ppm) 152.14, 149.97, 149.81, 144.43, 141.32, 133.94, 129.55, 122.49, 120.25, 19.74.
HRMS (APCI): calcd. for C₁₃H₁₀ClN₂S [M+H]⁺ = 261.0248, found [M+H]⁺ = 261.0246.
FTIR (neat), cm⁻¹: 3065, 2918, 1601, 1558, 1435, 1379, 1362, 1302, 1212, 1144, 992, 853, 833, 811, 641, 563, 532.

### Preparative Example 157: 7-methyl-5-(1-methyl-1H-pyrazol-4-yl)-3-(pyridin-4-yl)thieno[3,2-b]pyridine

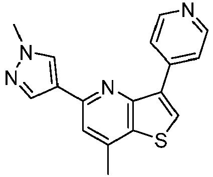

To a solution of 5-chloro-7-methyl-3-(pyridin-4-yl)thieno[3,2-*b*]pyridine **(Prep. Example 156)** (20 mg, 0.077 mmol) in dioxane (1.5 mL) were added potassium phosphate (49 mg, 0.231 mmol), 1-methyl-4-(4,4,5,5-tetramethyl-1,3,2-dioxaborolan-2-yl)-1*H*-pyrazole (19 mg, 0.092 mmol), [1,1'-bis(diphenylphosphino)ferrocene]dichloropalladium(II) (3 mg, 0.004 mmol) and H₂O (0.3 mL), and the mixture was refluxed for 2 hours. The solvents were evaporated and the residue was purified by flash chromatography (EtOAc/methanol, gradient from 100:0 to 90:10). The product was obtained as a pale yellow solid (18 mg, 77 %).
¹H NMR (500 MHz, Chlorofonn-*d*) *δ* (ppm) 8.74 (s, 2H), 8.12 (d, *J* = 4.5 Hz, 2H), 8.03 (d, *J=* 4.7 Hz, 2H), 8.00 (s, 1H), 7.36 (s, 1H), 4.00 (s, 3H), 2.64 (s, 3H).
¹³C NMR (126 MHz, Chloroform-*d*) *δ* (ppm) 152.78, 150.42, 149.82, 142.15, 141.98, 137.77, 134.10, 132.60, 129.06, 128.58, 123.94, 122.71, 116.40, 39.22, 19.95.
HRMS (APCI): calcd. for C₁₇H₁₅N₄S [M+H]⁺ = 307.1012, found [M+H]⁺ = 307.1009.
FTIR (neat), cm⁻¹: 3081, 1585, 1556, 1517, 1412, 1197, 987, 845, 811, 698, 648, 529.

### Preparative Example 158: 7-methyl-5-(1-methyl-1H-pyrazol-3-yl)-3-(pyridin-4-yl)thieno[3,2-b]pyridine

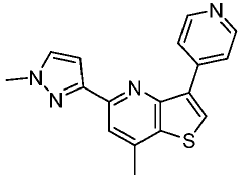

To a solution of 5-chloro-7-methyl-3-(pyridin-4-yl)thieno[3,2-*b*]pyridine **(Prep. Example 156)** (32 mg, 0.122 mmol) in dioxane (2.5 mL) were added potassium phosphate (78 mg, 0.366 mmol), 1-methyl-3-(4,4,5,5-tetramethyl-1,3,2-dioxaborolan-2-yl)-1*H*-pyrazole (28 mg, 0.134 mmol), [1,1'-bis(diphenylphosphino)ferrocene]dichloropalladium(II) (5 mg, 0.006 mmol) and H₂O (0.6 mL), and the mixture was refluxed for 2 hours. Additional potassium phosphate (38 mg, 0.183 mmol) and [1,1'-bis(diphenyl-phosphino)ferrocene]dichloro-palladium(II) (5 mg, 0.006 mmol) were added, and the reaction mixture was refluxed for additional 45 min. The solvents were evaporated and the residue was purified by flash chromatography (cyclohexane/EtOAc, gradient from 80:20 to 10:90) and reverse phase preparative TLC (methanol/H₂O, 98:2). The product was obtained as a white solid (10 mg, 28 %).
¹H NMR (500 MHz, Chloroform-*d*) *δ* (ppm) 8.73 (dd, *J* = 4.6, 1.4 Hz, 2H), 8.15 (dd, *J* = 4.6, 1.6 Hz, 2H), 8.03 (s, 1H), 7.93 (s, 1H), 7.44 (d, *J* = 2.2 Hz, 1H), 7.03 (d, *J* = 2.2 Hz, 1H), 4.01 (s, 3H), 2.66 (s, 3H).
¹³C NMR (126 MHz, Chloroform-*d*) *δ* (ppm) 152.58, 152.36, 150.78, 149.95, 142.06, 142.03, 134.26, 133.86, 131.60, 128.35, 122.65, 116.37, 105.01, 39.25, 19.91.
HRMS (APCI): calcd. for C₁₇H₁₅N₄S [M+H]⁺ = 307.1012, found [M+H]⁺ = 307.1015.
FTIR (neat), cm⁻¹: 3413, 3097, 2924, 2853, 1632, 1598, 1580, 1521, 1367, 1280, 1206, 829, 777, 765, 702, 638, 530.

### Preparative Example 159: 5,7-dichlorothieno[3,2-b]pyridine (intermediate)

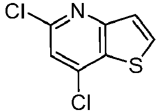

To a solution of thiophen-3-amine hydrochloride (1.32 g, 9.67 mmol) in oxalyl chloride (12 mL, 128 mmol) was added malonic acid (1.00 g, 9.67 mmol) and the mixture was stirred at 100 °C in a sealed tube for 16 hours. The reaction mixture was dissolved in EtOAc (40 mL) at -60 °C, followed by addition of H₂O (30 mL). Then, saturated aqueous solution of NaOH was added dropwise to adjust pH to 7. The combined organic extracts were washed with brine (30 mL), dried over MgSO₄, filtered, and the solvent was evaporated *in vacuo.* The solvents were evaporated and the residue was purified by flash chromatography (cyclohexane/EtOAc, 50:50). The product was obtained as a brown solid (852 mg, 43 %).
¹H NMR (500 MHz, Chlorofonn-*d*) *δ* (ppm) 7.84 (d, *J=* 5.5 Hz, 1H), 7.53 (d, *J=* 5.5 Hz, 1H), 7.34 (s, 1H).
¹³C NMR (126 MHz, Chloroform-*d*) *δ* (ppm) 156.30, 149.24, 139.58, 132.82, 131.71, 125.20, 118.82.
HRMS (APCI): calcd. C₇H₄Cl₂NS [M+H]⁺ = 203.9436, found [M+H]⁺ = 203.9436.
FTIR (neat), cm⁻¹: 3091, 3068, 1557, 1526, 1484, 1353, 1220, 1142, 837, 815, 770, 701.

### Preparative Example 160: 3-bromo-5,7-dichlorothieno[3,2-b]pyridine (intermediate)

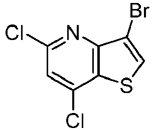

To a solution of 5,7-dichlorothieno[3,2-*b*]pyridine **(Prep. Example 159)** (300 mg, 1.47 mmol) in chloroform (7 mL) were added sodium bicarbonate (123 mg, 1.47 mmol), magnesium sulfate (265 mg, 2.21 mmol) and potassium hydrogenphosphate (314 mg, 2.05 mmol), and the reaction mixture was refluxed for 5 min, followed by the addition of bromine (140 µL, 2.72 mmol). After 3 hours, additional bromine (50 µL, 0.975 mmol) was added, and the mixture was refluxed for additional 24 hours. The reaction mixture was quenched with saturated aqueous solution of Na₂S₂O₃ (30 mL) and the resulting mixture was extracted with dichloromethane (2 × 25 mL). The combined organic extracts were dried over MgSO₄, filtered, and the solvent was evaporated *in vacuo.* The residue was purified by flash chromatography (cyclohexane/EtOAc, 10:90). The product was obtained as a white solid (343 mg, 82 %).
¹H NMR (500 MHz, Chlorofonn-*d*) *δ* (ppm) 7.86 (s, 1H), 7.43 (s, 1H).
¹³C NMR (126 MHz, Chloroform-*d*) *δ* (ppm) 152.60, 150.48, 139.83, 130.80, 129.51, 120.03, 109.90.
HRMS (APCI): calcd. for C₇H₃BrCl₂NS [M+H]⁺ = 281.8541, found [M+H]⁺ = 281.8542.
FTIR (neat), cm⁻¹: 3074, 1556, 1517, 1477, 1344, 1260, 1143, 1053, 845, 814, 770, 550.

### Preparative Example 161: 5,7-dichloro-3-(pyridin-4-yl)thieno[3,2-b]pyridine (intermediate)

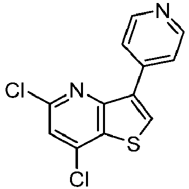

To a solution of 5-chloro-3-(pyridin-4-yl)thieno[3,2-*b*]pyridine **(Prep. Example 160)** (100 mg, 0.182 mmol) in dioxane (4 mL) were added potassium phosphate (224 mg, 1.06 mmol), pyridin-4-ylboronic acid (48 mg, 0.388 mmol), [1,1'-bis(diphenylphosphino)-ferrocene]dichloropalladium(II) (21 mg, 0.028 mmol) and H₂O (1 mL), and the reactiom mixture was refluxed for 4 hours. The solvents were evaporated and the residue was purified by flash chromatography (cyclohexane/EtOAc, gradient from 50:50 to 20:80). The product was obtained as a white solid (57 mg, 58 %).
¹H NMR (500 MHz, Chlorofonn-*d*) *δ* (ppm) 8.74 (dd, *J* = 4.7, 1.5 Hz, 2H), 8.15 (s, 1H), 7.98 (dd, *J* = 4.7, 1.6 Hz, 2H), 7.45 (s, 1H).
¹³C NMR (126 MHz, Chloroform-*d*) *δ* (ppm) 153.03, 149.78, 149.73, 141.10, 139.91, 134.14, 133.01, 131.33, 122.53, 119.67.
HRMS (APCI): calcd. for C₁₂H₇Cl₂N₂S [M+H]⁺ = 280.9702, found [M+H]⁺ = 280.9705.
FTIR (neat), cm⁻¹: 3030, 1601, 1560, 1513, 1354, 1270, 1143, 845, 815, 776, 717, 634, 557, 535.

### Preparative Example 162: 7-chloro-5-(1-methyl-1H-pyrazol-4-yl)-3-(pyridin-4-yl)thieno[3,2-b]pyridine

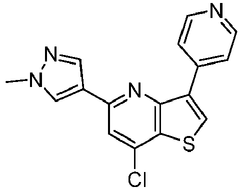

To a solution of 5,7-dichloro-3-(pyridin-4-yl)thieno[3,2-b]pyridine **(Prep. Example 161)** (48 mg, 0.170 mmol) in dioxane (3 mL) were added potassium phosphate (108 mg, 0.510 mmol), 1-methyl-4-(4,4,5,5-tetramethyl-1,3,2-dioxaborolan-2-yl)-1*H*-pyrazole (39 mg, 0.187 mmol), [1,1'-bis(diphenylphosphino)-ferrocene]dichloropalladium(II) (10 mg, 0.014 mmol) and H₂O (0.6 mL), and the reaction mixture was refluxed for 3 hours. The solvents were evaporated and the residue was purified by flash chromatography (cyclohexane/EtOAc, gradient from 50:50 to 0:100). The product was obtained as a white solid (30 mg, 54 %).
¹H NMR (500 MHz, Chloroform-*d*) *δ* (ppm) 8.76 (s, 2H), 8.10 (s, 3H), 8.03 (s, 1H), 7.99 (s, 1H), 7.55 (s, 1H), 4.01 (s, 3H).
¹³C NMR (126 MHz, Chloroform-*d*) *δ* (ppm) 154.02, 151.37, 149.86, 141.73, 138.69, 137.91, 134.38, 131.23, 130.15, 129.35, 123.02, 122.78, 115.65, 39.33.
HRMS (APCI): calcd. for C₁₆H₁₂ClN₄S [M+H]⁺ = 327.0466, found [M+H]⁺ = 327.0464.
FTIR (neat), cm⁻¹: 3424, 3038, 2940, 1598, 1574, 1526, 1181, 1008, 992, 817, 699, 627, 535.

### Preparative Example 163: 7-chloro-5-(1-methyl-1H-pyrazol-3-yl)-3-(pyridin-4-yl)thieno[3,2-b]pyridine

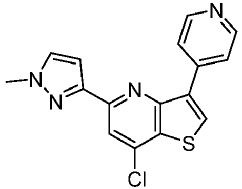

To a solution of 5,7-dichloro-3-(pyridin-4-yl)thieno[3,2-b]pyridine **(Prep. Example 161)** (45 mg, 0.160 mmol) in dioxane (3 mL) were added potassium phosphate (102 mg, 0.480 mmol), 1-methyl-3-(4,4,5,5-tetramethyl-1,3,2-dioxaborolan-2-yl)-1*H*-pyrazole (37 mg, 0.176 mmol), [1,1'-bis(diphenyl-phosphino)ferrocene]dichloropalladium(II) (10 mg, 0.013 mmol) and H₂O (0.6 mL), and the reaction mixture was refluxed for 2.5 hours. The solvents were evaporated and the residue was purified by flash chromatography (cyclohexane/EtOAc/methanol, gradient from 30:70:0 to 0:95:5). The product was obtained as a white solid (29 mg, 56 %).
¹H NMR (500 MHz, Chlorofonn-*d*) *δ* (ppm) 8.76 (s, 2H), 8.24 (s, 2H), 8.17 (s, 1H), 8.16 (s, 1H), 7.46 (d, *J* = 2.0 Hz, 1H), 7.00 (d, *J=* 2.1 Hz, 1H), 4.01 (s, 3H).
¹³C NMR (126 MHz, Chloroform-*d*) *δ* (ppm) 153.58, 152.00, 151.07, 148.34, 143.16, 138.83, 133.81, 132.53, 131.85, 130.75, 123.02, 116.08, 105.25, 39.35.
HRMS (APCI): calcd. for C₁₆H₁₂ClN₄S [M+H]⁺ = 327.0466, found [M+H]⁺ = 327.0468.
FTIR (neat), cm⁻¹: 3418, 2917, 2849, 1603, 1569, 1545, 1518, 1478, 1434, 1366, 1269, 1057, 811, 767, 760, 630, 533.

### Preparative Example 164: 5-chloro-7-methoxy-3-(pyridin-4-yl)thieno[3,2-b]pyridine (intermediate)

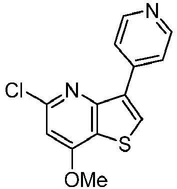

To a solution of 5,7-dichloro-3-(pyridin-4-yl)thieno[3,2-b]pyridine **(Prep. Example 161)** (100 mg, 0.355 mmol) in methanol (3 mL) were added sodium methoxide (29 mg, 0.532 mmol) and 15-crown-5 (105 µL, 0.532 mmol) and the mixture was refluxed for 16 hours. Additional sodium methoxide (58 mg, 1.06 mmol) and 15-crown-5 (210 µL, 1.06 mmol) were added and the reaction mixture was refluxed for additional 16 hours. The solvents were evaporated and the residue was purified by flash chromatography (cyclohexane/EtOAc, gradient from 30:70 to 50:50). The product was obtained as a partly impure white solid (39 mg, 40 %, 70 % purity), which was used without further purification in the next step.
¹H NMR (500 MHz, Chloroform-*d*) *δ* (ppm) 8.70 - 8.67 (m, 2H), 7.97 (s, 1H), 7.96 - 7.94 (m, 2H), 6.79 (s, 1H), 4.06 (s, 3H).
¹³C NMR (126 MHz, Chloroform-*d*) *δ* (ppm) 162.18, 153.19, 151.14, 150.42, 149.16, 142.03, 130.10, 122.62, 121.40, 101.54, 56.32.
HRMS (APCI): calcd. for C₁₃H₁₀ClN₂OS [M+H]⁺ = 277.0197, found [M+H]⁺ = 277.0196.

### Preparative Example 165: 7-methoxy-5-(1-methyl-1H-pyrazol-3-yl)-3-(pyridin-4-yl)thieno[3,2-b]pyridine

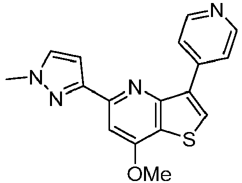

To a solution of 5-chloro-7-methoxy-3-(pyridin-4-yl)thieno[3,2-*b*]pyridine **(Prep. Example 164)** (34 mg, 0.123 mmol) in dioxane (3 mL) were added potassium phosphate (78 mg, 0.369 mmol), 1-methyl-3-(4,4,5,5-tetramethyl-1,3,2-dioxaborolan-2-yl)-1*H*-pyrazole (30 mg, 0.147 mmol), [1,1'-bis(diphenylphosphino)ferrocene]dichloropalladium(II) (8 mg, 0.009 mmol) and H₂O (0.6 mL), and the reaction mixture was refluxed for 16 hours. The solvents were evaporated and the residue was purified by three preparative TLC (cyclohexane/EtOAc, 0:100 - 1 elution and 50:50 - 2 elutions; cyclohexane/EtOAc, 50:50 - 1 elution, 80:20 - 1 elution, 100:0 - 1 elution; toluene - 2 elutions; toluene methanol, 95:5 - 2 elutions). The product was obtained as a white solid (21 mg, 54 %).
¹H NMR (500 MHz, Chloroform-*d*) *δ* (ppm) 8.72 (d, *J* = 5.7 Hz, 2H), 8.35 (d, *J* = 5.7 Hz, 2H), 8.08 (s, 1H), 7.61 (s, 1H), 7.46 (d, *J* = 2.2 Hz, 1H), 7.02 (d, *J* = 2.2 Hz, 1H), 4.17 (s, 3H), 4.02 (s, 3H).
¹³C NMR (126 MHz, Chloroform-*d*) *δ* (ppm) 161.55, 154.17, 153.26, 152.29, 147.39, 132.97, 131.76, 130.09, 123.05, 122.71, 105.18, 97.36, 56.02, 39.28.
HRMS (APCI): calcd. for C₁₇H₁₄N₄OS [M+H]⁺ = 323.0961, found [M+H]⁺ = 323.0958.
FTIR (neat), cm⁻¹: 3387, 3093, 2937, 1598, 1564, 1496, 1374, 1325, 1293, 1212, 1096, 848, 830, 771, 729.

### II. Biological assays

The compounds were profiled in the radiometric assays (KinaseProfiler, Eurofins).

Briefly, kinases were diluted in the buffer shown below prior to addition to the reaction mix:
CDKL3, TAF1L: 20 mM Tris/HCl pH 8.5, 0.2 mM EDTA, 0.1% β-mercaptoethanol, 0.01% Brij-35, 5% glycerol, 1 mg/ml BSA;
Haspin: 15 mM MOPS, 0.75 mM EDTA, 0.0075% Brij-35, 3.75% Glycerol, 150 mM NaCl, 0.1% β-mercaptoethanol, 1 mg/mLBSA;
CDKL4, CLK2, PIM1, TRB2: PS, 1 mM EDTA, 0.01% Brij-35, 5% Glycerol, 0.1% β-mercaptoethanol, 1 mg/mL BSA.

All compounds were prepared to 50x final assay concentration in 100% DMSO. This working stock of the compound was added to the assay well as the first component in the reaction, followed by the remaining components as detailed in the general assay protocols below. There was no pre-incubation step between the compound and the kinase prior to initiation of the reaction.

CDKL3(h) was incubated with 8 mM MOPS pH 7.0, 0.2 mM EDTA, 250 µM RRRFRPASPLRGPPK (SEQ ID NO. 1), 10 mM magnesium acetate and γ-³³P-ATP (specific activity and concentration as required). The reaction was initiated by the addition of the Mg/ATP mix. After incubation for 120 minutes at room temperature, the reaction was stopped by the addition of phosphoric acid to a concentration of 0.5%. An aliquot of the stopped reaction was spotted onto a filter and washed four times for 4 minutes in 0.425% phosphoric acid and once in methanol prior to drying and scintillation counting.

CDKL4(h) was incubated with 8 mM MOPS pH 7.0, 0.2 mM EDTA, 250 µM RRRFRPASPLRGPPK (SEQ ID NO. 1), 10 mM magnesium acetate and γ-³³P-ATP (specific activity and concentration as required). The reaction was initiated by the addition of the Mg/ATP mix. After incubation for 120 minutes at room temperature, the reaction was stopped by the addition of phosphoric acid to a concentration of 0.5%. An aliquot of the stopped reaction was spotted onto a filter and washed four times for 4 minutes in 0.425% phosphoric acid and once in methanol prior to drying and scintillation counting.

CLK2 (h) was incubated with 8 mM MOPS pH 7.0, 0.2 mM EDTA, 20 µM YRRAAVPPSPSLSRHSSPHQS(p)EDEEE (SEQ ID NO. 2), 10 mM magnesium acetate and γ-³³P-ATP (specific activity and concentration as required). The reaction was initiated by the addition of the Mg/ATP mix. After incubation for 40 minutes at room temperature, the reaction was stopped by the addition of phosphoric acid to a concentration of 0.5%. An aliquot of the reaction was then spotted onto a filter and washed four times for 4 minutes in 0.425% phosphoric acid and once in methanol prior to drying and scintillation counting.

DYRK2 (h) was incubated with 8 mM MOPS pH 7.0, 0.2 mM EDTA, 2 mg/mL casein, 10 mM magnesium acetate and γ-³³P-ATP (specific activity and concentration as required). The reaction was initiated by the addition of the Mg/ATP mix. After incubation for 40 minutes at room temperature, the reaction was stopped by the addition of phosphoric acid to a concentration of 0.5%. An aliquot of the reaction was then spotted onto a filter and washed four times for 4 minutes in 0.425% phosphoric acid and once in methanol prior to drying and scintillation counting.

Haspin (h) was incubated with 8 mM MOPS pH 7.0, 0.2 mM EDTA, 500 µM RARTLSFAEPG (SEQ ID NO. 3), 10 mM magnesium acetate and γ-³³P-ATP (specific activity and concentration as required). The reaction was initiated by the addition of the Mg/ATP mix. After incubation for 40 minutes at room temperature, the reaction was stopped by the addition of phosphoric acid to a concentration of 0.5%. An aliquot of the reaction was then spotted onto a filter and washed four times for 4 minutes in 0.425% phosphoric acid and once in methanol prior to drying and scintillation counting.

PIM1 (h) was incubated with 8 mM MOPS pH 7.0, 0.2 mM EDTA, 100 µM KKRNRTLTV (SEQ ID NO. 4), 10 mM magnesium acetate and γ-³³P-ATP (specific activity and concentration as required). The reaction was initiated by the addition of the Mg/ATP mix. After incubation for 40 minutes at room temperature, the reaction was stopped by the addition of phosphoric acid to a concentration of 0.5%. An aliquot of the reaction was then spotted onto a filter and washed four times for 4 minutes in 0.425% phosphoric acid and once in methanol prior to drying and scintillation counting.

TAF1L(h) was incubated with 20 mM Tris/HCl pH 8.5, 0.2 mM EDTA, 250 µM RRRFRPASPLRGPPK (SEQ ID NO. 1), 10 mM magnesium acetate and γ-³³P-ATP (specific activity and concentration as required). The reaction was initiated by the addition of the Mg/ATP mix. After incubation for 120 minutes at room temperature, the reaction was stopped by the addition of phosphoric acid to a concentration of 0.5%. An aliquot of the stopped reaction was spotted onto a filter and washed four times for 4 minutes in 0.425% phosphoric acid and once in methanol prior to drying and scintillation counting.

TRB2(h) was incubated with 8 mM MOPS pH 7.0, 0.2 mM EDTA, 250 µM RRRFRPASPLRGPPK (SEQ ID NO. 1), 10 mM magnesium acetate and γ-³³P-ATP (specific activity and concentration as required). The reaction was initiated by the addition of the Mg/ATP mix. After incubation for 120 minutes at room temperature, the reaction was stopped by the addition of phosphoric acid to a concentration of 0.5%. An aliquot of the stopped reaction was spotted onto a filter and washed four times for 4 minutes in 0.425% phosphoric acid and once in methanol prior to drying and scintillation counting.

### Data Analysis:

Kinase activity data were expressed as the percent remaining kinase activity in tested samples compared to the vehicle (DMSO) reactions. The compounds were tested at the following concentrations: 1 nM, 3 nM, 10 nM, 30 nM, 100 nM, 300 nM, 1000 nM, 3000 nM, and 10 000 nM; IC₅₀ values and curve fits were then obtained.

Table 1 summarizes the inhibitory activities (IC₅₀ values) of indicated compounds tested in the *in vitro* kinase assays described above.
A: 20 nM < IC₅₀ < 99 nM
B: 100 nM < IC₅₀ < 499 nM
C: 500 nM < IC₅₀ < 2999 nM
D: 3000 nM < IC₅₀ < 10000 nM

**Table 1**

| **compound** | **kinase** | | | | | | | |
|---|---|---|---|---|---|---|---|---|
| | **CDKL3** | **CDKL4** | **CLK2** | **DYRK2** | **Haspin** | **PIM1** | **TAF1L** | **TRB2** |
| Preparative Example 2: 5-(1-methyl-1H-pyrazol-4-yl)-3-phenylthieno[3,2-*b*]pyridine | B | B | D | C | B | C | B | C |
| Preparative Example 4: 4-(5-(1-methyl-1*H*-pyrazol-4-yl)thieno[3,2-*b*]pyridin-3-yl)benzo-nitrile | | | | | B | | | |
| Preparative Example 6: 3-(4-fluorophenyl)-5-(1-methyl-1*H*-pyrazol-4-yl)-thieno[3,2-*b*]pyridine | | | | | B | | | |
| Preparative Example 9: 5-(1-methyl-1*H*-pyrazol-4-yl)-3-(1,2,3,6-tetrahydro-pyri-din-4-yl)-thieno[3,2-*b*]pyridine | C | C | D | D | C | C | C | D |
| Preparative Example 11:5-(1-methyl-1H-pyrazol-4-yl)-3-(pyridin-3-yl)thieno[3,2-*b*]pyridine | B | C | D | C | C | C | C | C |
| Preparative Example 13: 5-(1-methyl-1H-pyrazol-4-yl)-3-(pyridin-4-yl)thieno[3,2-*b*]pyridine | B | B | D | C | A | B | B | C |
| Preparative Example 15: 5-(1-methyl-1H-pyrazol-4-yl)-3-(2-methylpyridin-4-yl)-thieno[3,2-*b*]pyridine | B | B | C | C | B | B | B | B |
| Preparative Example 17: 3-(2-methoxy- pyridin-4-yl)-5-(1-methyl-1*H*-pyrazol-4-yl)thieno[3,2-*b*]pyridine | B | B | C | B | B | B | B | C |
| Preparative Example 18: 4-(5-(1-methyl-1*H*-pyrazol-4-yl)thieno[3,2-*b*]-pyridin-3-yl)pyridin-2-ol | | | | | B | | | |
| Preparative Example 20: 4-(5-(1-methyl-1*H*-pyrazol-4-yl)thieno[3,2-*b*]-pyridin-3-yl)picolino-nitrile | B | B | D | C | B | C | B | B |
| Preparative Example 22: 3-(2-fluoro-pyridin-4-yl)-5-(1-methyl-1*H*-pyrazol-4-yl)thieno[3,2-*b*]pyridine | B | B | D | C | B | B | B | B |
| Preparative Example 24: *tert-butyl* (4-(5-(1-methyl-1*H*-pyrazol-4-yl)thieno-[3,2-*b*]pyridin-3-yl)pyridin-2-yl)carbamate | | | | | B | | | |
| Preparative Example 25: 4-(5-(1-methyl-1*H*-pyrazol-4-yl)thieno[3,2-*b*]-pyridin-3-yl)pyridin-2-amine | | | | | A | | | |
| Preparative Example 27: (4-(5-(1-methyl-1*H*-pyrazol-4-yl)thieno[3,2-*b*]-pyridin-3-yl)pyridin-2-yl)methanamine | | | | | D | | | |
| Preparative Example 28: (4-(5-(1-methyl-1*H*-pyrazol-4-yl)thieno[3,2-*b*]pyridin-3-yl)pyridin-2-yl)methanamine | C | C | D | B | A | B | C | C |
| Preparative Example 30: 3-(2,6-dime-thylpyridin-4-yl)-5-(1-methyl-1*H-*pyrazol-4-yl)thieno-[3,2-*b*]pyridine | C | B | D | C | B | B | B | C |
| Preparative Example 32: 5-(1-methyl-1H-pyrazol-4-yl)-3-(3-methylpyridin-4-yl)thieno[3,2-*b*]pyridine | C | C | D | D | D | C | C | D |
| Preparative Example 34: 4-(5-(1-methyl-1*H*-pyrazol-4-yl)thieno[3,2-*b*]-pyridin-3-yl)nicotino-nitrile | C | D | D | C | C | C | C | D |
| Preparative Example 36: 3-(3-fluoro-pyridin-4-yl)-5-(1-methyl-1*H*-pyrazol-4-yl)thieno[3,2-*b*]pyridine | B | B | C | C | B | B | B | B |
| Preparative Example 38: 5-(1-methyl-1H-pyrazol-4-yl)-3-(pyrimidin-5-yl)-thieno-[3,2-*b*]pyridine | | | | | B | | | |
| Preparative Example 40: 5-(1-methyl-1*H*-pyrazol-4-yl)-3-(pyridazin-4-yl)-thieno-[3,2-*b*]-pyridine | C | C | D | C | B | C | B | C |
| Preparative Example 42: 4-(5-(5-(1-methyl-1*H*-pyrazol-4-yl)thieno[3,2-*b*]-pyridin-3-yl)pyridin-2-yl)morpholine | | | | | C | | | |
| Preparative Example 44: 5-(1-methyl-1*H*-pyrazol-4-yl)-3-(1-tosyl-1*H-*pyrrolo[2,3-*b*]pyridin-4-yl)thieno[3,2-*b*]pyridine | | | | | D | | | |
| Preparative Example 45: 5-(1-methyl-1*H*-pyrazol-4-yl)-3-(1*H*-pyrrolo[2,3-*b*]-pyridin-4-yl)thieno[3,2-*b*]pyridine | | | | | B | | | |
| Preparative Example 47: 5-(1-methyl-1*H*-pyrazol-4-yl)-3-(quinazolin-6-yl)thieno[3,2-*b*]pyridine | | | | | B | | | |
| Preparative Example 49: 5-(1-methyl-1*H*-pyrazol-4-yl)-3-(quinoxalin-6-yl)-thieno[3,2-*b*]pyridine | | | | | A | | | |
| Preparative Example 51: *3-*(*2H-*indazol-5-yl)-5-(1-methyl-1*H*-pyrazol-4-yl)-thieno[3,2-*b*]-pyridine | | | | | B | | | |
| Preparative Example 53: 3-([1,2,4]-triazolo[1,5-a]pyridin-6-yl)-5-(1-methyl-1H-pyrazol-4-yl)thieno[3,2-b]pyridine | B | B | C | B | A | B | B | A |
| Preparative Example 55: 5-(1-methyl-1*H*-pyrazol-4-yl)-3-(pyrazolo[1,5-*a*]-pyrimidin-6-yl)thieno[3,2-*b*]pyridine | | | | | B | | | |
| Preparative Example 57: 3-(imidazo[1,2-*a*]pyridin-6-yl)-5-(1-methyl-1*H*-pyrazol-4-yl)thieno[3,2-*b*]pyridine | | | | | A | | | |
| Preparative Example 59: 3-([1,2,4]-triazolo[4,3-*a*]pyridin-6-yl)-5-(1-methyl-1*H*-pyrazol-4-yl)thieno[3,2-*b*]pyridine | | | | | B | | | |
| Preparative Example 61: 5-(1-methyl-1*H*-pyrazol-4-yl)-3-(pyrazolo[1,5-*a*]-pyrimidin-2-yl)thieno[3,2-*b*]pyridine | | | | | B | | | |
| Preparative Example 63: 3,5-bis(1-methyl-1*H*-pyrazol-4-yl)thieno[3,2-*b*]pyridine | B | B | C | B | B | B | B | C |
| Preparative Example 65: 3-(1-benzyl-1*H*-pyrazol-4-yl)-5-chlorothieno[3,2-*b*]pyridine | C | B | D | D | B | A | B | C |
| Preparative Example 67: 3-(isothiazol-5-yl)-5-(1-methyl-1*H*-pyrazol-4-yl)thieno[3,2-*b*]pyridine | A | A | C | B | A | B | A | A |
| Preparative Example 69: 5-(1-methyl-1*H*-pyrazol-4-yl)-3-(thiazol-5-yl)thieno[3,2-*b*]pyridine | B | B | C | B | A | B | A | B |
| Preparative Example 71: 3-(3-methyl-1,2,4-thiadiazol-5-yl)-5-(1-methyl-1*H-*pyrazol-4-yl)thieno[3,2-*b*]pyridine | B | B | C | B | A | B | B | B |
| Preparative Example 73: (5-(1-methyl-1*H*-pyrazol-4-yl)thieno[3,2-*b*]pyridin-3-yl)(phenyl)-methanone | D | D | D | D | D | D | D | D |
| Preparative Example 74: (5-(1-methyl-1*H*-pyrazol-4-yl)thieno[3,2-*b*]pyridin-3-yl)(phenyl)methanone | B | B | C | D | D | C | B | C |
| Preparative Example 76: 5-(1-methyl-1*H*-pyrazol-4-yl)-*N*-phenylthieno[3,2-*b*]pyridin-3-amine | A | A | C | B | B | B | A | B |
| Preparative Example 77: 5-(1-ethyl-1*H-*pyrazol-4-yl)-3-(pyridin-4-yl)thieno[3,2-*b*]pyridine | C | C | D | C | C | C | C | C |
| Preparative Example 78: 3-(pyridin-4-yl)-5-(1-(trifluoromethyl)-1*H*-pyrazol-4-yl)thieno[3,2-*b*]pyridine | C | C | D | D | D | D | D | D |
| Preparative Example 79: 5-(1-isopropyl-1*H*-pyrazol-4-yl)-3-(pyridin-4-yl)thieno[3,2-*b*]pyridine | D | D | D | D | C | D | D | C |
| Preparative Example 80: 5-(1-(tert-butyl)-1H-pyrazol-3-yl)-3-(pyridin-4-yl)thieno[3,2-b]pyridine | | | | | D | | | |
| Preparative Example 81: 5-(1-benzyl-1*H*-pyrazol-4-yl)-3-(pyridin-4-yl)thieno[3,2-*b*]pyridine | C | C | D | D | B | B | C | D |
| Preparative Example 82: 5-(5-(cyclopropylmethyl)-1-methyl-1*H*-pyrazol-4-yl)-3-(pyridin-4-yl)thieno[3,2-*b*]pyridine | C | C | C | D | B | A | C | B |
| Preparative Example 83: *tert*-butyl(1-methyl-4-(3-(pyridin-4-yl)thieno[3,2-*b*]pyridin-5-yl)-1*H*-pyrazol-3-yl)carbamate | C | C | D | D | D | D | C | C |
| Preparative Example 84: 1-methyl-4-(3-(pyridin-4-yl)thieno[3,2-b]pyridin-5-yl)-1*H*-pyrazol-3-amine | C | C | D | D | D | D | C | C |
| Preparative Example 85: 3-(pyridin-4-yl)-5-(1,3,5-trimethyl-1*H*-pyrazol-4-yl)thieno[3,2-*b*]pyridine | C | C | D | D | C | D | C | C |
| Preparative Example 86: 5-(1-methyl-1*H*-pyrazol-3-yl)-3-(pyridin-4-yl)thieno[3,2-*b*]pyridine | C | D | D | D | A | D | C | D |
| Preparative Example 87: 5-(1-ethyl-1*H-*pyrazol-3-yl)-3-(pyridin-4-yl)thieno[3,2-*b*]pyridine | | | | | C | | | |
| Preparative Example 88: 5-(1-phenyl-1*H*-pyrazol-3-yl)-3-(pyridin-4-yl)thieno[3,2-*b*]pyridine | | | | | C | | | |
| Preparative Example 89: 5-(1-benzyl-1*H*-pyrazol-3-yl)-3-(pyridin-4-yl)thieno[3,2-*b*]pyridine | | | | | C | | | |
| Preparative Example 90: 5-(1-methyl-1*H*-pyrazol-5-yl)-3-(pyridin-4-yl)thieno[3,2-*b*]pyridine | C | C | D | D | C | D | C | C |
| Preparative Example 91: 5-(1-methyl-1*H*-imidazol-5-yl)-3-(pyridin-4-yl)thieno[3,2-*b*]pyridine | C | C | C | C | A | B | B | C |
| Preparative Example 92: 5-(isothiazol-5-yl)-3-(pyridin-4-yl)thieno[3,2-*b*]pyridine | | | | | C | | | |
| Preparative Example 93: 5-(1-methyl-1*H*-1,2,3-triazol-4-yl)-3-(pyridin-4-yl)thieno[3,2-*b*]pyridine | D | D | D | D | C | C | C | C |
| Preparative Example 94: 5-(2-methyl-2*H*-1,2,3-triazol-4-yl)-3-(pyridin-4-yl)thieno[3,2-*b*]pyridine | C | C | D | D | C | D | C | C |
| Preparative Example 95: 5-(1-methyl-1*H*-1,2,3-triazol-4-yl)-3-(pyridin-4-yl)thieno[3,2-*b*]pyridine | | | | | C | | | |
| Preparative Example 96: 5-(1-methyl-1*H*-1,2,4-triazol-5-yl)-3-(pyridin-4-yl)thieno[3,2-*b*]pyridine | | | | | D | | | |
| Preparative Example 97: *N*-(1-methyl-1*H*-pyrazol-4-yl)-3-(pyridin-4-yl)thieno[3,2-*b*]pyridin-5-amine | | | | | B | | | |
| Preparative Example 98: *N*-(1*H-*pyrazol-4-yl)-3-(pyridin-4-yl)thieno[3,2-*b*]pyridin-5-amine | | | | | A | | | |
| Preparative Example 99: *N*-(1-methyl-1*H*-pyrazol-3-yl)-3-(pyridin-4-yl)thieno[3,2-*b*]pyridin-5-amine | B | C | C | D | A | C | C | B |
| Preparative Example 100: *N*-methyl-*N-*(1-methyl-1*H*-pyrazol-3-yl)-3-(pyridin-4-yl)thieno[3,2-*b*]pyridin-5-amine | | | | | C | | | |
| Preparative Example 101: *N*-(1H-pyrazol-3-yl)-3-(pyridin-4-yl)thieno[3,2-*b*]pyridin-5-amine | A | B | B | C | A | C | A | A |
| Preparative Example 102: *N*-(1,5-dimethyl-1*H*-pyrazol-3-yl)-3-(pyridin-4-yl)thieno[3,2-*b*]pyridin-5-amine | | | | | B | | | |
| Preparative Example 103: *tert*-butyl (1- methyl-3-((3-(pyridin-4-yl)thieno[3,2-*b*]pyridin-5-yl)amino)-1*H*-pyrazol-5-yl)carbamate | | | | | D | | | |
| Preparative Example 104: 1-methyl-*N*3-(3-(pyridin-4-yl)thieno[3,2-*b*]pyridin-5-yl)-1*H*-pyrazole-3,5-diamine | | | | | C | | | |
| Preparative Example 105: Ethyl 1-methyl-3-((3-(pyridin-4-yl)thieno[3,2-*b*]pyridin-5-yl)amino)-1*H*-pyrazole-5-carboxylate | | | | | D | | | |
| Preparative Example 106: *N*-(1-methyl-4-(methylthio)-1*H*-pyrazol-3-yl)-3-(pyridin-4-yl)thieno[3,2-*b*]pyridin-5-amine | | | | | B | | | |
| Preparative Example 107: Ethyl 1-methyl-3-((3-(pyridin-4-yl)thieno[3,2-*b*]pyridin-5-yl)amino)-1*H*-pyrazole-4-carboxylate | | | | | D | | | |
| Preparative Example 108: *N*-((1-methyl-1*H*-pyrazol-3-yl)methyl)-3-(pyridin-4-yl)thieno[3,2-*b*]pyridin-5-amine | | | | | D | | | |
| Preparative Example 109: 5-((1-methyl-1*H*-pyrazol-3-yl)oxy)-3-(pyridin-4-yl)thieno[3,2-*b*]pyridine | | | | | C | | | |
| Preparative Example 110: 5-((1-methyl-1*H*-pyrazol-3-yl)oxy)-3-(pyridin-4-yl)thieno[3,2-*b*]pyridine | | | | | B | | | |
| Preparative Example 111: 5-(pyridin-2-yl)-3-(pyridin-4-yl)thieno[3,2-*b*]pyridine | | | | | B | | | |
| Preparative Example 112: 3-(pyridin-4-yl)-5-(pyrimidin-2-yl)thieno[3,2-*b*]pyridine | | | | | D | | | |
| Preparative Example 113: *N*-(pyridazin-3-yl)-3-(pyridin-4-yl)thieno[3,2-*b*]pyridin-5-amine | | | | | B | | | |
| Preparative Example 114: 3-(pyridin-4-yl)*-N*-(pyrimidin-4-yl)thieno[3,2-*b*]pyridin-5-amine | | | | | C | | | |
| Preparative Example 115: 5-(pyrazolo[1,5-*a*]pyrimidin-3-yl)-3-(pyridin-4-yl)thieno[3,2-*b*]pyridine | | | | | C | | | |
| Preparative Example 116: *tert*-butyl 2-(((3-(pyridin-4-yl)thieno[3,2-*b*]pyridin-5-yl)oxy)-methyl)morpholine-4-carboxylate | | | | | D | | | |
| Preparative Example 117: *tert*-butyl (S)-2-(((3-(pyridin-4-yl)thieno[3,2-b]pyridin-5-yl)oxy)-methyl)morpholine-4-carboxylate | | | | | D | | | |
| Preparative Example 118: 2-(((3-(pyridin-4-yl)thieno[3,2-b]pyridin-5-yl)oxy)methyl)-morpholine | | | | | B | | | |
| Preparative Example 119: 3-(isothiazol-5-yl)-5-(1-methyl-1*H*-pyrazol-3-yl)thieno[3,2-*b*]pyridine | | | | | B | | | |
| Preparative Example 120: 3-((3-(isothiazol-5-yl)thieno[3,2-*b*]pyridin-5-yl)amino)-1-methyl-1*H*-pyrazole-4-carbonitrile | | | | | A | | | |
| Preparative Example 121: 3-phenyl-5-(1*H*-pyrazol-4-yl)thieno[3,2-*b*]pyridine | | | | | B | | | |
| Preparative Example 122: 3-phenyl-5-(3,4,5-trifluorophenyl)thieno[3,2-*b*]pyridine | D | D | D | D | D | D | D | D |
| Preparative Example 123: 5-(furan-3-yl)-3-phenylthieno[3,2-*b*]pyridine | D | D | D | D | D | D | D | D |
| Preparative Example 124: 4-(3-phenylthieno[3,2-*b*]pyridin-5-yl)isoxazole | D | D | D | D | D | D | D | D |
| Preparative Example 125: 4-(5-chlorothieno[3,2-*b*]pyridin-3-yl)-*N*,*N-*dimethylpyridin-2-amine | | | | | D | | | |
| Preparative Example 126: Ethyl 3-((3-(2-((*tert*butoxycarbonyl)amino)pyridin-4-yl)thieno[3,2-*b*]pyridin-5-yl)amino)-1-methyl-1*H*-pyrazole-4-carboxylate | | | | | D | | | |
| Preparative Example 128: Methyl 3-((3-(2-(aminomethyl)pyridin-4-yl)thieno[3,2-*b*]pyridin-5-yl)amino)-1-methyl-1*H*-pyrazole-4-carboxylate | | | | | D | | | |
| Preparative Example 129: 3-([1,2,4]triazolo[1,5-*a*]pyridin-6-yl)-5-(1-methyl-1*H*-pyrazol-3-yl)thieno[3,2-*b*]pyridine | | | | | B | | | |
| Preparative Example 130: 3-([1,2,4]triazolo[1,5-*a*]pyridin-6-yl)-5-(pyrimidin-2-yl)thieno[3,2-*b*]pyridine | | | | | C | | | |
| Preparative Example 131: 3-([1,2,4]triazolo[1,5-*a*]pyridin-6-yl)-5-(pyridin-3-yl)thieno[3,2-*b*]pyridine | | | | | B | | | |
| Preparative Example 132: 3-([1,2,4]triazolo[1,5-*a*]pyridin-6-yl)-5-(pyridin-4-yl)thieno[3,2-*b*]pyridine | | | | | A | | | |
| Preparative Example 133: *tert*-butyl 4-((3-([1,2,4]triazolo[1,5-*a*]pyridin-6-yl)thieno[3,2-*b*]pyridin-5-yl)amino)-1*H*-pyrazole-1-carboxylate | | | | | D | | | |
| Preparative Example 134: 3-([1,2,4]triazolo[1,5-*a*]pyridin-6-yl)-*N-*(1*H*-pyrazol-4-yl)thieno[3,2-*b*]pyridin-5-amine | | | | | B | | | |
| Preparative Example 135: 3-([1,2,4]triazolo[4,3-*a*]pyridin-6-yl)-*N-*(1-methyl-1*H*-pyrazol-3-yl)thieno[3,2-*b*]pyridin-5-amine | | | | | C | | | |
| Preparative Example 137: 5-(1-methyl-1*H*-pyrazol-4-yl)-3-(1-phenylvinyl)thieno[3,2-*b*]pyridine and 3-(1-methyl-1*H*-pyrazol-4-yl)-5-(1-phenylvinyl)thieno[3,2-*b*]pyridine | C | C | C | D | B | C | C | C |
| | C | B | D | D | D | C | B | C |
| Preparative Example 139: 3-(1-methyl-1*H*-pyrazol-4-yl)-5-phenoxythieno[3,2-*b*]pyridine | D | C | C | D | B | C | C | C |
| Preparative Example 142: 2-methyl-5-(1-methyl-1*H*-pyrazol-4-yl)-3-(pyridin-4-yl)thieno[3,2-*b*]pyridine | C | C | D | C | C | D | C | C |
| Preparative Example 143: 2-methyl-5-(1-methyl-1*H*-pyrazol-3-yl)-3-(pyridin-4-yl)thieno[3,2-*b*]pyridine | | | | | C | | | |
| Preparative Example 150: 6-methyl-5-(1-methyl-1*H*-pyrazol-4-yl)-3-(pyridin-4-yl)thieno[3,2-*b*]pyridine | C | C | D | D | C | C | C | C |
| Preparative Example 151: 6-methyl-5-(1-methyl-1*H*-pyrazol-3-yl)-3-(pyridin-4-yl)thieno[3,2-*b*]pyridine | D | D | D | D | A | D | D | D |
| Preparative Example 157: 7-methyl-5-(1-methyl-1*H*-pyrazol-4-yl)-3-(pyridin-4-yl)thieno[3,2-*b*]pyridine | C | C | D | D | C | D | D | C |
| Preparative Example 158: 7-methyl-5-(1-methyl-1*H*-pyrazol-3-yl)-3-(pyridin-4-yl)thieno[3,2-*b*]pyridine | | | | | B | | | |
| Preparative Example 162: 7-chloro-5-(1-methyl-1*H*-pyrazol-4-yl)-3-(pyridin-4-yl)thieno[3,2-*b*]pyridine | B | B | C | D | C | C | B | B |
| Preparative Example 163: 7-chloro-5-(1-methyl-1*H*-pyrazol-3-yl)-3-(pyridin-4-yl)thieno[3,2-*b*]pyridine | C | C | D | D | B | D | C | C |
| Preparative Example 165: 7-methoxy-5-(1-methyl-1*H*-pyrazol-3-yl)-3-(pyridin-4-yl)thieno[3,2-*b*]pyridine | C | C | D | D | B | D | C | C |

## Claims

1. A compound of general formula I or a pharmaceutically acceptable salt thereof 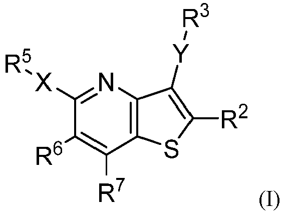 wherein:
R² is selected from H, C1-C4 alkyl and CF₃;
Y is selected from bond, -C(=O)-, -CH(OH)-, -C(=CH₂)-, -CH₂-O-, -CH₂-S-, -CH₂-NH₂-, O, S, SO₂, NR⁸ or CR⁸R⁸;
R³ is selected from the group consisting of:
- C6-C14 aryl,
- 3-10-membered heteroaryl comprising at least one heteroatom selected from S, O, N,
- C5-C8 cycloalkyl,
- 5-8-membered heterocycloalkyl comprising at least one heteroatom selected from S, O, N,
- C5-C8 cycloalkenyl,
- 5-8-membered heterocycloalkenyl comprising at least one heteroatom selected from S, O, N,
wherein each of the listed substituents can optionally be substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, cyclo(C3-C6 alkyl), cyclo(C3-C6 alkyl)-C1-C2 alkyl-, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NO₂, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, - (C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, - COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SO₂-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SOz-NH-(C1-C4 alkylene)-, (C1-C4 alkyl)-SO₂-N(C1-C4 alkyl)-, (C1-C4 alkyl)-SO₂-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CON(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
X is selected from bond, O, S, SO₂, -C(=O)-, -CH(OH)-, -CH₂-O-, -CH₂-S-, -CH₂-NH₂-, -C(=CH₂)-, NR⁸ or CR⁸R⁸;
R⁵ is selected from the group consisting of:
- C6-C14 aryl,
- 3-10-membered heteroaryl comprising at least one heteroatom selected from S, O, N,
- C5-C8 cycloalkyl,
- 5-8-membered heterocycloalkyl comprising at least one heteroatom selected from S, O, N,
- C5-C8 cycloalkenyl,
- 5-8-membered heterocycloalkenyl comprising at least one heteroatom selected from S, O, N,
wherein each of the listed substituents can optionally be substituted by at least one substituent selected independently from C1-C4 alkyl, C6-C10 aryl, 3-7-membered heteroaryl comprising at least one heteroatom selected from S, O, N, 3-7-membered cycloheteroalkyl comprising at least one heteroatom selected from S, O, N, cyclo(C3-C6 alkyl), cyclo(C3-C6 alkyl)-C1-C2 alkyl-, halogen, OH, HO-C1-C4 alkyl, O(C1-C4 alkyl), O(C3-C7 cycloalkyl), O(C1-C4 halogenalkyl), (C1-C4 alkyl)-O-C1-C4 alkyl, O(C5-C6 aryl or 5-6-membered heteroaryl), SH, S(C1-C4 alkyl), S(C3-C7 cycloalkyl), S(C1-C4 halogenalkyl), S(C5-C6 aryl or 5-6-membered heteroaryl), SO(C1-C4 alkyl), SO₂(C1-C4 alkyl), CF₃, C₂F₅, OCF₃, OC₂F₅, amino (NH₂), NH₂-(C1-C4 alkyl)-, HCO-NH-(C1-C4 alkyl)-, NO₂, CN, N₃, C1-C4 alkylamino, di(C1-C4 alkyl)amino, (C5-C6 aryl or 5-6-membered heteroaryl)amino, di(C5-C6 aryl or heteroaryl)amino, (C1-C4 alkyl)-NH-C1-C4 alkyl, (C1-C4 alkyl)₂-N-C1-C4-alkyl, =O, =S, =N-OH, - (C1-C4 alkylene)=N-OH, =N-O(C1-C4 alkyl), -(C1-C4 alkylene)=N-O(C1-C4 alkyl), -(C1-C4 alkylene)-CHO, -CHO, -COOH, -(C1-C4 alkylene)-COOH, -CONH₂, -(C1-C4 alkylene)-CONH₂, - COO(C1-C4 alkyl), -(C1-C4 alkylene)-COO(C1-C4 alkyl), -CO(C1-C4 alkyl), -(C1-C4 alkylene)-CO(C1-C4 alkyl), -CO(C5-C6 aryl or 5-6-membered heteroaryl), -(C1-C4 alkylene)-CO(C5-C6 aryl or 5-6-membered heteroaryl), (C1-C4 alkyl)-SO₂-, (C1-C4 alkyl)-SO₂-(C1-C4 alkylene)-, (C1-C4 alkyl)-SO-, (C1-C4 alkyl)-SO-(C1-C4 alkylene)-, (C1-C4 alkyl)-SO₂-NH-, (C1-C4 alkyl)-SOz-NH-(C1-C4 alkylene)-, (C1-C4 alkyl)-SO₂-N(C1-C4 alkyl)-, (C1-C4 alkyl)-SO₂-N(C1-C4 alkyl)-(C1-C4 alkylene)-, (C1-C4 alkyl)-O-CO-, (C1-C4 alkyl)-O-CO-(C1-C4 alkylene)-, (C1-C4 alkyl)-NH-CO-, (C1-C4 alkyl)-NH-CO-(C1-C4 alkylene)-, (C6-C10 aryl)-NH-CO, (C6-C10 aryl)-NH-CO-(C1-C4 alkylene)-, (C1-C4 alkyl)₂N-CO-, (C1-C4 alkyl)₂N-CO-(C1-C4 alkylene)-, NH₂-SO₂-, NH₂-SO₂-(C1-C4-alkylene)-, (C6-C10 aryl)-NH-SO₂-, (C6-C10 aryl)-NH-SO₂-(C1-C4 alkylene)-, (C1-C4 alkyl)-NH-SO₂-, (C1-C4 alkyl)-NH-SO₂-(C1-C4 alkylene)-, (C1-C4 alkyl)₂N-SO₂-, (C1-C4 alkyl)₂N-SO₂-(C1-C4 alkylene)-, (C1-C4 alkyl)-CO-NH-, (C1-C4 alkyl)-CO-NH-(C1-C4 alkylene)-, (C1-C4 alkyl)-CO-N(C1-C4 alkyl)-, (C1-C4 alkyl)-CO-N(C1-C4 alkyl)-(C1-C4 alkylene)-, (C1-C4 alkyl)-OCO-NH-, (C1-C4 alkyl)-OCO-NH-(C1-C4 alkylene)-, (C1-C4 alkyl)-OCO-N(C1-C4 alkyl)-, (C1-C4 alkyl)-OCO-N(C1-C4 alkyl)-(C1-C4 alkylene)-, (C1-C4 alkyl)-CO-NH-CO-, (C1-C4 alkyl)-CO-N(C1-C4 alkyl)-CO-, NH₂-CO-NH-, (C1-C4 alkyl)-NH-CO-NH-, (C1-C4 alkyl)₂N-CO-NH-, NH₂-CO-N(C1-C4 alkyl)-, (C1-C4 alkyl)-NH-CON(C1-C4 alkyl)-, (C1-C4 alkyl)₂N-CO-N(C1-C4 alkyl)-, NH₂-S(O)₂-NH-, (C1-C4 alkyl)-NH-S(O)₂-NH-, (C1-C4 alkyl)₂N-S(O)₂-NH-, NH₂-S(O)₂-N(C1-C4 alkyl)-, (C1-C4 alkyl)-NH-S(O)₂-N(C1-C4 alkyl)-, (C1-C4 alkyl)₂N-S(O)₂-N(C1-C4 alkyl)-, (C1-C4 alkyl)₂N-(C1-C4 alkylene)-CO-, (C1-C4 alkyl)₂N-(C1-C4 alkylene)-SO₂-, (C1-C4 alkyl)₂N-(C1-C4 alkylene)-SO₂-NH-, (C1-C4 alkyl)₂N-(C1-C4 alkylene)-NH-SO₂-;
R⁶ is selected from H, C1-C4 alkyl and CF₃;
R⁷ is selected from H, C1-C4 alkyl, F, Cl, Br, OR⁸ and CF₃;
R⁸ is independently selected from H and C1-C4 alkyl.

2. Compound according to claim 1, wherein R³ is selected from the group consisting of C6-C10 aryl and 5-10-membered heteroaryl comprising at least one heteroatom selected from S, O, N, wherein the aryl or heteroaryl are optionally substituted.

3. Compound according to claim 1, wherein R³ is selected from the group consisting of phenyl and 5-10-membered heteroaryl comprising at least one heteroatom N and optionally at least one other heteroatom selected from S, O, N, wherein the phenyl or heteroaryl are optionally substituted.

4. Compound according to claim 1, wherein R³ is selected from the group consisting of pyridinyl, phenyl, thiazolyl, thiadiazolyl, isothiazolyl, pyrimidinyl, pyridazinyl, pyrazolyl, pyrrolopyridinyl, quinazolinyl, quinoxalinyl, indazolyl, triazolopyridinyl, pyrazolopyrimidinyl, pyrazolopyridinyl, imidazopyridinyl, dihydropyridinyl and tetrahydropyridinyl, wherein any of these substituent groups is optionally substituted.

5. Compound according to any one of claims 1 to 4, wherein R³ is substituted by one or more substituents selected independently from C1-C4 alkyl, halogen, morpholinyl, benzyl, amino, aminomethyl and trifluoromethyl.

6. Compound according to any one of claims 1 to 5, wherein R⁵ is selected from the group consisting of C6-C10 aryl, 5-10-membered heteroaryl comprising at least one heteroatom selected from S, O, N, and 5-6-membered heterocycloalkyl comprising at least one heteroatom selected from S, O, N, wherein the aryl or heteroaryl or heterocycloalkyl are optionally substituted.

7. Compound according to any one of claims 1 to 5, wherein R⁵ is selected from the group consisting of phenyl, morpholinyl and 5-10-membered heteroaryl comprising at least one heteroatom N and optionally at least one other heterotom selected from S, O, N, wherein the phenyl, morpholinyl, or heteroaryl are optionally substituted.

8. Compound according to any one of claims 1 to 5, wherein R⁵ is selected from the group consisting of pyrazolyl, phenyl, morpholinyl, imidazolyl, isothiazolyl, triazolyl, pyridinyl, pyrimidinyl, pyridazinyl, pyrazolopyrimidinyl, furanyl, and isoxazolyl, wherein any of these substituent groups is optionally substituted.

9. Compound according to any one of claims 1 to 8, wherein R⁵ is substituted by one or more substituents selected independently from C1-C4 alkyl, trifluoromethyl, benzyl, phenyl, cyclo(C3-C6 alkyl), cyclo(C3-C6 alkyl)-C1-C2 alkyl-, amino, halogen, -COO(C1-C4 alkyl), C1-C4 alkylthio, C1-C4-alkoxy, CN.

10. Compound according to any one of claims 1 to 9, which is selected from 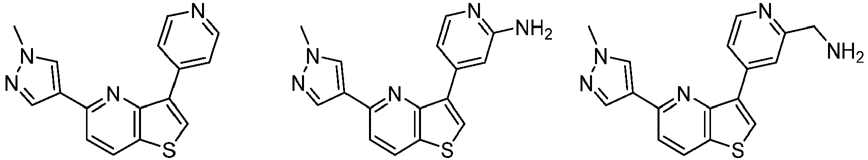 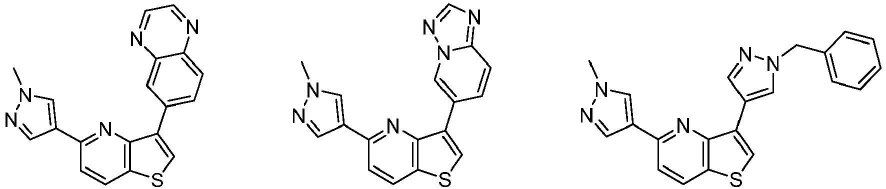 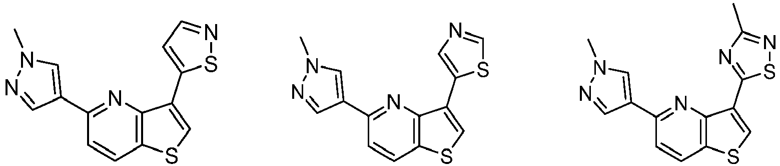 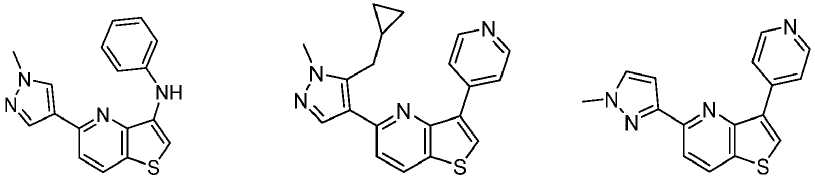 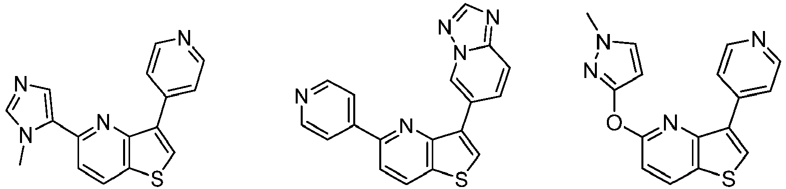 and pharmaceutically acceptable salts thereof.

11. Compounds of formula I according to any one of claims 1-10 for use as medicaments.

12. Compounds of formula I according to any one of claims 1-10 for use in the treatment of diseases involving the kinases CDKL, CLK, DYRK, Haspin, PIM, TAF1L and/or TRB.

13. Compounds of formula I according to any one of claims 1-10 for use in the treatment of cancers or neurodegenerative diseaes.

14. Compounds of formula I according to any one of claims 1-10 for use in the treatment of leukemia such as acute myeloid leukemia; autoimmune diseases such as rheumatoid arthritis or autoimmune hepatitis; peripheral neuropathic pain; cancers such as non-small-cell lung carcinoma (NSCLC), ovarian cancer, glioblastoma, breast cancer, lung cancer, lung adenocarcinoma, pancreas cancer, prostate cancer, brain cancer, ovarian cancer, colorectal cancer, liver cancer, hepatocellular carcinoma, squamous cell carcinoma; periodontitis; pulmonary fibrosis; obesity; insulin resistance; obesity-induced hyperinslinemia; atherosclerosis; neurodegenerative disorders such as Alzheimer's disease.
